(19) Europäisches Patentamt
European Patent Office
Office européen des brevets

(11) Veröffentlichungsnummer : **0 107 622**
**B1**

(12) # EUROPÄISCHE PATENTSCHRIFT

(45) Veröffentlichungstag der Patentschrift : **11.11.87**

(21) Anmeldenummer : **83810479.2**

(22) Anmeldetag : **17.10.83**

(51) Int. Cl.[4] : **C 07 D317/46**, C 07 D405/06, A 61 K 31/335, A 61 K 31/395, C 07 D409/12, C 07 D417/12

(54) **Neue Benzodioxolderivate, Verfahren zu deren Herstellung und entsprechende pharmazeutische Zusammensetzungen.**

(30) Priorität : **22.10.82 CH 6163/82**

(43) Veröffentlichungstag der Anmeldung : **02.05.84 Patentblatt 84/18**

(45) Bekanntmachung des Hinweises auf die Patenterteilung : **11.11.87 Patentblatt 87/46**

(84) Benannte Vertragsstaaten : **AT BE CH DE FR GB IT LI LU NL SE**

(56) Entgegenhaltungen : **DE-A- 2 426 505**

(73) Patentinhaber : **CIBA-GEIGY AG**
**Klybeckstrasse 141**
**CH-4002 Basel (CH)**

(72) Erfinder : **Ballenegger, Marc Ernest, Dr.**

**CH-1188 Gimel (CH)**
Erfinder : **Zbinden, Paul**
**Im Rainacker 6**
**CH-4108 Witterswil (CH)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die Erfindung betrifft neue Benzodioxolderivate, Verfahren zu deren Herstellung und entsprechende pharmazeutische Zusammensetzungen, sowie die Verwendung dieser neuen Stoffe und Zusammensetzungen.

In der Deutschen Offenlegungsschrift Nr. 2 426 505 werden 1,3-Benzodioxol-2-carbonsäuren, deren Salze, Ester und Amide, die durch einen gegebenenfalls substizuierten Phenylrest am aromatischen Teil des 1,3-Benzodioxolkerns substituiert sind, beschrieben, die sich zur Herabsetzung von Cholesterin und verschiedene Triglyceride enthaltenden Blutlipoproteinen eignen, wogegen die erfindungsgemässen substituierten Arylcarbonyl- und Arylniederalkanoyl-2-benzodioxolcarbonsäuren und deren Derivate diuretische, saliuretische und uricosurische Wirkungen aufweisen.

Die erfindungsgemässen neuen Verbindungen entsprechen der allgemeinen Formel I

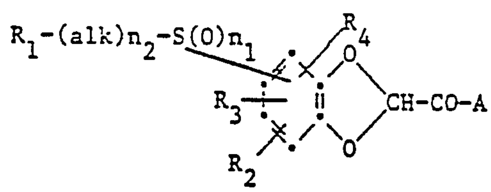

(I)

in denen $R_1$ ein unsubstituierter oder durch Alogen bis Atomnummer 35, Niederalkoxy oder Niederalkylthio substituierter Alkyl-, Alkenyl- oder Alkinylrest mit insgesamt, d. h. einschliesslich der Substituenten, höchstens 12 Kohlenstoffatomen, oder ein unsubstituierter oder durch Niederalkyl, Niedercycloalkyl, Niederalkoxy, Niederalkylthio, Niederalkylsulfonyl, Trifluormethyl, Hydroxy, Niederalkanoyl, Niederalkanoylamino, Niederalkoxycarbonylamino und/oder Niederalkylsulfonylamino substituierter Phenyl oder Naphthylrest, ein monocyclischer fünfgliedriger Heteroring aromatischen Charakters mit 2 Stickstoffatomen oder 1 Stickstoffatom und/oder ein Sauerstoff- oder Schwefelatom im Ring oder ein sechsgliedriger Heteroring aromatischen Charakters mit 1 oder 2 Stickstoffatomen im Ring oder ein bicylischer Rest, bestehend aus den genannten fünf- oder sechsgliedrigen Heteroringen aromatischen Charakters mit ankondensiertem Benzolring ist, alk einem Alkylen-, Alkenylen- oder Alkylidenrest mit höchstens 5 Kohlenstoffatomen, $n_1$ Null, eins oder zwei und $n_2$ Null oder eins bedeutet, $R_2$, $R_3$ und $R_4$ unabhängig voneinander Wasserstoff, Niederalkyl, Niederalkoxy oder Halogen bedeuten und A den Rest $-O-R_5$, worin $R_5$ für Wasserstoff oder einen unsubstituierten oder durch Niederalkoxy oder Halogen substituierten Alkylrest mit höchstens 12 C-Atomen, 2- oder 3-Niederalkenyl, 2-Niederalkinyl, Phenylniederalkyl oder Cinnamyl, in welchem der Phenylrest in gleicher Weise wie unter $R_1$ substituiert sein kann, oder den Rest

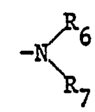

bedeutet, worin $R_6$ und $R_7$ unabhängig voneinander für Wasserstoff oder Niederalkyl, oder unter sich verbunden und zusammen mit dem anliegenden Stickstoffatom für gegebenenfalls niederalkylsubstituiertes Tetra- bis Hexamethylenimino, 4-Morpholinyl oder 1H-Tetrazol-1-yl stehen, Salze von Verbindungen der allgemeinen Formel I, in denen A $OR_5$ und darin $R_5$ Wasserstoff bedeuten, mit Basen, sowie Säureadditionssalze von Verbindungen der allgemeinen Formel I, deren Rest $R_1$ basischen Charakter aufweist, sowie die Herstellung dieser Salze. Sie können als Racemate oder optische Antipoden, oder bei entsprechender Bedeutung der Variablen auch als Racemategemische vorliegen. Soweit nicht anders bemerkt, werden vor- und nachstehend unter niederen Resten oder Verbindungen solche mit höchstens 7, vorzugsweise höchstens 4 Kohlenstoffatomen verstanden.

Ein Rest $R_1$ ist wie angegeben ein Alkyl-, Alkenyl- oder Alkinylrest mit höchstens 12 Kohlenstoffatomen, vorzugsweise Niederalkyl, Niederalkenyl oder Niederalkinyl, wie Octyl, Nonyl, Decyl, Undecyl, Dodecyl, 2,2,4-Trimethyl-1-pentenyl, 1-Octinyl bzw. Pentyl, Isopentyl, Hexyl, Heptyl, 1-Hexenyl, 1-Heptenyl bzw. insbesondere Methyl, Aethyl, Propyl, Isopropyl, Butyl, Isobutyl, Tert.-butyläthenyl, 1-Methyläthenyl, 2-Propenyl, 2-Butenyl, 2-Methyl-2-propenyl, Aethinyl oder 1-Propinyl.

$R_1$ als Phenyl- oder Naphthylrest ist insbesondere ein 1- oder 2-Naphthylrest und vor allem ein Phenylrest. Ein entsprechender monocyclischer fünfgliedriger Heteroring aromatischen Charakters $R_1$ mit zwei Stickstoffatome oder vorzugsweise ein Stickstoffatom und/oder ein Sauerstoff- oder Schwefelatom, und ist beispielsweise ein mono- oder diazacyclischer, oxa- oder thiacyclischer oder oxaza- oder thiazacyclischer Rest mit 5 Ringgliedern, z. B. 1H-Pyrrolyl, wie 1H-Pyrrol-2-yl oder -3-yl, 1H-Pyrazolyl, wie 1H-Pyrazol-3-yl, -4-yl- oder -5-yl, 1H-Imidazolyl, wie 1H-Imidazol-2-yl, -4-yl oder -5-yl, Furyl, wie 2- oder 3-Furyl, Thienyl, wie 2- or 3-Thienyl, Oxazolyl, wie 2-Oxazolyl, Isoxazolyl, wie 3- oder 5-Isoxazolyl, Thiazolyl, wie 2- oder 4-Thiazolyl, oder ein sechsgliedriger Heteroring aromatischen Charakters mit 1 oder 2

Stickstoffatomen im Ring ist z. B. Pyridyl, wie 2-, 3- oder 4-Pyridyl, Pyridazinyl, wie 3-Pyridazinyl, Pyrimidinyl, wie 2-, 4- oder 5-Pyrimidinyl, oder 2-Pyrazinyl. Entsprechende bicyclische Reste $R_1$ bestehen beispielsweise aus einem 5-gliedrigen Heteroring aromatischen Charakters mit zwei Stickstoffatomen oder mit einem Stickstoffatom und/oder einem Sauerstoff- oder Schwefelatom als Ringgliedern und einem ankondensierten Benzolring, oder aus einem 6-gliedrigen Heteroring aromatischen Charakters mit zwei oder insbesondere einem Stickstoffatom als Ringgliedern und einem ankondensierten Benzolring. Dementsprechend ist bicyclisches Heteroaryl z. B. 1H-Indolyl, wie 1H-Indol-2-yl, -3-yl, -4-yl, -5-yl, oder -6-yl, 1H-Indazolyl, wie 1H-Indazol-3-yl, 1H-Benzimidazolyl, wie 1H-Benzimidazol-2-yl, -4-yl, -5-yl oder -6-yl, Benzofuranyl, wie 2-, 3-, 5- oder 6-Benzofuranyl, Benzo[b]thienyl, wie Benzo[b]thien-2-yl, -3-yl, -5-yl oder -6-yl, Benzoxazolyl, wie 2-, 4-, 5- oder 6-Benzoxazolyl, Benzothiazolyl, wie 2-, 4-, 5- oder 6-Benzothiazolyl, bzw. Chinolinyl, wie 2-, 4-, 5- oder 6-Chinolinyl, Isochinolinyl, wie 1-, 3- oder 4-Isochinolinyl, Chinazolinyl, wie 2-, 4- oder 6-Chinazolinyl, Chinoxalinyl, wie 2- oder 6-Chinoxalinyl, oder Phthalazinyl, wie 1- oder 6-Phthalazinyl.

Als substituierter Alkyl- oder Alkenyl-Rest in $R_1$ z. B. durch Halogen wie Brom oder insbesondere Fluor oder Chlor substituiert, wobei die Polysubstition am gleichen Kohlenstoffatom, d. h. geminal, und die Substitution an einer Mehrfachbindung, wie z. B. Trichlormethyl, 2,2,2-Trichloräthyl bzw. 2-Chloräthenyl, besonders erwähnt seien. Weiter kann ein Alkyl-Rest $R_1$ z. B. auch durch Niederalkoxy oder Niederalkylthio, wie z. B. durch die entsprechenden, weiter unten genannten Reste, substituiert sein und dann z. B. Methoxyäthyl, Aethoxyäthyl bzw. Methylthioäthyl darstellen. Als substituierter Phenyl oder Naphtylrest ist $R_1$ z. B. durch Halogen, wie Brom, Jod oder insbesondere Fluor oder Chlor, durch Niederalkyl, wie z. B. Aethyl, Propyl, Isopropyl, Butyl, Isobutyl, tert.-Butyl oder insbesondere Methyl, Niedercycloalkyl, wie Cyclopentyl oder insbesondere Cyclohexyl, Niederalkoxy, Niederalkylthio oder Niederalkylsulfonyl wie Aethoxy, Propoxy, Isopropoxy, Butoxy, Isobutoxy bzw. Aethylthio, Isopropylthio, Butylthio bzw. Aethylsulfonyl und insbesondere Methoxy bzw. Methylthio bzw. Methylsulfonyl, und/oder Trifluormethyl, Hydroxy, Niederalkanoyl, Niederalkanoylamino, Niederalkoxycarbonylamino oder Niederalkylsulfonylamino, wie z. B. Formyl, Acetyl, Propionyl, Butyryl, Isobutyryl, bzw. Formamido, Acetamido, Propionamido, Butyramido oder Isobutyramido bzw. Methoxycarbonylamino oder Aethoxycarbonylamino bzw.

Methansulfonamido oder Aethansulfonamido ; einfach oder mehrfach, vorzugsweise höchstens dreifach substituiert. Ein Alkylen-, Alkenylen oder Alkylidenrest alk aknn gerade oder verzweigt sein und ist z. B. 1,1- bzw. 2,2- oder 1,2-Dimethyläthylen, 1-Aethyläthylen, Tetramethylen oder 1-Propyläthylen bzw. 3,3-Dimethylpropenylen bzw. 1-Methyl-propyliden, 2-Methylpropyliden, Butyliden oder 1-Aethylpropyliden, insbesondere aber ein Rest mit höchstens 3 Kohlenstoffatomen, wie Aethylen, Propylen, Trimethylen bzw. Aethenylen, Propenylen oder 1-Methyläthenylen bzw. Aethyliden, Propyliden oder 1-Methyl-äthyliden und vor allem Methylen.

$R_2$, $R_3$, und $R_4$ sind als Niederalkyl z. B. Aethyl, Propyl, Isopropyl, Butyl, Isobutyl oder tert. Butyl und insbesondere Methyl ; als Niederalkoxy z. B. Aethoxy, Propopxy, Isopropoxy, Butoxy, Isobutoxy und insbesondere Methoxy, und als Halogen Brom, Jod, insbesondere Fluor oder vor allem Chlor.

$R_5$ als unsubstituierter oder substituierter Alkylrest mit höchstens 12 Kohlenstoffatomen bedeuten insbesondere Niederalkyl. $R_5$ als Alkyl ist z. B. Propyl, Isopropyl, Butyl, Isobutyl, Pentyl, Isopentyl, Neopentyl, Hexyl, Heptyl, Octyl, Nonyl, Decyl, Dodecyl und vor allem Methyl oder Aethyl ; $R_5$ als 2- oder 3-Niederalkenyl ist z. B. Allyl (2-Thiopenyl) oder 2-Methylallyl, 2-Butenyl oder 3-Butenyl ; 2-Niederalkinyl als $R_5$ ist 2-Propinyl ; Phenylniederalkyl $R_5$ ist z. B. Benzyl, 2-Phenyläthyl, 2- oder 3-Phenylpropyl oder 2-, 3- oder 4-Phenylbutyl.

Als Niederalkyl $R_6$ und $R_7$ können z. B. die bereits als entsprechende Vertreter von $R_5$ genannten Reste voliegen, und als gegebenenfalls niederalkylsubstituiertes Tetra- bis Hexamethylenimino bedeuten $R_6$ und $R_7$ zusammen z. B. 2- oder 3-Methyl-1-pyrrolidinyl, 2,5-, 3,3- oder 3,4-Dimethyl-1-pyrrolidinyl, 1-Piperidinyl, 2-, 3- oder 4-Methyl-1-piperidinyl, 2,6- oder 4,4-Dimethyl-1-piperidinyl oder Hexahydro-1H-azepin-1-yl.

Salze der neuen Verbindungen sind insbesondere Salze von Verbindungen der allgemeinen Formel I, worin A Hydroxy bzw. $R_5$ Wasserstoff bedeutet, mit Basen, vor allem pharmazeutisch verwendbare Salze solcher Verbindungen mit Basen. Als solche Salze mit Basen kommen beispielsweise Alkalimetall- oder Erdalkalimetallsalze, wie Natrium-, Kalium-, Calcium- oder Magnesiumsalze, ferner Ammoniumsalze mit Ammoniak oder organischen Aminen, wie Mono- oder Diniederalkylaminen, z. B. Methylamin, Aethylamin, Dimethylamin oder Diäthylamin, oder mit gegebenenfalls niederalkylierten Mono- oder Di(hydroxyalkyl)-aminen, oder mit Tri(hydroxyalkyl)aminen, z. B. 2-Aminoäthanol, 2-(Diäthylamino)-äthanol, 2,2'-Iminodiäthanol, N-Methyl-2,2'-iminodiäthanol oder 2,2', 2''-Nitrilotri-äthanol, in Betracht.

Als Säureadditionssalze, insbesondere pharmazeutisch annehmbare Säureadditionssalze von Verbindungen der allgemeinen Formel I, in denen $R_1$ basischen Charakter aufweist, kommen beispielsweise solche mit geeigneten anorganischen Säuren, wie Halogenwasserstoffsäuren, z. B. Chlorwasserstoffsäure oder Bromwasserstoffsäure, ferner Salpetersäure, Schwefelsäure oder Phosphorsäure, oder mit geeigneten organischen Säuren, wie Carbonsäuren, z. B. Essigsäure, Propionsäure, Glykolsäure, Bernsteinsäure, Maleinsäure, Hydroxymaleinsäure, Methylmaleinsäure, Fumarsäure, Aepfelsäure, Weinsäure, Zitronensäure, Benzoesäure, Zimtsäure, Mandelsäure, Salicylsäure, 4-Amino-salicylsäure, 2-Phenoxybenzoesäure, 2-Acetoxybenzoesäure, Embonsäure, Nicotinsäure oder Isonicotinsäure, oder organiscphen Sulfonsäuren, wie gegebenenfalls Hydroxy enthaltenden Niederalkansulfonsäuren, z. B. Methansulfonsäure, Aethansulfonsäure, 2-Hydroxyäthansulfonsäure oder Aethan-1,2-disulfonsäure, oder Arylsulfonsäuren, z. B. Benzolsulfonsäure, 4-Methylbenzosulfonsäure oder Naphthalin-2-sulfonsäure, oder weiteren sauren Substanzen, wie Ascorbinsäure, in Frage.

Die neuen Verbindungen der allgemeinen Formel I und ihre Salze besitzen wertvolle pharmakologische Eigenschaften. Sie wirken insbesondere diuretisch und natriuretisch an der Ratte im Dosisbereich von 1 bis 300 mg/kg per os und am Hund in Dosen ab 1 bis 20 mg/kg per os, wie durch Sammeln des Harns während 3 Stunden nach Verabreichung (Ratte) bzw. stündlich während 5 Stunden nach Verabreichung (Hund) und Bestimmung des Harnvolumens und der Natrium-, Kalium- und Chlorionen festgestellt werden kann. Hierbei wird die Kalium-Ausscheidung weniger stark gesteigert als die Natrium Ausscheidung ; hervorzuheben ist auch die gute Verträglichkeit.

Beispielsweise wird durch die Verabreichung von 10 mg/kg per os 5-Methyl-6-phenylsulfonyl-1,3-benzodioxol-2-carbonsäure an Ratten (6 Tiere pro Dosis) im Vergleich zu unbehandelten Kontrolltieren die Ausscheidung von Natriumionen auf das 3,6-fache, von Kaliumionen auf das 1,1-fache und von Chlorionen auf das 2,6-fache gesteigert. Am Hund wird z. B. durch die Verabreichung von 20 mg/kg per os 5-Methyl-6-phenylsulfonyl-1,3-benzodioxol-2-carbonsäure (4 Tiere pro Dosis) die durchschnittliche Ausscheidung pro Minute während der ersten 5 Stunden nach Verabreichung des Wirkstoffes im Vergleich zur durchschnittlichen Ausscheidung pro Minute während der letzten Stunde vor Verabreichung bezüglich der Natriumionen auf das 13,3-fache, bezüglich der Kaliumionen auf bloss das 1,5-fache, bezüglich der Chlorionen auf das 10,4-fache und bezüglich des Harnvolumens auf das 3,2-fache gesteigert. Die Steigerung der Kaliumionenausscheidung durch die genannte Carbonsäure ist somit bei der Ratte wie auch beim Hund im Vergleich zur Steigerung der Natriumionen- und Chlorionenausscheidung sehr gering.

Weiter besitzen die Verbindungen der allgemeinen Formel I uricosurische Wirksamkeit, wie sich z. B. anhand von Versuchen am Cebus-Affen (Cebus apella) zeigen lässt. Bei diesem Versuchen wird den Versuchstieren in Pentobarbital-Narkose Polyfructosan in Ringer-Lösung intravenös infundiert und die Prüfsubstanz als wässrige Lösung in ansteigenden Dosen intravenös injiziert. Vor der ersten Prüfsubstanz-Verabreichung und nach jeder Prüfsubstanzdosis wird während je drei 10-minütigen Perioden der Harn gesammelt und vor der ersten und nach der letzten Sammelperiode jeweils arteriell Blut entnommen. Die Harnsäure- und Polyfructosan-clearance wird aus deren Plasma- und Urin-Konzentration berechnet und schliesslich die fraktionelle Harnsäureausscheidung (Fractional excretion of uric acid, $FE_{UR}$) als Quotient von Harnsäure-clearance und glomerulärer Filtrationsrate bestimmt. Verbindungen der allgemeinen Formel I, wie z. B. die 5-Methyl-6-phenylsulfonyl-1,3-beozodioxol-2-carbonsäure zeigen in diesem Test Wirkungen im Dosisbereich von 1 bis 10 mg/kg i. v. Entsprechend können die Verbindungen der allgemeinen Formel I und ihre pharmazeutisch annehmbaren Salze als kaliumschonende Diuretika mit urikosurischer Zusatzwirkung, z. B. zur Behandlung von Oedemen und der Hypertension, verwendet werden.

Die Erfindung betrifft insbesondere Verbindungen der allgemeinen Formel I, in denen $R_1$ durch Niederalkyl, Niederalkoxy, Halogen bis Atomnummer 35, Niederalkanoyl und/oder Niederalkanoylamino höchstens dreifach substituiertes oder unsubstituiertes Phenyl, Thienyl, Furyl oder ar-Benzothiazolyl, alk Alkylen, Alkyliden oder Alkenylen mit höchstens 3 Kohlenstoffatomen, $R_2$ Niederalkyl oder Halogen bis Atomnummer 35, $R_3$ Wasserstoff, Niederalkyl oder Halogen bis Atomnummer 35 und $R_4$ Wasserstoff bedeutet und $n_1$, $n_2$ und A die unter der Formel I angegebene Bedeutung haben, $n_1$ jedoch insbesondere zwei, $n_2$ null und A $OR_5$ und darin $R_5$ Wasserstoff oder Niederalkyl bedeutet, sowie die Salze von solchen Verbindungen, in denen $R_5$ Wasserstoff ist, mit Basen.

Die Erfindung betrifft ganz besonders Verbindungen der allgemeinen Formel I, in denen $R_1$ durch Niederalkyl, Niederalkoxy, Halogen bis Atomnummer 35, Niederalkanoyl und/oder Niederalkanoylamino höchstens dreifach substituiertes oder unsubstituiertes Phenyl oder Thienyl, alk Methylen, $n_1$ zwei, $n_2$ null oder eins, $R_2$ Niederalkyll, insbesondere Methyl, oder Halogen, insbesondere Fluor oder Chlor, $R_3$ Wasserstoff oder Niederalkyl, insbesondere Methyl, $R_4$ Wasserstoff, und A $OR_5$ und darin $R_5$ Wasserstoff oder Niederalkyl, bedeutet, sowie die pharmazeutisch annehmbaren Salze von solchen Verbindungen, in denen $R_5$ Wasserstoff ist, mit Basen.

Die Erfindung betrifft vor allem Verbindungen der allgemeinen Formel I, in denen $R_1$ durch Niederalkyl, Niederalkoxy und/oder Halogen bis Atomnummer 35, höchstens dreifach substituiertes oder unsubstituiertes Phenyl, alk Methylen, $n_1$ zwei, $n_2$ null oder eins, $R_2$ Niederalkyl, insbesondere Methyl, oder Halogen, insbesondere Fluor oder Chlor, $R_3$ Wasserstoff oder Niederalkyl, insbesondere Methyl, $R_4$ Wasserstoff und A $OR_5$ und darin $R_5$ Wasserstoff oder Niederalkyl bedeutet, und der Phenylsulfonylrest in 5- oder 6-Stellung gebunden ist, sowie die pharmazeutisch annehmbaren Salze von solchen Verbindungen, in denen $R_5$ Wasserstoff ist, mit Basen.

Die Erfindung betrifft in erster Linie Verbindungen der allgemeinen Formel I, in denen $R_1$ durch Niederalkyl, insbesondere Methyl, Niederalkoxy, insbesondere Methoxy und/oder Halogen, insbesondere Chlor, höchstens dreifach substituiertes oder vor allem unsubstituiertes Phenyl, alk Methylen, $n_1$ zwei, $n_2$ eins oder vor allem null, $R_2$ Niederalkyl, insbesondere Methyl, oder Halogen, insbesondere Chlor, $R_3$ und $R_4$ Wasserstoff und A $OR_5$ und darin $R_5$ Wasserstoff oder Niederalkyl bedeuten, und der Phenylsulfonylrest in 5- oder 6-Stellung gebunden ist, sowie die pharmazeutisch annehmbaren Salze von solchen

Verbindungen, in denen $R_5$ Wasserstoff ist, mit Basen, wie die bereits weiter oben genannten 5-Methyl-6-phenylsulfonyl-1,3-benzodioxol-2-carbonsäure und ihre pharmazeutisch annehmbaren Salze mit Basen.

Die neuen Verbindungen der allgemeinen Formel I und Salze solcher Verbindungen, in denen A $OR_5$ und darin $R_5$ Wasserstoff ist, oder welche basischen Charakter besitzen, werden in an sich bekannter Weise hergestellt, indem man

a) eine Verbindung der allgemeinen Formel II

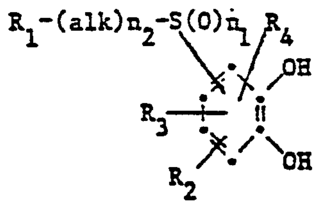

(II)

in welcher $R_1$, alk, $n_1$, $R_2$, $R_3$ und $R_4$ die unter der Formel I angegebene Bedeutung haben, oder ein Salz davon mit einer Verbindung der allgemeinen Formel III

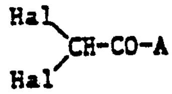

(III)

in welcher Hal Halogen bedeutet und A die unter der Formel I angegebene Bedeutung hat, oder einem Salz einer solchen Verbindung, in der A $OR_5$ und darin $R_5$ Wasserstoff bedeutet, umsetzt, oder

b) in einer Verbindung der allgemeinen Formel IV

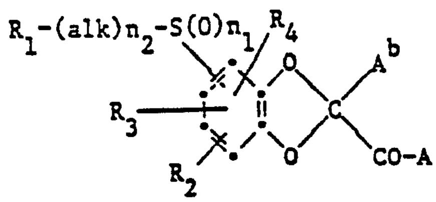

(IV)

in welcher $A^b$ Carboxy, Niederalkoxycarbonyl oder Acetyl bedeutet, und $R_1$, alkyl, $n_1$, $n_2$, $R_2$, $R_3$, $R_4$ und A die unter der Formel I angegebene Bedeutung haben, den Rest A durch Wasserstoff ersetzt, oder

c) zur Herstellung einer Verbindung der allgemeinen Formel I, in welcher A die unter der Formel I angegebene Bedeutung hat mit Ausnahme eines Restes $OR_5$, in welchem $R_5$ Wasserstoff bedeutet, und $R_1$, alk, $n_2$, $R_2$, $R_3$ und $R_4$ die unter der Formel I angegebene Bedeutung haben und $n_1$ eins oder zwei bedeutet, eine Verbindung der allgemeinen Formel V

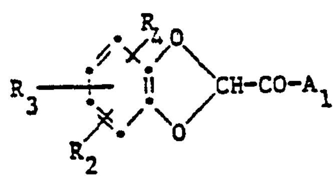

(V)

mit einer Verbindung der allgemeinen Formel VI,

$$R_1\text{—}(alk)n_2\text{—}S(O)n_1{}^c\text{—}OH \qquad (VI)$$

oder einem Anhydrid derselben, worin $A_1$ die unter der Formel I für A angegebene Bedeutung hat mit Ausnahme eines Restes $OR_5$, in welchem $R_5$ Wasserstoff bedeutet, $n_1{}^c$ eins oder zwei bedeutet und $R_2$, $R_3$ und $R_4$ bzw. $R_1$, alk und $n_2$ die unter der Formel I angegebene Bedeutung haben, umsetzt, oder

d) zur Herstellung einer Verbindungen, in welcher $n_2$ eins oder $R_1$ ein definitionsgemässer aliphatischer Rest ist und $R_1$ bzw. $n_2$, alk, $n_2$, $R_2$, $R_3$, $R_4$ und A die unter der Formel I angegebene Bedeutung haben, eine Verbindung der allgemeinen Formel VII

$$R_1\text{—}(alk)n_2{}^d\text{—}Z^d \qquad (VII)$$

in welcher $Z^d$ eine rekationsfähig veresterte Hydroxygruppe, insbesondere Halogen mit einer Atomnummer von mindestens 17, bedeutet, $R_1$ die unter der Formel I angegebene Bedeutung hat und $n_2$ eins bedeutet, oder falls $R_2$ ein aliphatischer Rest ist, auch null sein kann, oder eine Verbindung der

allgemeinen Formel VIII

$$R_1\text{—}(alk)n_1\text{—}\overset{\oplus}{N}{\equiv}N \qquad Z^{dd\ominus} \qquad \text{(VIII)}$$

in welcher $Z^{dd\ominus}$ ein einwertiges Anion oder das Normaläquivalent eines mehrwertrigen Anions bedeutet und $R_1$ und $n_1$ die unter der Formel I angegebene Bedeutung haben, mit einem Salz einer Verbindung der allgemeinen Formel IX

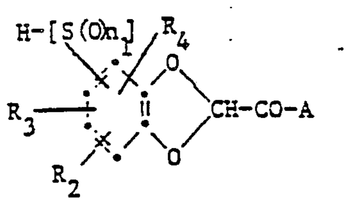

(IX)

in welcher $n_1$, $R_2$, $R_3$, $R_4$ und A die unter der Formel I angegebene Bedeutung haben, umsetzt, oder

e) eine gegebenenfalls in situ gebildete Metallverbindung der allgemeinen Formel Xa oder Xb

$R_1-(alk)n_2-M_1$ (Xa)

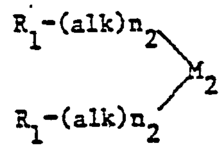

(Xb)

in welchen $M_1$ ein einwertiges bzw. $M_2$ ein zweiwertiges Metallion bedeutet und R, alk und $n_2$ die unter der Formel I angegebene Bedeutung haben, mit einer Verbindung der allgemeinen Formel XIa oder XIb,

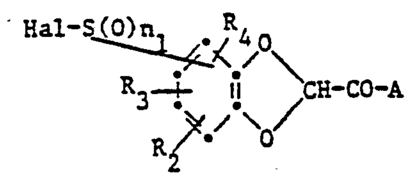

(XIa)

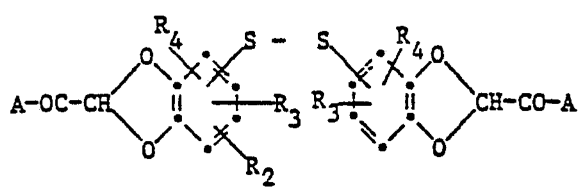

(XIb)

in welchen Hal Halogen bedeutet und $n_1$, $R_2$, $R_3$, $R_4$ und A die unter der Formel I angegebene Bedeutung haben, umsetzt, oder

f) zur Herstellung einer Verbindung der allgemeinen Formel I, in welcher A $OR_5$ und darin $R_5$ Wasserstoff bedeutet und $R_1$, alk, $n_1$, $n_2$, $R_2$, $R_3$ und $R_4$ die unter der Formel I angegebene Bedeutung haben, oder eines Salzes dieser Verbindung, in einer Verbindung der allgemeinen Formel XIII,

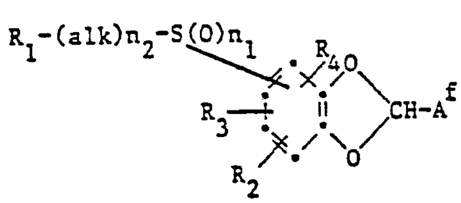

(XIII)

in welcher $A^f$ eine in die Carboxygruppe überführbare Gruppe bedeutet und $R_1$, alk, $n_1$, $n_2$, $R_2$, $R_3$ und $R_4$ die unter der Formel I angegebene Bedeutung haben, die Gruppe $A^f$ in die Carboxygruppe in freier oder Salzform überführt, oder

g) zur Herstellung einer Verbindung der allgemeinen Formel I, in welcher A die unter der Formel I angegebene Bedeutung hat mit Ausnahme eines Restes $OR_5$, in welchem $R_5$ Wasserstoff bedeutet, und

$R_1$, alk, $n_1$, $n_2$, $R_2$ und $R_3$ die unter der Formel I angegebene Bedeutung haben, in einer Verbindung der allgemeinen Formel XIV

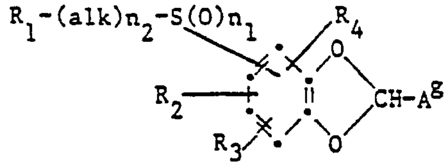

(XIV)

in welcher $A^g$ einen in einen Rest CO—$A_1$, in welchem $A_1$ die unter Formel I für A angegebene Bedeutung hat mit Ausnahme eines Restes $OR_5$, in welchem $R_5$ Wasserstoff bedeutet, überführbaren Rest bedeutet, und $R_1$, alk, $n_1$, $n_2$, $R_2$, $R_3$ und $R_4$ die unter der Formel I angegebene Bedeutung haben, den Rest $A^g$ in den Rest —CO—$A_1$ überführt, und

h) gewünschtenfalls eine Verbindung der allgemeinen Formel I, in der $n_1$ null oder eins bedeutet, zur entsprechenden Verbindung oxidiert, in der $n_1$ eins oder zwei bedeutet, oder

i) gewünschtenfalls eine Verbindung der allgemeinen Formel I in der $n_1$ zwei oder eins bedeutet, zur entsprechenden Verbindung reduziert, in der $n_1$ eins oder null bedeutet, und/oder gewünschtenfalls eine erhaltene Verbindung der allgemeinen Formel I in anderer an sich bekannter Weise in eine andere Verbindung der allgemeinen Formel I, überführt, und/oder eine als Racemat erhaltene Verbindung der allgemeinen Formel I in die optischen Antipoden zerlegt, und/oder eine erhaltene Verbindung der allgemeinen Formel I, in welcher A $OR_5$ und darin $R_5$ Wasserstoff bedeutet, in ein Salz mit einer Base überführt oder eine solche Verbindung aus einem erhaltenen Salz freisetzt, oder eine erhaltene Verbindung der allgemeinen Formel I von basischem Charakter in ein Säureadditonssalz überführt oder eine solche Verbindung aus einem erhaltenen Salz freisetzt.

In den Ausgangsstoffen der allgemeinen Formel III ist Hal vorzugsweise Chlor oder Brom, kann aber auch Fluor oder Jod sein, wobei auch zwei unter sich verschiedene Halogenatome vorliegen können. Die Umsetzungen gemäss Verfahren a) werden vorzugsweise in unter den Reaktionsbedingungen inerten organischen Lösungsmitteln, beispielsweise in ätherartigen, wie z. B. Dibutyläther, 1,2-Dimethoxyäthan, Diäthylenglykoldimethyläther, Tetrahydrofuran oder Dioxan ; alkoholartigen, wie z. B. Methanol, Aethanol, Isopropanol, Butanol, 2-Methoxy-äthanol oder 2-Aethoxyäthanol ; oder amidartigen, wie z. B. Dimethylformamid oder N,N,N',N',N'',N''-Hexamethylphosphorsäuretriamid ; oder in Kohlenwasserstoffen, wie z. B. Petroläther, Cyclohexan, Benzol oder Toluol durchgeführt. Umsetzungen mit freien Verbindungen der allgemeinen Formel II wie auch mit freien Halogenessigsäuren der allgemeinen Formel III werden vorzugsweise in Gegenwart von basischen Stoffen durchgeführt. Als solche können beispielsweise organische oder anorganische Derivate von Alkali- oder Erdalkalimetallen, als organische Derivate z. B. Alkalimetall- oder Erdalkalimetallalkoxide, wie Natrium- oder Lithiummethoxid, -äthoxid, -n-butoxid oder -tert.-butoxid, bzw. Barium-methoxid, oder als anorganische Derivate z. B. entsprechende Hydroxide, wie Natrium, Kalium- oder Calciumhydroxid, oder Carbonate, wie z. B. Natrium- oder Kaliumcarbonat, verwendet werden. Insbesondere Carbonate können in grösserem, z. B. bis fünffachem Ueberschuss eingesetzt werden. Bei Verwendung von Carbonaten können auch noch weitere organische Lösungsmittel, wie Niederalkanone, z. B. Aceton oder 2-Butanon, als genügend inert in Betracht kommen.

Als Salze von Verbindungen der allgemeinen Formel II und von gegebenenfalls verwendeten, unter die allgemeine Formel III fallenden Dihalogenessigsäuren eignen sich beispielsweise entsprechende Alkalimetallsalze oder Erdalkalimetallsalze. Die Reaktionstemperaturen liegen beispielsweise zwischen Raumtemperatur und etwa 150 °C und vorzugsweise zwischen etwa 70 und 120 °C.

Eine Anzahl Ausgangsstoffe der allgemeinen Formeln II und III sind bekannt und weitere analog zu den bekannten Verbindungen herstellbar. So kann man beispielsweise Ausgangsstoffe der allgemeinen Formel II herstellen, indem man zunächst 1,2-Dimethoxybenzol, das entsprechend der Definition für $R_2$, $R_3$ und $R_4$ substituiert sein kann, mit einem einer Sulfonsäure der allgemeinen Formel VI in Polyphosphorsäure in der Wärme, z. B. bei ca. 100-110 °C zum entsprechenden Sulfon kondensiert oder mit einem von einer solchen Sulfonsäure oder auch einer entsprechenden Sulfonsäure abgeleiteten Acylhalogenid nach Friedel-Crafts, z. B. mittels Aluminiumchlorid in 1,2-Dichloräthan bei Raumtemperatur, ebenfalls zum entsprechenden Sulfon bzw. zum entsprechenden Sulfoxid kondensiert und in diesem die beiden Methoxygruppen in an sich bekannter Weise, z. B. durch Erhitzen mit Pyridin-hydrochlorid oder mit 48 %-iger Bromwasserstoffsäure in Essigsäure, spaltet. Falls man Ausgangsstoffe der Formel II benötigt, in welchen alk ein Niederalkylidenrest, insbesondere aber ein 1-Niederalkyl-alkyliden wie der 1-Methyläthylidenrest ist, kann man nach der Friedel-Crafts-Kondensation und vor der Spaltung der Methoxygruppen in eine Sulfonverbindung, in der alk Methylen oder Nideralkyliden ist, ein oder vorzugsweise zwei Niederalkyl, bzw. ein Niederalkyl, insbesondere Methyl, durch Umsetzung mit einem Niederalkylhalogenid, wie Methyljodid, z. B. in einem zweiphasischen System aus einer konzentrierten wässrigen Lösung von Tetrabutylammoniumhydroxid oder -bromid und einem inerten organischen Lösungsmittel, z. B. Methylenchlorid, einführen. Ein direkt zu Dihydroxysulfonen der allgemeinen Formel II führendes und deshalb besonders vorteilhaftes Verfahren ist die Umsetzung von unter die allgemeinen Formel VI fallenden Sulfinsäuren als Natriumsalze mit gegebenenfalls entsprechend der Definition für $R_2$,

$R_3$ und $R_4$ substituierten 1,2-Benzendiolen in Gegenwart von Kalium-hexacyanoferrat-(III) und Natriumacetat in Wasser.

Ausgangstoffe der allgemeinen Formel II, in denen $n_1$ Null ist, können beispielsweise analog Verfahren d) hergestellt werden.

Zur Durchführung des Verfahrens b) erhitzt man beispielsweise einen Ausgangsstoff der allgemeinen Formel IV, in welchem $A^b$ Carboxy bedeutet und A wie auch $R_1$, $R_2$, $R_3$ und $R_4$ die unter der Formel I angegebene Bedeutung haben, in An- oder Abwesenheit eines Katalysators, z. B. Kupferpulver, und/oder eines Lösungs- oder Verdünnungsmittels, wie z. B. o-Dichlorbenzol oder 1,2,3,4-Tetrahydronaphthalin, bis die mindestens annähernd äquimolare Menge Kohlendioxid freigesetzt ist. Ausgangsstoffe der allgemeinen Formel IV, in denen $A^b$ Carboxy und A $OR_5$ und darin $R_5$ Wasserstoff bedeutet, werden beispielsweise durch Hydrolyse von entsprechenden Verbindungen, in denen A $OR_5$ und darin $R_5$ Niederalkyl ist und als bzw. anstelle von $A^b$ Niederalkoxycarbonyl oder Cyano steht, in saurem oder alkalischem Medium, beispielsweise durch Erwärmen mit einer starken Mineralsäure in wässrigem oder wässrig-organischem, z. B. wässrig-niederalkanolischem Medium, bzw. mit der mindestens doppeltmolaren Menge eines Alkalimetallhydroxids, insbesondere Natrium- oder Kaliumhydroxid, z. B. in einem Niederalkanol, wie Methanol, Aethanol, Isopropanol oder n-Butanol, oder in einem Niederalkandiol oder Monoalkyläther desselben, z. B. Aethylenglykol, 2-Methoxyäthanol oder 2-Aethoxyäthanol, hergestellt, wobei den genannten Lösungsmitteln gegebenenfalls Wasser im Volumenverhältnis von Lösungsmittel zu Wasser von ca. 10 : 1 bis 1 : 2 zugefügt wird. Ferner kann als Reaktionsmedium auch Wasser oder z. B. ein Gemisch von Wasser mit wasserlöslichen, ätherartigen Lösungsmitteln, wie Dioxan oder Tetrahydrofuran, verwendet werden.

Erfolgt die Hydrolyse in einer wasserhaltigen Mineralsäure, so kann die verfahrensgemässe Decarboxylierung daran anschliessend, d. h. im gleichen Medium und Arbeitsgang durchgeführt werden.

Ausgangsstoffe der allgemeinen Formel IV, in denen $A^b$ Carboxy und A ein Rest entsprechend der Definition unter der Formel I ist mit Ausnahme eines Restes $OR_5$, in welchem $R_5$ Wasserstoff bedeutet, kann man z. B. durch partielle Hydrolyse in alkalischem Medium aus entsprechenden Verbindung mit Niederalkoxycarbonyl als Rest $A^b$ unter Verwendung der etwa äquimolaren Menge eines Alkalimetallhydroxids anstelle der mindestens doppeltmolaren Menge herstellen. Eine andere Möglichkeit zur Herstellung solcher Ausgangsstoffe der allgemeinen Formel IV besteht in der Hydrogenolyse von entsprechenden Verbindungen, in denen sich anstelle von $A^b$ Benzyloxycarbonyl befindet.

Die Desalkoxycarbonylierung oder Desacetylierung von entsprechenden Ausgangsstoffen der allgemeinen Formel IV, d. h. solchen, in denen $A^b$ Niederalkoxycarbonyl oder Acetyl und A einen definitionsgemässen Rest mit Ausnahme eines Restes $OR_5$, in welchem $R_5$ Wasserstoff ist, bedeutet, erfolgt beispielsweise durch Umsetzung mit der etwa äquimolaren Menge eines Alkalimetallniederalkoxides in einem wasserfreien Niederalkanol, wobei, falls A ein Rest $OR_5$ ist, in welchem $R_5$ Niederalkyl bedeutet, vorzugsweise dasselbe niedere Alkanol, z. B. Methanol, Aethanol, n-Butanol, als Komponente des Ausgangsesters und des Niederalkoxids wie auch als Reaktionsmedium gewählt wird. Man kann aber auch durch Verwendung eines relativ höhersiedenenden, mit dem als Esterkomponente vorliegenden Niederalkanol nicht identischen Alkanols als Reaktionsmedium und Abdestillieren eines Teiles davon gleichzeitig mit der definitionsgemässen Umsetzung eine Umesterung durchführen oder aber eine partielle Umesterung in Kauf nehmen, falls der als Reaktionprodukt anfallende Ester der allgemeinen Formel I nicht direkt als Wirkstoff verwendet, sondern zur entsprechenden Säure hydrolysiert werden soll. Ferner kann als Reaktionsmedium anstelle eines niederen Alkanols z. B. auch ein inertes organisches Lösungsmittel, wie z. B. Benzol oder Toluol, verwendet werden. Die definitionsgemässe Umsetzung wird bei Raumtemperatur oder erhöhter Temperatur, z. B. bei Siedetemperatur des verwendeten Reaktionsmediums, durchgeführt. Gegebenenfalls kann der entstandene Ester der allgemeinen Formel I, wie bereits im Zusammenhang mit der Umesterung erwähnt, im gleichen Arbeitsgang zur entsprechenden Säure hydrolysiert werden, wenn man dem Reaktionsmedium Wasser zufügt.

Die Ausgangsstoffe der allgemeinen Formel IV, in den $A^b$ Niederalkoxycarbonyl oder Acetyl ist, sowie die weiter oben genannten Vorprodukte zu Verbindungen der allgemeinen Formel IV mit Carboxy als Rest $A^b$, die als bzw. anstelle von $A^b$ Niederalkoxycarbonyl bzw. Cyano enthalten, können analog Verfahren a) durch Umsetzung von Verbindungen der allgemeinen Formel II mit geminalen Dihalogenverbindungen, die sich von solchen der allgemeinen Formel III durch das Vorliegen von Niederalkoxycarbonyl, Acetyl oder Cyano anstelle des neben beiden Halogenatomen befindlichen Wasserstoffatoms unterscheiden, in Gegenwart einer Base hergestellt werden.

Gemäss Verfahren c) kann man beispielsweise freie Sulfonsäuren der allgemeinen Formel VI in Polyphosphorsäure oder Pyrophosphorsäure unter Erwärmen, z. B. bei etwa 80 bis etwa 120 °C, insbesondere bei etwa 100 bis 110 °C mit Verbindungen der allgemeinen Formel V kondensieren. Weiter kann man Anhydride von Verbindungen der allgemeinen Formel VI, beispielsweise deren Halogenide, wie Chloride oder Bromide, ferner z. B. deren symmetrische Anhydride, in Gegenwart üblicher Friedel-Crafts-Kondensationsmittel, wie Aluminiumchlorid oder Zinn(IV)- chlorid, ferner z. B. Zinkchlorid, weiter z. B. in konz. Schwefelsäure, Phosphorsäure, Polyphosphorsäure oder Pyrophosphorsäure mit Verbindungen der allgemeinen Formel V kondensieren. Die vorgenannten Säuren werden bevorzugt verwendet, wenn man als Ausgangsstoff ein symmetrisches Anhydrid einer Sulfonsäure der allgemeinen Formel VI einsetzt. Die Umsetzungen werden bei Verwendung von Kondensationsmitteln vorzugsweise in einem

Lösungsmittel vorgenommen. Als solche kann man beispielsweise Halogenkohlenwasserstoffe, wie 1,2-Dichloräthan, Tetrachlorkohlenstoff, Methylenchlorid, o-Dichlorbenzol, weiter z. B. aliphatische oder cycloaliphatische Kohlenwasserstoffe, wie Heptan oder Cyclohexan, Nitrokohlenwasserstoffe, wie Nitromethan, Nitrocyclohexan oder Nitrobenzol und ferner unter milden Bedingungen auch Schwefelkohlenstoff verwenden. Die Reaktionstemperatur liegt zwischen etwa —20° und +80 °C, vorzugsweise zwischen etwa 0° und Raumtemperatur.

Die Ausgangsstoffe der allgemeinen Formel V können ihrerseits analog Verfahren a) aus gegebenenfalls entsprechend der Definition für $R_2$, $R_3$ und $R_4$ substituiertem 1,2-Benzylendiol, wie z. B. 4-Methyl-1,2-benzendiol mit Dihalogenessigsäuren oder deren funktionellen Derivaten entsprechend der allgemeinen Formel III hergestellt werden. Von den als zweite Reaktionskomponenten benötigten Sulfon- oder Sulfinsäuren der allgemeinen Formel IV und ihren funktionellen Derivaten von Verbindungen der allgemeinen Formel VI sind manche bekannt und weitere analog zu den bekannten herstellbar.

Umsetzungen von Verbindungen der allgemeinen Formel VII, wie z. B. von gegebenenfalls substituierten Alkyl-, Alkenyl-, Alkinylhalogeniden oder Aralkyl- insbesondere Benzylhalogeniden, mit Salzen, insbesondere Alkalimetallsalzen, z. B. Natrium- oder Kaliumsalzen, von Verbindungen der allgemeinen Formel IX werden beispielsweise in Wasser, wässrig-organischem oder organischen Medium, z. B. in einem Niederalkanol, wie Methanol oder Aethanol, vorzugsweise in der Wärme, d. h. z. B. bei etwa 70 bis etwa 160°, vor allem bei etwa 80°-100° bzw. der Siedetemperatur des Mediums oder im geschlossenen Gefäss auch oberhalb dieser durchgeführt. Das Salz der Verbindung der allgemeinen Formel VII kann auch in situ durch Zusatz von Alkalihydroxiden oder Alkalimetallsalzen schwacher Säuren, z. B. von Natriumacetat, gebildet werden.

Die Diazoniumsalze der allgemeinen Formel VIII werden in üblicher Weise aus den entsprechenden primären Aminen, in denen $n_1$ vorzugsweise Null und $R_1$ ein aromatischer Rest ist, z. B. in wässriger Lösung durch Behandlung mit Salzsäure und Natriumnitrit hergestellt und unter Erwärmen und Zusatz von Natronlauge mit gegebenenfalls in situ gebildeten Salzen von Verbindungen der allgemeinen Formel IX umgesetzt. Ausgangsstoffe der allgemeinen Formeln VII und VIII sind bekannt bzw. analog zu bekannten herstellbar. Zu Ausgangsstoffen der allgemeinen Formel IX gelangt man z. B. durch Umsetzung von entsprechenden schwefelfreien Benzodioxolderivaten der allgemeinen Formel V mit Chlorsulfonsäure zu entsprechenden Chlorsulfonylverbindungen und an sich bekannte Reduktion zu den entsprechenden Sulfinsäuren, z. B. mit Natriumsulfit, bzw. zu Mercaptanen, z. B. mit Zinkstaub und konz. Salzsäure in Diäthyläther oder einem ätherartigen Lösungsmittel.

Im Verfahren gemäss e) verwendet man als Ausgangsstoffe der allgemeinen Formel Xa z. B. Butyllithium, Phenyllithium oder 4-Methoxyphenyllithium, vgl. z. B. A. Schönberg et al. Chem. Ber. 66, 237-244 (1933), oder auch entsprechende Grignardverbindungen, bzw. als Ausgangsstoff der allgemeinen Formel Xb z. B. Diphenylcadmium, Bis-(4-chlorphenyl)-cadmium oder Bis-(4-methoxyphenyl)-cadmium, vgl. z. B. H. R. Henze et al, J. Chem. Soc. 1957, 1410-1413 oder Diphenylquecksilber, und arbeitet in beiden Fällen z. B. in abs. Diäthyläther oder einem anderen ätherartigen Lösungsmittel. Sulfochloride der allgemeinen Formel XIa wurden bereits erwähnt als Vorprodukte zu Verbindungen der allgemeinen Formel IX, ebenso die daraus durch Reduktion erhältlichen, entsprechenden Sulfinsäuren und Mercaptane. Aus ersteren erhält man z. B. mit Thionylchlorid Sulfinylchloride der allgemeinen Formel XIa, bzw. aus letzteren durch an sich bekannten Oxidation Disulfide der allgemeinen Formel XIb.

Bei der Herstellung von Verbindung der allgemeinen Formel I, in denen A den Rest $OR_5$ und darin $R_5$ Wasserstoff bedeutet, gemäss Verfahren f) kann die Umwandlung einer Gruppe $A^f$ in die Carboxygruppe in an sich bekannter Weise, insbesondere durch Hydrolyse in alkalischem oder sauren Medium erfolgen, wobei man im ersteren Fall auch direkt ein Salz erhalten kann. Ausgangsstoffe für die Hydrolyse sind zunächst solche Verbindungen der allgemeinen Formel I, in denen A kein Rest $OR_5$ ist, in dem $R_5$ Wasserstoff bedeutet, insbesondere leicht hydrolysierbare unter diesen Verbindungen, wie z. B. die Niederalkylester, im weiteren aber auch andere funktionelle Derivate der als Enstoffe gewünschten Carbonsäuren, wie z. B. Nitrile und Imidoester, insbesondere Imidoniederalkylester, von unter die allgemeine Formel I fallenden Carbonsaüren. Die Hydrolyse erfolgt beispielsweise in niederalkanolischen oder wässrig-niederalkanolischen Alkalihydroxidlösungen bei Raumtemperatur bis etwa 100 °C bzw. Siedetemperatur des Reaktionsmediums Niederalkylester, wie Methyl- oder Aethylester und andere leicht spaltbare Ester von unter die allgemeine Formel I fallenden Carbonsäuren können unter noch milderen Bedingungen, z. B. in Gegenwart von Kalium- oder Natriumcarbonat bei Raumtemperatur oder nötigenfalls schwach erhöhten Temperaturen von z. B. 40 °C, in wässerig-organischem Medium, z. B. in dem beim Versetzen des bei der Umsetzung gemäss a) erhaltenen Rekationsgemisches in einem mit Wasser mischbaren Lösungsmittel, wie z. B. 1,2-Dimethoxyäthan, mit Wasser hydrolysiert werden. Aus den dabei zunächst erhaltenen Alkalimetallsalzlösungen der unter die allgemeine Formel I fallenden Carbonsäuren kann man entweder durch Einengen und Abfiltrieren bzw. Eindampfen und Umkristallisieren direkt das entsprechende reine Alkalisalz gewinnen oder zunächst die Carbonsäure freisetzen, anschliessend z. B. durch Umkristallisation reinigen und gewünschtenfalls wiederum in ein Salz mit einer geeigneten anorganischen oder organischen Base überführen. Funktionelle Derivate der unter die allgemeine Formel I fallenden Carbonsäuren lassen sich ferner auch in saurem Medium, z. B. durch Erwärmen in einer mit Wasser verdünnten, z. B. 60-70 %igen Schwefelsäure oder in niederalkanolisch-wässeriger Salzsäure, in die freie Carbonsäure der allgemeinen Formel I überführen.

Die benötigten funktionellen Derivate von Carbonsäuren, welche unter die allgemeine Formel I fallen, werden z. B. gemäss einem der vorgenannten Verfahren, und andere funktionelle Derivate, wie z. B. Nitrole, analog zu diesen Verfahren hergestellt.

Ausgangsstoffe der allgemeinen Formel XIV sind entsprechend dem in ihnen enthaltenden Rest A beispielsweise Carbonsäuren, Carbonsäurehalogenide oder -anhydride, insbesondere gemischte Anhydride, weiter aktivierte Ester, z. B. Cyanomethylester, sowie auch Niederalkylester, die, gegebenenfalls in Gegenwart von Kondensationsmitteln, mit Hydroxyverbindungen der allgemeinen Formel XV

$R_5$—OH (XV)

oder Ammoniak bzw. Aminen der allgemeinen Formel XVI

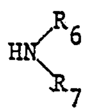

(XVI)

in welchen Formeln $R_5$ bzw. $R_6$ und $R_7$ die unter der Formel I angegebene Bedeutung haben, umgesetzt werden können, oder Salze, insbesondere Alkalimetall- oder Erdalkalimetallsalze der freien Carbonsäuren, die mit reaktionsfähigen Estern von Hydroxyverbindungen der allgemeinen Formel XV, wie Halogeniden oder organischen Sulfosnäureestern, z. B. Niederalkansulfonsäure- oder Arensulfonsäureestern, wie Methansulfonsäure- bzw. p-Toluolsulfonsäureestern, oder auch mit von Aminen der allgemeinen Formel XVI, deren Reste $R_6$ und $R_7$ von Wasserstoff verschieden sind, abgeleiteten Carbaminsäurehalogeniden, insbesondere -chloriden, umgesetzt werden können ; weiter z. B. die zu Estern, insbesondere Niederalkylestern bzw. zu unsubstituierten Amiden hydrolysierbaren Imidoester, insbesondere Imidoniederalkylester, bzw. Nitrile. Freie Carbonsäuren können z. B. auch mit Diazoniederalkanen zu Niederalkylestern oder mit Isocyanaten, die von unter die allgemeine Formel XVI fallenden primären Aminen abgeleitet sind, zu N-monosubstituierten Amiden, umgesetzt werden.

Die Umsetzungen von freien Carbonsäuren mit Hydroxyverbindungen der allgemeinen Formel XV erfolgen vorteilhaft in Gegenwart eines sauren wasserabspaltenden Katalysators, wie einer Protonensäure, z. B. von Chlor- oder Bromwasserstoff-, Schwefel-, Phosphor- oder Borsäure, Benzolsulfon- oder Toluolsulfonsäure, oder einer Lewissäure, z. B. von Bortrifluorid-Aetherat, in einem Ueberschuss der eingesetzten Hydroxyverbindung und/oder in einem inerten Lösungsmittel, z. B. in einem Kohlenwasserstoff der Benzolreihe, wie Benzol oder Toluol, einem halogenierten Kohlenwasserstoff wie Chloroform, Methylenchlorid oder Chlorbenzol, oder in einem ätherartigen Lösungsmittel, wie Tetrahydrofuran, erforderlichenfalls unter destillativer, z. B. azeotroper, Entfernung des bei der Reaktion freigesetzten Wassers. Weiter kann man die Umsetzungen auch in Gegenwart anderer wasserbindender Kondensationsmittel, z. B. von durch Kohlenwasserstoffreste substituierten Carbodiimiden, wie N,N'-Diäthyl-, N,N'-Dicyclohexyl- oder N-Aethyl-N'-(3-dimethylaminopropyl)-carbodiimid, in inerten organischen Lösungsmitteln, z. B. den vorgenannten, durchführen. Halogenide und gemischte Anhydride werden beispielsweise in Gegenwart säurebindender Mittel, z. B. organischer, insbesondere tertiärer Stickstoffbasen, wie z. B. Triäthylamin, Aethyldiisopropylamin oder Pyridin, oder auch anorganischer Basen, z. B. von Alkalimetall- oder Erdalkalimetallhydroxiden oder -carbonaten, wie Natrium-, Kalium- oder Calciumhydroxid bzw. -carbonat, in inerten organischen Lösungsmitteln, z. B. den obengenannten, und nötigenfalls unter Erwärmen umgesetzt. Die Umsetzungen von reaktionsfähigen Estern von Carbonsäuren der allgemeinen Formel I, z. B. den Cyanomethylestern, mit Hydroxyverbindungen der allgemeinen Formel XV werden beispielsweise in einem gegenüber den Reaktionsteilnehmern inerten Lösungsmittel, z. B. in einem Kohlenwasserstoff wie Toluol oder Xylol, einem ätherartigen Lösungsmittel, wie Tetrahydrofuran oder Dioxan, oder bei mässigen Temperaturen auch einem Ester, wie Aethylacetat, im Temperaturbereich von etwa 0 °C bis etwa 120 °C, vorzugsweise bei Raumtemperatur bis etwa 60 °C durchgeführt. Zu Umesterungen von Niederalkylestern von Carbonsäuren der allgemeinen Formel I verwendet man vorzugsweise Hydroxyverbindungen der allgemeinen Formel XV mit deutlich über demjenigen des veresterten Niederalkanols liegendem Siedepunkt und führt die Reaktion z. B. in einem Ueberschuss der Hydroxyverbindung und/oder einem inerten organischen, vorzugsweise ebenfalls deutlich über dem Niederalkanol siedenden Lösungsmittel, vorzugsweise in Gegenwart eines Katalysators, z. B. eines Alkalimetall-niederalkoxids, wie Natrium- oder Kalium-methoxid oder -äthoxid, in der Wärme und vorzugsweise unter Abdestillieren des freigesetzten Niederalkanols durch. Die Hydrolyse von Imidoestern, insbesondere Imidoniederalkylestern, von Carbonsäuren der allgemeinen Formel I erfolgt beispielsweise mittels wasserhaltiger Mineralsäure, wie Salzsäure oder Schwefelsäure, wobei man z. B. die bei der Addition von Chlorwasserstoff an Nitrile und Umsetzung mit wasserfreien Hydroxyverbindungen der allgemeinen Formel XV insbesondere Niederalkanolen, erhaltenen Imidoester-hydrochloride nach Zusatz von Wasser direkt zu den entsprechenden Estern hydrolysieren kann, oder z. B. auch aus einem Gemisch von Nitril, Hydroxyverbindung und Schwefelsäure von geeignetem Wassergehalt ohne Isolierung des in situ entstandenen Imidoesters den entsprechenden Ester der allgemeinen Formel I erhalten kann.

Die Umsetzung von freien Carbonsäuren der allgemeinen Formel I mit Verbindungen der allgemeinen Formel XVI erfolgt beispielsweise in Gegenwart der obengenannten wasserbindenden Mittel und in den dort genannten inerten organischen Lösungsmitteln, man kann aber auch die aus den freien Carbonsaüren und den Verbindungen der allgemeinen Formel XVI zunächst gebildeten Ammoniumsalze durch Erhitzen, gegebenenfalls in einem geeigneten organischen Lösungsmittel von mittlerem oder höherem Siedepunkt, wie z. B. Xylol, Chlorbenzol oder 1,2,3,4-Tetrahydronaphthalin, und destillative, gegebenenfalls azeotropische, Entfernung des bei der Reaktion freigesetzten Wassers, in Amide der allgemeinen Formel I überführen.

Als reaktionsfähige funktionelle Derivate von Carbonsäuren der allgemeinen Formel I zur Umsetzung mit Verbindungen der allgemeinen Formel XVI und als zugehörige Kondensationsmittel und Lösungsmittel kommen im wesentlichen dieselben in Betracht, wie sie oben für Umsetzungen mit Hydroxyverbindungen der allgemeinen Formel XV angegeben wurden, mit dem Unterschied, dass man als säurebindendes Mittel und gegebenenfalls einziges Reaktionsmedium anstelle anderer, d. h. tertiärer organischer Basen auch einen Ueberschuss an der umzusetzenden Verbindung der allgemeinen Formel XVI verwenden kann. Die als weitere Möglichkeit zur Bildung N-unsubstituierter Amide erwähnte partielle Hydrolyse der entsprechenden Nitrile kann beispielesweise mittels wasserhaltiger Mineralsäuren, wie Salzsäure oder verdünnter Schwefelsäure bei Raumtemperatur oder mässig erhöhter Temperatur durchgeführt werden.

Die als Ausgangsstoffe für das Verfahren g) benötigten freien Carbonsäuren der allgemeinen Formel I sind insbesondere gemäss Verfahren a) oder b) und deren reaktionsfähige funktionelle Derivate z. B. aus den freien Carbonsäuren in an sich bekannter Weise herstellbar.

Die Oxidation gemäss h) erfolgt beispielsweise mittels Wasserstoffperoxid in einem gegenüber diesem inerten organischen oder organischwässrigen Lösungsmittel, wie gegebenenfalls Wasser enthaltender Essigsäure, z. B. in dem aus Eisessig und wässriger Wasserstoffperoxidlösung entstehenden Gemisch, bei mässig erhöhten Temperaturen zwischen etwa 60 und 100 °C, insbesondere bei etwa 80-90 °C, und mit der mehr als doppeltmolaren Menge Wasserstoffperoxid, wenn als Oxidationsprodukt eine Sulfonylverbindung, entsprechend $n_1$ = zwei erhalten werden soll. Die Oxidation von entsprechenden Thio- zu Sulfinylverbindungen, entsprechend $n_1$ = Null bzw. Eins, wird z. B. entweder nach obigem Verfahren im Temperaturbereich von etwa 20 bis 60 °C mit nötigenfalls einer bloss etwa äquimolaren Menge Wasserstoffperoxid, oder z. B. insbesondere unter Verwendung von Alkalimetall-, insbesondere Natrium- oder Kalium-perjodat, wie Natriummetaperjodat in organisch-wässrigem, z. B. niederalkanolisch-wässrigem, insbesondere äthanolisch-wässrigem Medium in der Kälte, z. B. bei 0 °C bis Raumtemperatur durchgeführt.

Gemäss Verfahren i) können z. B. Verbindungen der allgemeinen Formel I, in denen $n_1$ Eins ist, mittels Triphenylphosphin in siedender Tetrachlorkohlenstoff zu den entsprechenden Verbindungen, in denen $n_1$ Null ist, reduziert werden.

Erhaltene salzbildende Verbindungen der Formel I können in an sich bekannter Weise in Salze übergeführt werden, z. B. solche mit Hydroxy als Rest A mit entsprechenden Basen, wie z. B. Alkalimetallhydroxiden, in Salze mit Basen, oder solche von basischem Charakter in ihre Säureadditionssalze. Vorzugsweise werden pharmazeutisch annehmbare Salze hergestellt.

Erhaltene Salze können in an sich bekannter Weise in die freien Verbindungen umgewandelt werden, z. B. durch Behandeln mit einem sauren Reagens, wie einer Mineralsäure, bzw. mit einer Base, z. B. einer Alkalimetallhydroxidlösung, wie Natronlauge.

Die Verbindungen einschliesslich ihrer Salze können auch in Form ihrer Hydrate erhalten werden, oder ihre Kristalle können das zur Kristallisation verwendete Lösungsmittel einschliessen.

Infolge der engen Beziehung zwischen den neuen Verbindungen der allgemeinen Formel I, in welchen A Hydroxy bedeutet, in freier Form und in Form ihrer Salze mit Basen, sowie von solchen Verbindungen, deren Rest $R_1$ basischen Charakter aufweist, in freier Form oder in Form von Säureadditionssalzen, sind im vorausgegangenen und nachfolgend unter den freien Verbindungen oder ihren Salzen sinn- und zweckmässig gegebenenfalls auch die entsprechenden Salze bzw. freien Verbindungen zu verstehen.

Die neuen Verbindungen können, je nach der Anzahl der Asymmetriezentren sowie auch nach der Wahl der Ausgangsstoffe und Arbeitsweisen, in Form von Racematen oder Racematgemischen (Diastereomerengemischen) oder gegebenenfalls auch als reine Antipoden erhalten werden.

Erhaltene Racematgemische können auf Grund der physikalisch-chemischen Unterschiede der Bestandteile in bekannter Weise in die reinen Racemate bzw. Diastereomeren aufgetrennt werden, beispielsweise durch Chromatographie und/oder fraktionierte Kristallisation. Erhaltene Racemate lassen sich ferner nach bekannten Methoden in die optischen Antipoden zerlegen, beispielsweise durch Umkristallisation aus einem optisch aktiven Lösungsmittel, mit Hilfe von Mikroorganismen oder durch Umsetzung eines sauren Endstoffes der allgemeinen Formel I mit einer mit der racemischen Säure Salze bildenden optisch aktiven Base, bzw. Umsetzung eines basischen Endstoffes der allgemeinen Formel I mit einer optisch aktiven Säure, und Trennung der auf diese Weise erhaltenen Salze, z. B. auf Grund ihrer verschiedenen Löslichkeiten, in die Diastereomeren, aus denen die Antipoden durch Einwirkung geeigneter Mittel freigesetzt werden können. Vorteilhaft isoliert man den wirksameren der beiden Antipoden.

Die Erfindung betrifft auch diejenigen Ausführungsformen des Verfahrens, nach denen man von

einer auf irgendeiner Stufe des Verfahrens als Zwischenprodukt erhältlichen Verbindung ausgeht und die fehlenden Schritte durchführt oder einen Ausgangsstoff in Form eines Salzes und/oder Racemates bzw. Antipoden verwendet oder insbesondere unter den Reaktionsbedingungen bildet.

Bei den Verfahren der vorliegenden Erfindung werden vorzugsweise solche Ausgangsstoffe verwendet, welche zu den eingangs als besonders wertvoll geschilderten Verbindungen führen. Neue Ausgangsstoffe und Verfahren zu ihrer Herstellung bilden ebenfalls einen Gegenstand der vorliegenden Erfindung.

Die Erfindung betrifft im weiteren pharmazeutische Präparate, die Verbindungen der allgemeinen Formel I als Wirkstoffe enthalten, sowie Verfahren zu ihrer Herstellung.

Bei den erfindungsgemässen pharmazeutischen Präparaten handelt es sich um solche zur enteralen, wie oralen oder rektalen, sowie zur parenteralen Verabreichung an Warmblüter. Die Dosierung des Wirkstoffs, der alleine oder zusammen mit dem üblichen Träger- und Hilfsmaterial appliziert wird, hängt von der Warmblüterspezies, deren Alter und dem individuellen Zustand sowie der Applikationsweise ab. Die täglichen Dosen bewegen sich zwischen 0,15 und 20 mg/kg für Mammalien und liegen für solche von etwa 70 kg Gewicht je nach individuellem Zustand und Alter vorzugsweise zwischen 10 und 600 mg, insbesondere zwischen 25 und 300 mg. Entsprechende orale Doseneinheitsformen, z. B. Dragées oder Tabletten oder Kapseln, enthalten vorzugsweise 5 bis 150 mg, insbesondere 10 bis 100 mg eines erfingungsgemässen Wirkstoffes, d. h. einer Verbindung der allgemeinen Formel I oder eines pharmazeutisch annehmbaren Salzes einer zur Salzbildung befähigten Verbindung der allgemeinen Formel I, zusammen mit pharmazeutischen Trägerstoffen.

Die pharmazeutischen Präparate der vorliegenden Erfindung werden in an sich bekannter Weise, z. B. mittels konventioneller Misch-, Granulier-, Dragier-, Lösungs- oder Lyophilisierungsverfahren hergestellt. So kann man pharmazeutische Präparate zur oralen Anwendung erhalten, indem man den Wirkstoff mit festen Trägerstoffen kombiniert, ein erhaltenes Gemisch gegebenenfalls granuliert, und das Gemisch bzw. Granulat, wenn erwünscht oder notwendig, nach Zugabe von geeigneten Hilfsstoffen, zu Tabletten oder Dragée-Kernen verarbeitet.

Geeignete Trägerstoffe sind insbesondere Füllstoffe, wie Zucker, z. B. Lactose, Saccharose, Mannit oder Sorbit, Cellulosepräparate und/oder Calciumphosphate, z. B. Tricalciumphosphat oder Calciumhydrogenphosphat, ferner Bindemittel, wie Stärkekleister und Verwendung z. B. von Mais-, Weizen-, Reis- oder Kartoffelstärke, Gelatine, Traganth, Methylcellulose und/oder Polyvinylpyrrolidon, und/oder, wenn erwünscht, Sprengmittel, wie die obgenannten Stärken, ferner Carboxymethylstärke, quervernetztes Polyvinylpyrrolidon, Agar, Alginsäure oder ein Salz davon, wie Natriumalginat. Hilfsmittel sind in erster Linie Fliessregulier- und Schmiermittel, z. B. Kieselsäure, Talk, Stearinsäure oder Salze davon, wie Magnesium- oder Calciumstearat, und/oder Polyäthylenglykol. Dragée-Kerne werden mit geeigneten, gegebenenfalls Magensaft-resistenten Ueberzügen versehen, wobei man u. a. konzentrierte Zuckerlösungen, welche gegebenenfalls arabischen Gummi, Talk, Polyvinylpyrrolidon, Polyäthylenglykol und/oder Titandioxid enthalten. Lacklösungen in geeigneten organischen Lösungsmitteln oder Lösungsmittelgemischen oder, zur Herstellung von Magensaft-resistenten Ueberzügen, Lösungen von geeigneten Cellulosepräparaten, wie Acetylcellulosephthalat oder Hydroxypropylmethylcellulosephthalat, verwendet. Den Tabletten oder Dragée-Ueberzügen können Farbstoffe oder Pigmente, z. B. zur Identifizierung oder zur Kennzeichnung verschiedener Wirkstoffdosen, beigefügt werden.

Weitere, oral anwendbare pharmazeutische Präparate sind Steckkapseln aus Gelatine, sowie weiche, geschlossene Kapseln aus Gelatine und einem Weichmacher, wie Glycerin oder Sorbitol. Die Steckkapseln können den Wirkstoff in Form eines Granulats, z. B. im Gemisch mit Füllstoffen, wie Lactose, Bindemitteln, wie Stärken, und/oder Gleitmitteln, wie Talk oder Magnesiumstearat, und gegebenenfalls von Stabilisatoren, enthalten. In weichen Kapseln ist der Wirkstoff vorzugsweise in geeigneten Flüssigkeiten, wie fetten Oelen, Paraffinöl oder flüssigen Polyäthylenglykolen, gelöst oder suspendiert, wobei ebenfalls Stabilisatoren zugefügt sein können.

Als rektal anwendbare pharmazeutische Präparate kommen z. B. Suppositorien in Betracht, welche aus einer Kombination des Wirkstoffs mit einer Suppositoriengrundmasse bestehen. Als Suppositoriengrundmasse eignen sich z. B. natürliche oder synthetische Triglyceride, Paraffinkohlenwasserstoffe, Polyäthylenglykole oder höhere Alkanole. Ferner können auch Gelatine-Rektalkapseln verwendet werden, die eine Kombination des Wirkstoffes mit einer Grundmasse enthalten ; als Grundmassenstoffe kommen z. B. flüssige Triglyceride, Polyathylenglykole oder Paraffinkohlenwasserstoffe in Frage.

Zur parenteralen Verabreichung eignen sich in erster Linie wässrige Lösungen eines Wirkstoffes in wasserlöslicher Form, z. B. eines wasserlöslichen Salzes, ferner Suspensionen des Wirkstoffes, wie entsprechende ölige Injektionssuspensionen, wobei man geeignete lipophile Lösungsmittel oder Vehikel, wie fette Oele, z. B. Sesamöl, oder synthetische Fettsäureester, z. B. Aethyloleat oder Triglyceride, verwendet, oder wässrige Injektionssuspensionen, welche viskositätserhöhende Stoffe, z. B. Natriumcarboxymethylcellulose, Sorbit und/oder Dextran und gegebenenfalls Stabilisatoren enthalten.

Die Erfindung betrifft ebenfalls die Verwendung der neuen Verbindungen der Formel I und der pharmazeutisch annehmbaren Salze von solchen als pharmakologisch aktive Verbindungen, insbesondere als Diuretika mit urikosurischer Zusatzwirkung, vorzugsweise in Form von pharmazeutischen Präparaten in einem Verfahren zur prophylaktischen und/oder therapeutischen Behandlung des tierischen oder menschlichen Körpers, insbesondere zur Behandlung von Oedemen und/oder der Hypertension.

Die nachfolgenden Beispiele illustrieren die oben beschriebene Erfindung; sie sollen jedoch diese in ihrem Umfang in keiner Weise einschränken. Temperaturen werden in Celsiusgraden angegeben.

Beispiel 1

Zu einer Suspension von 10,6 g (40 mMol) 4-Methyl-5-phenyl-sulfonyl-1,2-benzendiol in 65 ml kaltem 1,2-Dimethoxyäthan gibt man 27,7 g (200 mMol) wasserfreies Kaliumcarbonat. Unter starkem Rühren tropft man innerhalb 30 Minuten eine Lösung von 7,85 g (50 mMol) Dichloressigsäure-äthylester in 5 ml 1,2-Dimethoxyäthan zu. Man rührt das Gemisch eine Stunde bei Raumtemperatur weiter und kocht es dann unter Rühren und unter Rückfluss während 3 Stunden. Dann wird es auf 30° abgekühlt, mit Wasser versetzt, während 30 Minuten gerührt und mit Salzsäure auf pH 1-2 gestellt. Im Rotationsverdampfer wird ein Teil des 1,2-Dimethoxyäthans entfernt und die zurückbleibende Lösung dreimal mit Aethylacetat extrahiert. Die vereinigten Aethylacetatlösungen werden mit Wasser, und dann mit gesättigter Natriumchloridlösung gewaschen, über Natriumsulfat getrocknet, mit Aktivkohle behandelt und eingedampft. Das erhaltene gelb-grüne Oel wird aus Aethylacetat-Hexan kristallisiert. Die sandfarbenen Kristalle werden in Aethylacetat gelöst und wiederum mit Aktivkohle behandelt. Man entfernt das Lösungsmittel im Rotationsverdampfer. Das zurückbleibende gelbe Oel wird bei 0° aus Methylenchlorid kristallisiert, wobei die 5-Methyl-6-phenylsulfonyl-1,3-benzodioxol-2-carbonsäure vom Smp. 169-171° erhalten wird.

Der Ausgangsstoff kann wie folgt hergestellt werden

a) Zu einer Lösung von 16,4 g (100 mMol) Benzolsulfinsäure-Natriumsalz und 13,6 g (110 mMol) 4-Methyl-1,2-benzendiol in 150 ml Wasser tropft man unter Stickstoff bei Raumtemperatur innerhalb 30 Minuten eine Lösung von 50 g (152 mMol) Kalium-hexacyano-ferrat-(III) und 95 g Natriumacetat-trihydrat in 200 ml Wasser. Nach 1 1/2 Stunden Rühren wird das Reaktionsgemisch angesäuert und mit Aethylacetat mehrmals extrahiert. Die vereinigten organischen Phasen werden mit Wasser und gesättigter Natriumchloridlösung gewaschen, über Natriumsulfat getrocknet und eingedampft. Der Rückstand wird aus Aether-Hexan umkristallisiert, wobei das 4-Methyl-5-phenylsulfonyl)-1,2-benzendiol vom Smp. 145-149° erhalten wird.

Beispiel 2

Ein Gemisch von 20,8 g (100 mMol) 5-Methyl-1,3-benzodioxol-2-carbonsäure-äthylester, 18.2 g (105 mMol) Benzolsulfonsäure-Natriumsalz, 200 g Polyphosphorsäure und 85 ml Methansulfonsäure wird bei 25° unter Stickstoff während vier Tagen kräftig gerührt. Dann versetzt man das Reaktionsgemisch unter Eiskühlung mit 500 ml Eiswasser und extrahiert es viermal mit Aether. Die vereinigten organischen Phasen werden zuerst mit Wasser, dann mit einer gesättigten Natriumchloridlösung gewaschen, über Natriumsulfat getrocknet und zur Trockene eingedampft. Der zurückbleibende 5-Methyl-6-phenylsulfonyl-1,3-benzodioxol-2-carbonsäure-äthylester wird durch Säulenchromatographie an Kieselgel unter Elution mit Aethylacetat-Hexan im Verhältnis 1:1 gereinigt. Die einheitliche Substanz enthaltenden Fraktionen werden vereinigt und eingedampft. Der gelbe, ölige Rückstand kristallisiert bei Stehen. Er wird noch während zwei Stunden bei 0° mit Aether verrührt, abfiltriert, mit kaltem Aether gewaschen und getrocknet. Der in hellgelben Nadeln kristallisierende Ester schmilzt bei 89-90°.

8,6 mg (24,7 mMol) 5-Methyl-6-phenylsulfonyl-1,3-benzodioxol-2-carbonsäure-äthylester werden in 37 ml Methanol suspendiert. Dann gibt man bei Raumtemperatur langsam 37 ml 1-n. Natronlauge zu, worauf der Ausgangsstoff bald in Lösung geht. Nach einer Stunde kühlt man das Reaktionsgemisch, versetzt es mit konz. Salzsäure und extrahiert es dreimal mit Aethylacetat. Die vereinigten organischen Phasen werden mit Wasser und dann mit einer gesättigten Natriumchloridlösung gewaschen, über Natriumsulfat getrocknet und eingedampft. Das zurückbleibende gelbe Oel wird mit 50 ml Methylenchlorid versetzt und bis zur Kristallisation gerührt. Die abfiltrierten Kristalle werden aus wenig 1,2-Dichloräthan umkristallisiert, wobei man die 5-Methyl-6-phenyl-sulfonyl-1,3-benzodioxol-2-carbonsäure als weisse Kristalle vom Smp. 169°-171° erhält.

Der als Ausgangsstoff verwendete 5-Methyl-1,3-benzodioxol-2-carbonsäureäthylester kann z. B. nach einem der beiden nachstehend beschriebenen Verfahren hergestellt werden :

a) eine Lösung von 38,7 g (0,3 Mol) Dichloressigsäure, 12 g (0,3 Mol) Natriumhydroxid, 40 ml Wasser und 0,6 g Aliquat wird unter Stickstoff gerührt und auf 95° erhitzt. Bei dieser Temperatur tropft man innerhalb von 2 1/2 Stunden gleichmässig eine Lösung von 24.8 g (0,2 Mol) 4-Methyl-1,2-benzendiol (Homobrenzkatechin), 100 ml Wasser und 20 g (0,5 Mol) Natriumhydroxid, die vorher unter Stickstoff hergestellt wurde, zu. Man rührt während 1 1/2 Stunden bei 95° weiter und kühlt dann auf Raumtemperatur ab. Das Reaktionsgemisch wird mit Salzsäure auf pH 8 angesäuert und zweimal mit Aether gewaschen. Die wässerige Phase wird jetzt mit konz. Salzsäure auf pH 1-2 eingestellt und mit Aether extrahiert. Die vereinigten Aetherlösungen werden mit Wasser und gesättigter Natriumchloridlösung gewaschen, über Natriumsulfat getrocknet und im Rotationsverdampfer vollständig eingedampft.

b) Die bei a) als Rückstand erhaltene rohe 5-Methyl-1,3-benzodioxol-2-carbonsäure wird in 400 ml

wasserfreiem Aethanol gelöst und mit 0,5 ml konz. Salzsäure während 2 Stunden gekocht. Die Lösung wird stark eingeengt und der entstandene rohe Ester in Aether oder Methylenchlorid gelöst. Die Lösung wird mit gesättigter Natriumbicarbonatlösung, Wasser und gesättigter Natriumchloridlösung gewaschen, über Natriumsulfat getrocknet und zur Trockene eingedampft. Das als braunes Oel zurückbleibende Rohprodukt wird unter Wasserstrahl-Vacuum destilliert wobei der 5-Methyl-1,3-benzodioxol-2-carbonsäure-äthylester bei 105°-108°/5 mbar übergeht.

c) Zu einer Suspension von 34,6 g (250 mMol) wasserfreiem Kaliumcarbonat in 150 ml Dimethylformamid fügt man eine Lösung von 6,2 g (50 mMol) 4-Methyl-1,2-benzendiol in 20 ml Dimethylformamid. Nach 5 Minuten gibt man innerhalb 15 Minuten eine Lösung von 12 g (55 mMol) Dibromessigsäure in 25 ml Dimethylformamid zu und erhitzt während 4 Stunden auf 80°. Dann wird das Reaktionsgemisch mit Wasser versetzt, angesäuert und mehrmals mit Aethylacetat extrahiert. Die vereinigten Extrakte werden mit Wasser und mit gesättigter Natriumchloridlösung gewaschen, über Magnesiumsulfat getrocknet und eingedampft. Der Rückstand wird wie unter a) beschrieben mit Aethanol in Gegenwart von wenig konz. Salzsäure verestert.

## Beispiel 3

Analog Beispiel 1 kocht man unter Rückfluss ein Gemisch von 27,1 g (196 mMol) Kaliumcarbonat, 12,5 g (39,16 mMol) 4-Chlor-5-(2-chlorphenylsulfonyl)-1,2-benzendiol und 6,76 g (43,08 mMol) Dichloressigsäureäthylester in 200 ml 1,2-Dimethoxyäthan während 15 Stunden. Das feste Material, das sich an der Wand abgesetzt hat, wird weggekratzt, und man gibt noch 27,1 g (196 mMol) Kaliumcarbonat und 6,76 g (43,08 mMol) Dichloressigsäureäthylester zu und kocht unter Rückfluss während weiteren 8 Stunden. Das Reaktionsgemisch wird auf Eis/Wasser gegossen, angesäuert und mit Aethylacetat extrahiert. Die nach dem Eindampfen als braunes Oel zurückbleibende 5-Chlor-6-(2-chlorphenylsulfonyl)-1,3-benzodioxol-2-carbonsäure wird aus Acetonitril zur Kristallisation gebracht und noch einmal daraus umkristallisiert, worauf sie als weisse Kristalle vom Smp. 226-27° erhalten wird.

Das benötigte 4-Chlor-5-(2-chlorphenylsulfonyl)-1,2-benzendiol wird wie folgt hergestellt:

a) Zu einer Lösung von 28,8 g (163 mMol) 2-Chlorbenzolsulfinsäure (durch Reduktion von 2-Chlorbenzolsulfonylchlorid mit Natriumsulfit erhalten) in 163 ml 1 N-Natronlauge wird unter Rühren und unter Stickstoff 24,6 g (170 mMol) 4-Chlor-1,2-benzendiol zugegeben. Innerhalb 40 Minuten tropft man bei Raumtemperatur eine Lösung von 82 g (240 mMol) Kalium-hexacyano-ferrat-(III) und 150 g Natriumacetat-trihydrat in 300 ml Wasser zu. Die Lösung wird trüb und es bildet sich ein Oel, welches nach 2 Stunden Rühren kristallisiert. Das Reaktionsgemisch wird dreimal mit Aethylacetat extrahiert. Die organischen Phasen werden mit Wasser und gesättigter Natriumchloridlösung gewaschen, getrocknet und eingedampft. Die rohen braunen Kristalle werden in warmem Isopropanol gelöst und mit Aktivkohle behandelt. Die Lösung wird filtriert, auf 200 ml eingeengt, in 800 ml eiskaltes Wasser eingetropft und während 2 Stunden unter Eiskühlung gerührt. Die weisse Suspension wird genutscht, das Nutschengut mit Wasser gewaschen und getrocknet, wobei man das 4-Chlor-5-(2-chlorphenylsulfonyl)-1,2-benzendiol als weisse Kristalle vom Smp. 226-229° erhält.

## Beispiel 4

Eine Suspension von 12,0 g (43,1 mMol) 4-Methyl-5-(4-methylphenylsulfonyl)-1,2-benzendiol und 30 g (216 mMol) frisch ausgeglühtem Kaliumcarbonat in 200 ml trockenem 1,2-Dimethoxyäthan wird während 5 Minuten unter Stickstoff mit einem hochtourigen Rührer gerührt. Die Temperatur steigt dabei bis auf 75°. Dann tropft man unter mässigem Rühren (Propellerrührer) innerhalb einer Stunde eine Lösung von 6,77 g (43,1 mMol) Dichloressigsäureäthylester in 50 ml trockenem 1,2-Dimethoxyäthan zu und kocht das Gemisch anschliessend während 15 Stunden unter Rückfluss. Dann wird es auf Eis/Wasser Gemisch gegossen und mit konz. Salzsäure auf pH 1-2 eingestellt. Ein Teil des 1,2-Dimethoxyäthans wird im Rotationsverdampfer abgedampft und die zurückbleibende Lösung mit Aethylacetat extrahiert. Die vereinigten organischen Phasen werden mit Wasser und gesättigter Natriumchloridlösung gewaschen, über Natriumsulfat getrocknet und im Rotationsverdampfer eingedampft. Das erhaltene schwarze Oel wird in Aethanol gelöst, mit 0,5 g p-Toluolsulfonsäure versetzt und in einem mit einem Soxhlet-Aufsatz, der mit Molekularsieb gefüllt ist, versehenen Gefäss unter Rückfluss gekocht. Die äthanolische Lösung wird dann völlig eingedampft und an Kieselgel chromatographiert (Lösungsmittel Aethylacetat/Hexan im Verhältnis 1:1). Die die gewünschte Substanz enthaltenden, einheitlichen Fraktionen werden vereinigt und eingedampft. Man erhält so den 5-Methyl-6-(4-methylphenylsulfonyl)-1,3-benzodioxol-2-carbonsäure-äthylester als gelbes Oel, das direkt hydrolysiert werden kann.

Eine Lösung von 10 g 4-Methyl-5-(4-methylphenylsulfonyl)-1,3-benzodioxol-2-carbonsäureäthylester in 30 ml Methanol wird auf 5° abgekühlt. Unter Stickstoff und unter Rühren werden 41,3 ml 1-n. Natronlauge zugegeben wobei die Temperatur auf 30° steigt. Man kühlt das Gemisch auf 20° und rührt es bei Raumtemperatur noch 30 Minuten. Dann wird es mit Eiswasser versetzt, filtriert, das Filtrat mit konz. Salzsäure auf pH 1-2 eingestellt und dreimal mit Aethylacetat extrahiert. Die vereinigten Aethylacetat-Phasen werden mit Wasser und mit einer gesättigten Natriumchloridlösung gewaschen, über Natriumsulfat getrocknet und vollständig eingedampft. Der Rückstand wird aus Acetonitril kristallisiert. Das Kristallisat wird noch mit kaltem Aether verrührt, dann abfiltriert und getrocknet. Die so erhaltene 5-Methyl-6-(4-methyl-phenylsulfonyl)-1,3-benzodioxol-2-carbonsäure schmilzt bei 162-165°.

Das benötigte 4-Methyl-5-(4-methylphenylsulfonyl)-1,2-benzendiol wird wie folgt hergestellt :

a) Unter Stickstoff wird ein Gemisch von 500 g Polyphosphorsäure und 47,6 g (250 mMol) p-Toluolsulfonsäure-Monohydrat auf 100° aufgeheizt und dann 41,9 g (275 mMol) 1,2-Dimethoxy-4-methylbenzol (4-Methylveratrol) zugegeben. Die rötliche Suspension wird bei 105° während 30 Minuten gerührt, auf 80° abgekühlt, mit 1 500 ml Wasser versetzt und bis auf Raumtemperatur gekühlt. Das Reaktionsgemisch wird mit Aethylacetat extrahiert. Die organische Phase wird mit Wasser gewaschen, getrocknet, mit Aktivkohle behandelt und eingedampft. Die erhaltene feste Substanz wird aus Toluol umkristallisiert, kann aber auch direkt weiter umgesetzt werden. Das 1,2-Dimethoxy-4-methyl-5-(4-methylphenylsulfonyl)-benzol wird aus Toluol als weisse Kristalle vom Smp. 133-134,5° erhalten.

b) 326 g wasserfreies Pyridin-hydrochlorid werden auf 180° erhitzt. Unter Rühren gibt man rasch 33 g (113 mMol) 1,2-Dimethoxy-4-methyl-5-(phenylsulfonyl)-benzol zu. Das Reaktionsgemisch wird noch 15 Minuten auf 190° erhitzt und bei dieser Temperatur während einer Stunde gerührt. Man giesst das heisse Reaktionsgemisch auf ein Eis-Wasser-Gemisch und extrahiert dreimal mit Aether. Die Aetherphasen werden vereinigt, mit Wasser und gesättigter Natriumchloridlösung gewaschen, über Natriumsulfat getrocknet und im Rotationsverdampfer eingedampft. Man erhält so dunkelbraune Kristalle, die man aus Aether-Hexan kristallisiert. Man erhält so das 1,2-Dimethoxy-4-methyl-5-(phenylsulfonyl)-benzol vom Smp. 162-163° als sandfarbene Kristalle. Diese werden ohne zusätzliche Reinigung weiterverwendet.

Beispiel 5

Analog Beispiel 4 erhält man unter Verwendung von 25,0 g (83,7 mMol) 4-(4-Chlorphenylsulfonyl)-5-methyl-1,2-benzendiol, 13,2 g (83,7 mMol) Dichloressigsäureäthylester, 57 g (418 mMol) Kaliumcarbonat, 220 ml 1,2-Dimethoxyäthan und anschliessender Verseifung mit Natronlauge in Methanol die gewünschten 5-(4-Chlorphenyl-sulfonyl)-6-methyl-1,3-benzodioxol-2-carbonsäure als weisse Kristalle vom Smp. 169-171° aus 1,2-Dichloräthan.

Das benötigte 4-(4-Chlorphenylsulfonyl)-5-methyl-1,2-benzendiol wird wie folgt hergestellt :

a) Zu einer Lösung von 35,0 g (198.2 mMol) 4-Chlorbenzolsulfinsäure (durch Reduktion von 4-Chlorbenzolsulfonylchlorid mit Natriumsulfit erhalten) in 198.2 ml wässriger 1-n. Natriumhydroxidlösung werden unter Rühren und unter Stickstoff 27,9 g (224,6 mMol) 4-Methyl-1,2-benzendiol zugegeben. Bei Raumtemperatur tropft man innerhalb 40 Minuten eine Lösung von 109 g (330,3 mMol) Kaliumhexacyanoferrat-(III) und 198,2 g Natriumacetat-trihydrat in 400 ml Wasser zu. Nach 1 1/2 Stunden Rühren wird das Reaktionsgemisch angesäuert und mit Aethylacetat extrahiert. Die vereinigten organischen Phasen werden mit Wasser und gesättigter Natriumchloridlösung gewaschen, über Natriumsulfat getrocknet und eingedampft. Der feste Rückstand wird aus 1,2-Dichloräthan umkristallisiert. Das so gewonnene 4-(4-Chlorphenylsulfonyl)-5-methyl-1,2-benzendiol schmilzt bei 184-186°.

Beispiel 6

Analog Beispiel 4 erhält man durch Kondensation von 25,0 g (72,2 mMol) 4-(4-Cyclohexylphenylsulfonyl)-5-methyl-1,2-benzendiol mit 11,3 g (72,7 mMol) Dichloressigsäureäthylester und 50 g (361 mMol) Kaliumcarbonat in 190 ml 1,2-Dimethoxyäthan und anschliessender Verseifung mit Natronlauge in Methanol die 5-(4-Cyclohexylphenylsulfonyl)-6-methyl-1,3-benzodioxol-2-carbonsäure als weisse Kristalle vom Smp. 159-161,5 aus Aethylacetat/Hexan.

Das als Ausgangsmaterial verwendete 4-(4-Cyclohexylphenylsulfonyl)-5-methyl-1,2-benzendiol wird aus 28,2 g (226,7 mMol) 4-Methyl-1,2-benzendiol und 44,9 g (200 mMol) 4-Cyclohexylbenzolsulfinsäure (durch Reduktion des entsprechenden Sulfonylchlorids mit Natriumsulfit hergestellt) durch oxidative Kondensation analog Beispiel 5a) erhalten.

Beispiel 7

Eine Suspension von 34,6 g (250 mMol) frisch ausgeglühtem Kaliumcarbonat in 150 ml Dimethylformamid wird zuerst mit einem hochtourigen Rührer während 5 Minuten und dann mit einem normalen Rührer gerührt. Man gibt eine Lösung von 16,07 g (50 mMol) 4-(3-Acetamidophenylsulfonyl)-5-methyl-1,2-benzendiol in 50 ml Dimethylformamid zu. Nach 5 Minuten gibt man innerhalb 15 Minuten eine Lösung von 11,98 g (55 mMol) Dibromessigsäure in 25 ml Dimethylformamid zu und erhitzt dieses Reaktionsgemisch während 4 Stunden auf 80°. Dann wird es mit Wasser versetzt, mit Salzsäure angesäuert und genutscht. Das Filtrat wird mehrmals mit Aethylacetat extrahiert. Die feste Substanz wird in der wereinigten organischen Phase gelöst und die Lösung mit Wasser und gesättigter Natriumchloridlösung gewaschen, über Magnesiumsulfat getrocknet und bei 60° bis zum Kristallisationsbeginn eingeengt. Die 5-(3-Acetamidophenylsulfonyl)-6-methyl-1,3-benzodioxol-2-carbonsäure wird aus Aethylacetat umkristallisiert, Smp. 186-188°.

Das benötigte 4-(3-Acetamidophenylsulfonyl)-5-methyl-1,2-benzendiol wird analog Beispiel 3a) aus 29,7 g (150 mMol) 3-Acetamidobenzolsulfinsäure und 21,1 g (170 mMol) 4-Methyl-1,2-benzendiol durch oxidative Kopplung erhalten, Smp. 220-222° aus Isopropanol/Wasser.

Beispiel 8

Analog Beispiel 4 erhält man durch Kondensation von 19,0 g (67,3 mMol) 4-(4-Fluorphenylsulfonyl)-5-methyl-1,2-benzendiol mit 10,6 g (67,3 mMol) Dichloressigsäureäthylester und 46,5 g (336,5 mMol) Kaliumcarbonat in 200 ml 1,2-Dimethoxyäthan und anschliessender Verseifung mit Natronlauge in Methanol die gewünschte 5-(4-Fluorphenylsulfonyl)-6-methyl-1,3-benzodioxol-2-carbonsäure als weisse Kristalle vom Smp. 157-158.5° (aus 1,2-Dichloräthan).

Das benötigte 4-(4-Fluorphenylsulfonyl)-5-methyl-1,2-benzendiol wird durch oxidative Kondensation von 31,6 g (197 mMol) p-Fluorbenzolsulfinsäure (entweder durch Reduktion des entsprechenden sulfonylchlorids mit Natriumsulfit oder durch Friedel-Crafts-Reaktion von Fluorbenzol und Schwefelkohlenstoff hergestellt) mit 38,7 g (312 mMol) 4-Methyl-1,2-benzendiol analog Beispiel 5a) als gelbe Kristalle vom Smp. 175-177° aus Acetonitril erhalten.

Beispiel 9

Eine Suspension von 72,8 g (526 mMol) frisch ausgeglühtem Kaliumcarbonat in 300 ml 1,2-Dimethoxyäthan wird unter Stickstoff während 10 Minuten mit einem hochtourigen Rührer gerührt und anschliessend werden unter normalem Rühren 37,0 g (105,3 mMol) 4-(3-Acetamido-4-methoxyphenylsulfonyl)-5-methyl-1,2-benzendiol, 16,5 g (105,3 mMol) Dichloressigsäure-äthylester und 3 g Tetrabutylammonium-bromid zugegeben. Man kocht das Gemisch unter Rückfluss, gibt 300 ml Aceton zu, kocht 3 Stunden unter Rückfluss weiter, gibt nochmals 60 g Kaliumcarbonat und 16,5 g Dichloressigsäureäthylester zu und kocht unter Rückfluss während 9 weiteren Stunden. Das Reaktionsgemisch wird auf Wasser gegossen und mit Salzsäure bis pH 1-2 angesäuert. Ein Teil des Lösungsmittels wird unter Vakuum abgedampft und die zurückbleibende Wasserphase mit Aethylacetat extrahiert. Die vereinigten organischen Phasen werden mit Wasser und gesättigter Natriumchloridlösung gewaschen, über Natriumsulfat getrocknet und im Rotationszsverdampfer eingedampft. Der Rückstand wird viermal aus Acetonitril umkristallisiert, wobei man die 5-(3-Acetamido-4-methoxyphenylsulfonyl)-6-methyl-1,3-benzodioxol-2-carbonäure als weisse Kristalle vom Smp. 230° (sintern bei 150°) erhält.

Das benötigte 4-(3-Acetamido-4-methoxyphenylsulfonyl)-5-methyl-1,2-benzendiol wird analog Beispiel 5a) aus 34,4 g (150 mMol) 3-Acetamido-4-methoxybenzol-sulfinsäure und 211 g (170 mMol) 4-Methyl-1,2-benzendiol als Kristalle vom Smp. 230-232° (aus Isopropanol) erhalten.

Beispiel 10

Analog Beispiel 4 kocht man 30,0 g (111 mMol) 4-Methyl-5-(2-thienylsulfonyl)-1,2-benzendiol, 17,4 g (111 mMol) Dichloressigsäure-äthylester und 76,7 g (555 ml Kaliumcarbonat in 300 ml 1,2-Dimethoxyäthan unter Rückfluss. Nach 18 Stunden wird das Reaktionsgemisch in Wasser gegossen, eine Stunde bei Raumtemperatur gerührt und dann mittels Salzsäure auf pH 1-2 gestellt. Ein Teil 1,2-Dimethoxyäthans wird unter Vakuum abdestilliert und das zurückbleibende Gemisch mit Aethylacetat extrahiert. Die vereinigten organischen Phasen werden mit Wasser und gesättigter Natriumchloridlösung gewaschen, über Natriumsulfat getrocknet und im Rotationsverdampfer eingedampft. Das erhaltene dunkelrote Oel wird an Kieselgel chromatographiert. Als Eluiermittel wird ein Gemisch von Chloroform-Methanol-konz. Ammoniak im Verhältnis 40-10-1 bis zur Elution von Ausgangs-Diol benützt, dann die analoge Mischung im Verhältnis 12-5-1. Die einheitlichen Fraktionen werden vereinigt und fast vollständig eingedampft. Der Rückstand wird mit Eis/Wasser versetzt und mit konz. Salzsäure auf pH 1-2 gestellt. Die saure Lösung wird mit Aethylacetat extrahiert. Die vereinigten organischen Phasen werden gewaschen, getrocknet und eingedampft. Der Rückstand wird in Diisopropyläther gekocht und vom ausgefallenen schwarzen Schlamm abdekantiert. Die klare Lösung wird stark eingeengt, und die auskristallisierte Substanz abgenutscht. Die Kristalle werden aus 1,2-Dichloräthan umkristallisiert, wobei man 5-Methyl-6-(2-thienylsulfonyl)-1,3-benzodioxol-2-carbonsäure als weisse Kristalle vom Smp. 162,5-164,5° erhält.

a) Das als Ausgangsstoff benötigte 4-Methyl-5-(2-thienylsulfonyl)-1,2-benzendiol erhält man analog Beispiel 5a) aus 90 g (600 mMol) 2-Thiophensulfinsäure (erhalten durch Reduktion von 2-Thiophensulfonylchlorid mit Natriumsulfit) und 84,5 g (681 mMol) 4-Methyl-1,2-benzendiol als gelbliche Kristalle vom Smp. 161-163,5° (aus Aethylacetat).

Beispiel 11

Analog Beispiel 4 kocht man 21,3 g (64 mMol) 4-(2,5-Dichlorphenylsulfonyl)-5-methyl-1,2-benzendiol, 10,0 g (64 mMol) Dichloressigsäureäthylester und 44,2 g (320 mMol) Kaliumcarbonat in 160 ml 1,2-Dimethoxyäthan unter Rückfluss. Nach 18 Stunden wird das Reaktionsgemisch filtriert und das feste Filtergut zweimal mit 1,2-Dimethoxyäthan gewaschen. Zu den vereinigten organischen Lösungen gibt man 44 g frisches Kaliumcarbonat und 10 g Dichloressigsäure-äthylester zu, rührt während 5 Minuten mit einem hochtourigen Rührer und kocht unter normalem Rühren während 17 Stunden weiter. Die Aufarbeitung findet analog Beispiel 10 statt. Nach sorgfältigem Trocknen erhält man die 5-(2,5-Dichlorphenylsulfonyl)-6-methyl-1,3-benzodioxol-2-carbonsäure als weisse Kristalle vom Smp. 155-157° (aus Diisopropyläther).

a) Das als Ausgangsmaterial verwendete 4-(2,5-Dichlorbenzolsylfonyl)-5-methyl-1,2-benzendiol wird aus 40 g (189.5 mMol) 2,5-Dichlorbenzolsulfinsäure (durch Reduktion von 2,5-Dichlorbenzolsulfochlorid mit Natriumsulfit hergestellt) und 26.7 g (215 mMol) 4-Methyl-1,2-benzendiol analog Beispiel 5a) als sandfarbene Kristalle vom Smp. 209,5-212° (aus Acetonitril) erhalten.

## Beispiel 12

Zu einer feinkörnigen Suspension von 50,9 g (385 mMol) frisch ausgeglühtem Kaliumcarbonat in 180 ml trockenem Dimethylformamid gibt man 23 g (77 mMol) 4-(2-Chlorphenylsulfonyl)-5-methyl-1,2-benzendiol und tropft innerhalb von 15 Minuten eine Lösung von 8,50 g (39 mMol) Dibromessigsäure in 80 ml Dimethylformamid zu. Anschliessend rührt man das Gemisch während 15 Stunden bei 80°.

Dann wird das Reaktionsgemisch auf Eis gegossen und mit konz. Salzsäure auf pH 1-2 eingestellt. Die wässerige Phase wird nochmals mit Aethylacetat extrahiert. Die vereinigten organischen Phasen werden mit Wasser und gesättigter Natriumchloridlösung gewaschen, mit Natriumsulfat getrocknet und eingedampft. Das erhaltene braune Oel wird mit 150 ml Aethylacetat bei 0° bis zur Kristallisation gerührt. Die so erhaltene rohe 5-(2-Chlorphenylsulfonyl)-6-methyl-1,3-benzodioxol-2-carbonsäure wird zweimal aus Acetonitril umkristallisiert, worauf man sie als sandfarbene Kristalle vom Smp. 211-213,5° erhält.

a) Das 4-(2-Chlorphenylsulfonyl)-5-methyl-1,2-benzendiol wird aus 26,5 g (150 mMol) 2-Chlorbenzolsulfinsäure und 21,1 g (170 mMol) 4-Methyl-1,2-benzendiol analog Beispiel 5a) als weisse Kristalle, Smp. 225-229° aus Aethylacetat, erhalten.

## Beispiel 13

Analog Beispiel 12 erhält man die 5-(3-Acetamido-4-chlorphenylsulfonyl)-6-methyl-1,3-benzodioxol-2-carbonsäure durch Kondensation von 22,0 g (61,8 mMol) 4-(3-Acetamido-4-chlorphenyl-sulfonyl)-5-methyl-1,2-benzendiol mit 16,25 g (74,18 mMol) Dibromessigsäure und 42,7 g (309 mMol) Kaliumcarbonat in 225 ml Dimethylformamid, Smp. 198-200°.

Die Aufarbeitung erfolgt ebenfalls analog Beispiel 12. Indessen wird das als Rohprodukt erhaltene dunkelrote Oel über einen Kieselgeltrichter chromatographiert (Chloroform-Methanol-konz. Ammoniak 40:10:1).

Die einheitlichen Fraktionen werden mit Aethylacetat extrahiert, und die nach Eindampfen des Extraktes zurückbleibende Substanz wird aus Acetonitril umkristallisiert, wobei man den Endstoff als sandfarbene Kristalle vom Smp. 198-200° (sintert ab 120°) erhält.

Das als Ausgangsmaterial verwendete 4-(Acetamido-4-chlorphenylsulfonyl)-5-methyl-1,2-benzendiol wird wie folgt hergestellt.

a) 66 g (297,8 mMol 4-Chlor-3-nitro-benzolsulfinsäure (durch Reduktion von 4-Chlor-3-nitro-benzolsulfonylchlorid mit Natriumsulfit hergestellt werden mit 41,95 g (3379 mMol) 4-Methyl-1,2-benzendiol analog Beispiel 4 kondensiert, wobei man das 4-(4-Chlor-3-nitrophenyl-sulfonyl)-5-methyl-1,2-benzendiol als Kristalle vom Smp. 196-197° (aus Aethylacetat/Hexan) erhält.

b) 47,5 g (138,2 mMol) des gemäss a) erhaltenen Produkts werden katalytisch mit Wasserstoff in Gegenwart von Raney-Nickel zum 4-(3-Amino-(4-chlorphenylsulfonyl)-5-methyl-1,2-benzendiol hydriert. Letzteres kristallisiert aus 1,2-Dichloräthan als sandfarbene Kristalle vom Smp. 191-193°.

c) 40 g (112,42 mMol) des nach b) erhaltenen Amins werden mit 11,48 g (112,42 mMol) Acetanhydrid in 240 ml Eisessig zum gewünschten 4-(3-Acetamido-4-chlorphenylsulfonyl)-5-methyl-1,2-benzendiol acetyliert, das aus Acetonitril als weisse Kristalle vom Smp. 224-226,5° erhalten wird.

## Beispiel 14

21,0 g (70,3 mMol) 4-Chlor-5-(4-methylphenylsulfonyl)-1,2-benzendiol und 48,6 g (ca. 350 mMol) wasserfreies, pulverisiertes Kaliumcarbonat werden bei Raumtemperatur unter Inertgas in 160 ml Dimethylformamid suspendiert. Anschliessend tropft man bei Raumtemperatur innerhalb 15 Minuten eine Lösung von 16,8 g (77,4 mMol) Dibromessigsäure in 80 ml Dimethylformamid unter Rühren zu. Dann erhitzt man das Reaktionsgemisch unter Rühren während 15 Stunden auf 95°. Zum Aufarbeiten wird das Reaktionsgemisch auf Eis-Wasser-Gemisch gegossen und mit konz. Salzsäure auf pH 2 angesäuert. Das Rohprodukt wird mit Aethylacetat extrahiert und das Lösungsmittel abgedampft. Die zurückbleibende

rohe Säure löst man in 100 ml Methanol und setzt 0,5 ml konz. Salzsäure zu. Nach 5 Stunden Stehen bei Raumtemperatur setzt die Kristallisation ein. Man kühlt die Kristallsuspension im Eisbad für 18 Stunden, dann filtriert man die Kristalle ab und wäscht sie mit eiskaltem Methanol nach. Hierauf suspendiert man sie in 60 ml Methanol und gibt bei Raumtemperatur 35 ml 2-n. Natronlauge zu, dabei tritt Lösung ein. Nach einer Stunde Stehenlassen wird die Lösung auf Eis/Wasser-Gemisch ausgegossen und mit 6-n. Salzsäure angesäuert. Die 5-Chlor-6-(4-methylphenylsulfonyl)-1,3-benzodioxol-2-carbonsäure fällt aus und wird mit Aethylacetat extrahiert. Den Eindampfrückstand des Extraktes kristallisiert man aus Acetonitril um und erhält so die obige Substanz als sandfarbene Kristalle vom Smp. 181-183,5°.

Der aromatische Ausgangsstoff wird wie folgt hergestellt :

a) Analog zu Beispiel 5a) wird p-Toluolsulfinsäure mit 4-Chlor-1,2-benzendiol oxidativ kondensiert. Bei der Umkristallisation aus 1,2-Dichloräthan wird das 4-Chlor-(4-methylphenylsulfonyl)-1,2-benzendiol als farblose Kristalle vom Smp. 162-164° erhalten.

## Beispiel 15

42,6 g (ca. 0,13 Mol) 4-Brom-5-(phenylsulfonyl)-1,2-benzendiol und 90,0 g (ca. 0,65 Mol) wasserfreies, pulverisierte Kaliumcarbonat werden in 300 ml Dimethylformamid unter Inertgasschutz gerührt. Bei Raumtemperatur setzt man die Hälfte einer Lösung von 57,0 g (ca. 0,26 Mol) Dibromessigsäure in 100 ml Dimethylformamid zu. Man rührt das Reaktionsgemisch 8 Stunden bei 95°, setzt dann den Rest der Dibromessigsäure-Lösung zu und rührt weitere 8 Stunden bei 95°. Hierauf wird bei vermindertem Druck das Dimethylformamid abdestilliert, der Rückstand im Wasser gelöst, die Lösung mit konz. Salzsäure auf pH 1-2 eingestellt und mit Aethylacetat extrahiert. Die Aethylacetatlösung wird mit Wasser gewaschen, über Natriumsulfat getrocknet und eingedampft. Der Rückstand wird in 150 ml Methanol gelöst und mit 0,3 ml konz. Salzsäure versetzt. Nach Stehenlassen über Nacht bei Raumtemperatur wird die Lösung eingedampft und der Rückstand auf einer Säule aus Kieselgel mit dem Laufmittelgemisch Aethylacetat/Hexan 1 : 2 chromatographiert. Die einheitlichen Fraktionen werden vereinigt und eingedampft. Diesen Eindampfrückstand versetzt man mit 50 ml 2-n. Natronlauge und rührt mit einem Magnetrührer bei Raumtemperatur, bis eine Lösung enstanden ist. Man stellt diese mit konz. Salzsäure auf pH 1-2 und extrahiert die ausgefällte Säure wiederholt mit Aethylacetat. Die vereinigten Aethylacetatlösungen werden mit Wasser gemaschen, mit Natriumsulfat getrocknet, filtriert und eingedampft, worauf die 5-Brom-6-(phenylsulfonyl)-1,3-benzodioxol-2-carbonsäure als weisse Kristallkruste anfällt. Nach Umkristallisation aus Aethylacetat/Hexan erhält man obige Substanz als farblose Kristalle vom Smp. 219-222° (Zers.).

Den aromatischen Ausgangsstoff erhält man auf folgende Art.

a) 48,0 g 4-Brom-1,2-benzendiol [vgl. W. Rosenmund, Ber. 62, 1262 usf. (1923)] werden mit 33,4 g Benzolsulfinsäure-Natriumsalz analog Beispiel 5a) oxidativ kondensiert. Nach Kristallisation aus 1,2-Dichloräthan erhält man das 4-Brom-5-(phenylsulfonyl)-1,2-benzendiol als farblose Kristalle vom Smp. 166-168°.

## Beispiel 16

25,0 g (ca. 85 mMol) 4-(4-Methoxyphenylsulfonyl)-5-methyl-1,2-benzendiol werden in 200 ml Dimethylformamid unter Verwendung von 60 g Kaliumcarbonat mit 20,4 g (93,5 mMol) Dibromessigsäure in 100 ml Dimethylformamid ganz analog Beispiel 14 umgesetzt. Die erhaltene rohe Säure wird in Aethylacetat gelöst und mit einer Lösung von 5,8 g Hexansäure-Natriumsalz in Methanol versetzt. Die dabei ausfallenden Kristalle werden abfiltriert und getrocknet. Man erhält so das Natriumsalz der 5-(4-Methoxyphenylsulfonyl)-6-methyl-1,3-benzodioxol-2-carbonsäure als farblose Kristalle, die über 220° (Zers.) schmelzen.

Der aromatische Ausgangsstoff wird auf folgende Art erhalten :

a) Man kondensiert 37,5 g 4-Methoxybenzolsulfinsäure (z. B. durch Reduktion von 4-Methoxybenzolsulfonylchlorid mit Natriumsulfit erhalten) oxidativ mit 30,7 g 4-Methyl-1,2-benzendiol analog der im Beispiel 5a) beschriebenen Weise und erhält nach Kristallisation aus 1,2-Dichloräthan das 4-(4-Methoxyphenylsulfonyl)-5-methyl-1,2-benzendiol als farblose Kristalle vom Smp. 145-147,5°.

## Beispiel 17

15,0 g (0,05 Mol) 4-(2,4-Difluorbenzolsulfonyl)-5-methyl-1,2-benzendiol und 34,6 g (0,25 Mol) Kaliumcarbonat werden unter Stickstoffschutz in 100 ml Dimethylformamid bei Raumtemperatur suspendiert. Innerhalb 10 Minuten wird eine Lösung von 11,9 g (ca. 0,055 Mol) Dibromessigsäure in 30 ml Dimethylformamid zugetropft und das Reaktionsgemisch anschliessend unter rühren während 5 1/2 Stunden auf 90° erhitzt. Das Reaktionsgemisch wird in 1 500 ml Wasser gegossen und mit konz. Salzsäure auf pH 1-2 eingestellt. Die rohe Säure wird mit Aethylacetat extrahiert, die Aethylacetatlösung

mit Wasser gewaschen, über Natriumsulfat getrocknet, filtriert und eingedampft. Zum Eindampfrückstand gibt man 50 ml 1-n. Natronlauge und dampft die so erhaltene Lösung im Rotationsverdampfer ein, bis das Natriumsalz auszukristallisieren beginnt. Durch Zusatz von Isopropanol und Erwärmen zum Rückfluss erhält man wieder eine lösung, die mit Aktivkohle versetzt und filtriert wird. Aus dem Filtrat scheidet sich beim Abkühlen das Natriumsalz der 5-(2,4-Difluorphenylsulfonyl)-6-methyl-1,3-benzodioxol-2-carbonsäure in farblosen Nadeln ab. Nach Filtration und Trocknen schmilzt das Salz oberhalb 200°, nach Sintern ab 160°.

Der Ausgangsstoff wird wie folgt hergestellt :

a) Durch Meerweinreaktion ausgehend von 2,4-Difluoranilin erhält man das 2,4-Difluorbenzolsulfonylchlorid vom Kp : 87°/5 mbar.

b) Durch Reduktion von 2,4-Difluorbenzolsulfonylchlorid mit Natriumsulfit erhält man die 2,4-Difluorbenzolsulfinsäure.

c) Analog Beispiel 5a) erhält man durch oxidative Kondensation von 23,5 g 2,4-Difluorbenzolsulfinsäure mit 18,5 g 4-Methyl-1,2-benzendiol nach Umkristallisation aus 1,2-Dichloräthan das 4-(2,4-Difluorbenzolsulfonyl)-5-methyl-1,2-benzendiol vom Smp. 158-162°.

Beispiel 18

Analog Beispiel 1 setzt man 18,3 g 4-(3-Chlor-4-fluorphenylsulfonyl)-5-methyl-1,2-benzendiol in Gegenwart von 39,9 g Kaliumcarbonat mit 10,0 g Dichloressigsäureäthylester in 150 ml Dimethoxyäthan um. Man erhält nach analoger Aufarbeitung und Umkristallisation aus Acetonitril die 5-(3-Chlor-4-fluorphenylsulfonyl)-6-methyl-1,3-benzodioxol-2-carbonsäure als farblose Kristalle vom Smp. 188,5-190,5°.

Der aromatische Ausgangsstoff wird wie folgt hergestellt :

a) Das im Beispiel 18b eingesetzte 3-Chlor-4-fluorbenzolsulfochlorid Kp. 90-95°/5 mbar erhält man nach Literatur-bekannten Verfahren, so z. B. durch Meerwein-Reaktion aus 3-Chlor-4-fluoranilin.

b) Durch Reduktion von 3-Chlor-4-fluorbenzolsulfochloriud mit Natriumsulfit erhält man die 3-Chlor-4-fluorbenzolsulfinsäure vom Smp. 66-68°.

c) Analog Beispiel 5a) erhält man durch oxidative Kondensation von 3-Chlor-4-fluorbenzolsulfinsäure mit 4-Methyl-1,2-benzendiol das im obigen Beispiel 18 eingesetzte 4-(3-Chlor-4-fluorphenylsulfonyl)-5-methyl-1,2-benzendiol vom Smp. 166-167,5° als farblose Kristalle.

Beispiel 19

Analog Beispiel 17 setzt man 14 g (42,8 mMol) 5-(4-Chlor-2,5-dimethylphenylsulfonyl)-4-methyl-1,2-benzendiol und 10,3 g Dibromessigsäure in Gegenwart von 29,6 g Kaliumcarbonat in 150 ml Dimethylformamid um und erhält nach Aufarbeitung, Salzbildung mit Natronlauge und Umkristallisation aus Isopropanol/Wasser 5-(4-Chlor-2,5-dimethylphenylsulfonyl)-6-methyl-1,3-benzodioxol-1,2-carbonsäure Natriumsalz als farblose Kristalle vom Smp. > 180° (Zers.).

Das als Ausgangsstoff eingesetzte 5-(4-Chlor-2,5-dimethylphenylsulfonyl)-4-methyl-1,2-benzendiol erhält man durch oxidative Kondensation von 4-Chlor-2,5-dimethylbenzolsulfonsäure (seinerseits durch Reduktion von 4-Chlor-2,5-dimethylbenzolsulfochlorid mit Natriumsulfit erhalten) mit 4-Methyl-1,2-benzendiol analog Beispiel 5a) und Kristallisation aus Aethylacetat als gelbliche Kristalle vom smp. 193-196° (Sintern ab 143°).

Beispiel 20

31,2 g (0,1 Mol) 5-(4-Chlor-2,5-dimethylphenylsulfonyl)-4-methyl-1,2-benzendiol und 55 g (0,4 Mol) Kaliumcarbonat werden unter Inertgas in 150 ml Dimethylformamid suspendiert. Bei Raumtemperatur gibt man 17,3 g (0,11 Mol) Dichloressigsäure-äthylester zu und erhitzt das Gemisch anschliessend für 10 Stunden auf 80°. Zum Aufarbeiten wird unter Vakumm das Dimethylformamid abdestilliert und den Rückstand in Wasser gelöst. Die Lösung wird mit konz. Salzsäure auf pH 1-2 angesäuert und die ausgefällte rohe Säure mehrmals mit Aethylacetat extrahiert. Die vereinigten Aethylacetatlösungen werden eingedampft und der Rückstand in der Wärme in der äquivalenten Menge 2-n. Natronlauge gelöst. Beim Abkühlen scheidet sich das Natriumsalz der 5-(4-Chlor-2,5-dimethylphenylsulfonyl)-6-methyl-1,3-benzodioxol-2-carbonsäure aus. Nach Kühlen wird es abfiltriert und wie im Beispiel 19 aus Isopropanol umkristallisiert.

Beispiel 21

Analog Beispiel 17 werden 25 g (71,7 mMol) 4-(2,4-Dichlor-5-methylphenylsulfonyl)-5-methyl-1,2-benzendiol mit 17,14 g (78,7 mMol) Dibromessigsäure in Gegenwart von 49,5 g Kaliumcarbonat in 250 ml Dimethylformamid umgesetzt. Man erhält nach Aufarbeitung, Salzbildung und Umkristallisation analog

Beispiel 17 das Natriumsalz der 5-(2,4-Dichlor-5-methylphenylsulfonyl)-6-methyl-1,3-benzodioxol-2-carbonsäure als farblose Kristalle vom Smp. 200° (Zers.).

a) Das vorstehend eingesetzte 4-(2,4-Dichlor-5-methylphenylsulfonyl)-5-methyl-1,2-benzendiol erhält man durch oxidative Kondensation von 2,4-Dichlor-5-methyl-benzolsulfinsäure (aus 2,4-Dichlor-5-methylbenzolsulfochlorid durch Reduktion mit Na-Sulfit) mit 4-Methyl-1,2-benzendiol analog Beispiel 5a), Smp. 203,5-206° (Zers., aus Acetonitril).

Beispiel 22

Analog Beispiel 15 werden 60,0 g 4-(4-Bromphenylsulfonyl)-5-methyl-1,2-benzendiol in Gegenwart von 120 g Kaliumcarbonat in 300 ml Dimethylformamid mit einer Lösung von 76,2 g Dibromessigsäure in 100 ml Dimethylformamid umgesetzt. Nach Aufarbeitung und chromatographischer Reinigung analog Beispiel 15 und Kristallisation aus 1,2-Dichloräthan erhält man die 5-(4-Bromphenylfonyl)-6-methyl-1,3-benzodioxol-2-carbonsäure als sandfarbene Kristalle vom Smp. 170-172,5°.

a) Das im obigen Beispiel eingesetzte 4-(4-Bromphenylsulfonyl)-5-methyl-1,2-benzendiol erhält man durch oxidative Kondensation von 76,0 g p.-Brombenzolsulfinsäure (aus p-Brombenzolsulfochlorid durch Reduktion mit Natriumsulfit) mit 53,0 g 4-Methyl-1,2-benzendiol analog Beispiel 5a) als farblose Kristalle vom Smp. 190-191,5° (aus Chloroform).

Beispiel 23

39,6 g (ca. 0,105 Mol) 4-[(2-Tert.-Butyl-6-benzothiazolyl)-sulfonyl]-5-methyl-1,2-benzendiol und 72,3 g (0,523 Mol) Kaliumcarbonat werden unter Inertgasschutz bei Raumtemperatur in 300 ml Dimethylformamid gerührt. Innerhalb von 20 Minuten tropft man die Hälfte einer Lösung 45,6 g (0,209 Mol) Dibromessigsäure in 100 ml Dimethylformamid zu. Anschliessend rührt man das Reaktionsgemisch während 21 Stunden bei 100°, und setzt während dieser Zeit in kleinen Portionen den Rest der Dibromessigsäurelösung zu. Zum Aufarbeiten wird im Rotationsverdampfer unter Wasserstrahlvakuum das Dimethylformamid abdestilliert. Der Rückstand wird in Wasser gelöst und mit konz. Salzsäure auf pH 1-2 gestellt. Man extrahiert die ausgefällte Säure dreimal mit Aethylacetat. Die vereinigten Aethylacetatlösungen werden einmal mit Wasser gewaschen, über Natriumsulfat getrocknet, filtriert und eingedampft. Der Rückstand wird in 500 ml Aethanol gelöst, und die Lösung nach Zugabe von 0,5 ml konz. Salzsäure 10 Tage bei Raumtemperatur stehen gelassen. Der Aethylester der 5-Methyl-6-[(2-tert.-butyl-6-benzothiazolyl)-sulfonyl]-1,3-benzodioxol-2-carbonsäure fällt währenddessen als farblose Kristalle aus. Man kühlt die Kristallsuspension auf 0° und filtriert die Kristalle ab. Die noch Aethanolfeuchten Kristalle versetzt man mit 90 ml 2-n. Natronlauge und rührt das Gemisch 2 Stunden bei Raumtemperatur wobei eine Lösung entsteht. Diese wird mit konz. Salzsäure angesäuert und die ausgefällte Säure dreimal mit Aethylacetat extrahiert. Die Aethylacetatlösungen wäscht man mit Wasser, trocknet sie über Natriumsulfat, filtriert sie und dampft sie im Vakuum zur Trockene ein. Der Rückstand wird aus Aethylacetat umkristallisiert, wobei man 5-Methyl-6-[(2-tert.-butyl-6-benzothiazolyl)-sulfonyl]-1,3-benzodioxol-2-carbonsäure als farblose Kristalle vom Smp. 247° (Zers.), nach Sintern ab 203°, erhält.

Der Ausgangsstoff wird wie folgt hergestellt :

a) Die 2-Tert.-butyl-6-benzothiazolsulfochlorid erhält man durch die bekannte Meerweinreaktion, ausgehend vom 6-Amino-2-tert.-butyl-benzothiazol.

b) Durch Reduktion von 2-Tert.-butyl-6-benzothiazolsulfochlorid erhält man die 2-Tert.-butyl-benzothiazol-2-sulfinsäure, welche oberhalb 150° schmilzt.

c) Das 4-Methyl-5-[(2-tert.-butyl-6-benzothiazolyl)-sulfonyl]-1,2-benzendiol erhält man analog Beispiel 5a) und anschliessende Chromatographie mit einem Smp. von 185° (Zers.).

Beispiel 24

Analog Beispiel 15 setzt man entsprechende Mengen 4-Chlor-5-phenylsulfonyl-1,2-benzendiol und Dibromessigsäure in Dimethylformamid in Gegenwart von Kaliumcarbonat um. Man erhält nach Aufarbeitung, Reinigung und Umkristallisation analog Beispiel 15 die 5-Chlor-6-phenylsulfonyl-1,3-benzodioxol-2-carbonsäure vom Smp. 183-185°.

a) Das im obigen Beispiel eingesetzte 4-Chlor-5-phenylsulfonyl-1,2-benzendiol erhält man durch Umsetzung von Benzolsulfinsäure mit 4-Chlor-1,2-benzendiol analog Beispiel 5a), Smp. 161-165°.

Beispiel 25

Analog Beispiel 15 erhält man bei der Umsetzung entsprechender Mengen 4-Chlor-5-(4-chlorphenylsulfonyl)-1,2-benzendiol und Dibromessigsäure in Dimethylformamid in Gegenwart von Kaliumcarbonat

und analoger Aufarbeitung, Reinigung und Umkristallisation die 5-Chlor-6-(4-chlorphenylsulfonyl)-1,3-benzodioxol-2-carbonsäure ; Smp. 194-196°.

a) Das im obigen Beispiel eingesetzte 4-Chlor-5-(4-chlorphenylsulfonyl)-1,2-benzendiol wird durch Umsetzung von 4-Chlorbenzolsulfinsäure und 4-Chlor-1,2-benzendiol analog Beispiel 5a) erhalten, Smp. 165-176°.

Beispiel 26

20,1 g 4-(4-Acetylphenylsulfonyl)-5-methyl-1,2-benzendiol und 28,3 g Dibromessigsäure werden in 130 ml Dimethylformamid in Gegenwart von 45 g Kaliumcarbonat analog Beispiel 14 umgesetzt. Nach analogem Aufarbeiten und Reinigen erhält man die 5-(4-Acetylphenylsulfonyl)-6-methyl-1,3-benzodioxol-2-carbonsäure ; Smp. 107-110°.

Der aromatische Ausgangsstoff wird wie folgt hergestellt :

a) Durch Reduktion von 4-Acetylbenzolsulfonylchlorid mit Natriumsulfit erhält man die 4-Acetylbenzolsulfinsäure.

b) Durch oxidative Kondensation von 4-Acetylbenzosulfinsäure mit 4-Methyl-1,2-benzendiol erhält man das 4-(4-Acetylphenylsulfonyl)-5-methyl-1,2-benzendiol vom Smp. 184-188°.

Beispiel 27

10,0 g (0,035 9 Mol) 4-Phenylsulfonyl-5-äthyl-1,2-benzendiol und 31,5 g (0,288 Mol) Kaliumcarbonat werden in 100 ml Dimethylformamid bei Raumtemperatur unter Inertgasschutz vorgelegt und mit 9,0 g (0, 041 3 Mol) Dibromessigsäure versetzt. Das Reaktionsgemisch wird unter Rühren für 14 Stunden auf einer Innentemperatur von ca. 80° gehalten. Anschliessend wird am Rotationsverdampfer unter Wasserstrahlvacuum das Dimethylformamid abdestilliert und der Rückstand in Wasser aufgenommen. Man stellt mit HCl conc. auf pH 1-2, extrahiert die Rohsäure mit Aethylacetat, trocknet den Extrakt mit Natriumsulfat, filtriert und dampft am Rotationsverdampfer zur Trockne ein. Der Rückstand wird in 100 ml Aethanol abs. gelöst, 0,2 ml HCl conc. zugegeben und für zwei Stunden am Rückfluss gekocht. Man dampft zur Trockne ein, nimmt den Rückstand in Aethylacetat auf und wäscht im Scheidetrichter 1 Mal mit gesättigter Natriumbicarbonatlösung und zwei Mal mit Wasser. Die Aethylacetatphase wird über Natriumsulfat getrocknet, filtriert und eingedampft. Der Rückstand wird mit 20 ml 2-n NaOH versetzt und auf dem warmen Wasserbad erhitzt bis eine gelbliche Lösung entstanden ist. Man kühlt ab, stellt mit HCl conc. auf pH 1-2 und extrahiert die gereinigte 5-Phenylsulfonyl-6-äthyl-1,3-benzodioxol-2-carbonsäure mit Aethylacetat. Die Extraktlösung wird über Natriumsulfat getrocknet und filtriert. Man setzt 50 ml Toluol zu und dampft am Rotationsverdampfer das Aethylacetat ab. Die Säure fällt in Form von farblosen Kristallen an : Smp. 143-145°.

a) Das im obigen Beispiel eingesetzte 4-Phenylsulfonyl-5-äthyl-1,2-benzendiol erhält man durch oxidative Kupplung von Benzolsulfinsäure mit 4-Aethyl-1,2-benzendiol analog Beispiel 1a) ; Smp. 180-182°.

Beispiel 28

25,5 g (0,097 Mol) 4-(2-Methyl-phenylsulfonyl)-5-methyl-1,2-benzendiol, 62,1 g (0,45 Mol) Kaliumcarbonat und 32,7 g (0,145 Mol) Dibromessigsäure (97 %ig) werden in 300 ml Dimethylformamid unter Inertgasatmosphäre analog Beispiel 27 umgesetzt. Nach Aufarbeitung, Reinigung über den Aethylester und Verseifung, ganz analog Beispiel 27, erhält man farblose Kristalle vom Smp. 178-180° (auf Toluol) 5-(2-Methylphenylsulfonyl)-6-methyl-1,3-benzodioxol-2-carbonsäure.

Das oben eingesetzte 4-(2-Methylphenylsulfonyl)-5-methyl-1,2-benzendiol-wird wie folgt hergestellt :

a) 33,2 (0,108 Mol) 1,2-Dimethoxy-4-(2-methylphenylsulfonyl)-5-methylbenzol und 70 g (0,6 Mol) Pyridin-Hydrochlorid werden in einem Bad von 200° während 2 $^1/_2$ Stunden zusammen zur Schmelze erwärmt. Man giesst die Schmelze auf Eis und setzt 20 ml HCl conc. zu und extrahiert dann mit Aethylacetat. Die Extraktlösung wird zwei Mal mit Wasser gewaschen, mit Natiumsulfat getrocknet, filtriert und am Rotationsverdampfer eingeengt. Beim Erreichen eines Volumens von ca. 100 ml fängt das Produkt an auszukristalliseiren. Man kühlt $^1/_2$ Stunde im Eisbad, filtriert ab und trocknet die erhaltenen beigen Kristalle des 4-(2-Methylphenylsulfonyl)-5-methyl-1,2-benzendiol ; Smp. 221-223°.

Den im Beispiel 28a) eingesetzten Ausgangsstoff erhält man folgendermassen :

b) 50 g Phosphorpentoxid werden mit 400 ml Methansulfosäure übergossen und bei 60° gerührt bis eine klare Lösung entstanden ist. Nun gibt man 30,4 g (0,2 Mol) 1,2-Dimethoxy-4-methylbenzol und 43 g (0,2 Mol) 2-Methylbenzolsulfonsäure (80 %ig) zu und erhöht die Innentemperatur für 30 Minuten auf 80°. Man giesst auf 4 Liter Eiswasser und extrahiert mit Aethylacetat. Die Extraktlösung wird zwei Mal mit Wasser gewaschen, über Natriumsulfat getrocknet, filtriert und am Rotationsverdampfer eingedampft.

Das zurückbleibende Oel kristallisiert sofort. Man verreibt mit Diäthyläther und filtriert ab. So erhält man farblose Kristalle von 1,2-Dimethoxy-4-(2-methylphenylsulfonyl)-5-methylbenzol ; Smp. 126-128°.

Beispiel 29

24,5 g (0,088 Mol) 4-(3-Methylphenylsulfonyl)-1,2-benzendiol, 60,7 g (0,44 Mol) Kaliumcarbonat und 29,6 g (0,132 Mol) Dibromessigsäure (97 %ig) werden in 300 ml Dimethylformamid analog Beispiel 27 umgesetzt. Nach Aufarbeitung, Reinigung über den Aethylester und Verseifung analog Beispiel 27 erhält man aus Toluol/Aethylacetat farblose Kristalle der 5-(3-Methylphenylsulfonyl)-6-methyl-1,3-benzodioxol-2-carbonsäure ; Smp. 155-157°.

a) Das oben eingesetzte 4-(3-Methylphenylsulfonyl)-1,2-benzendiol wird analog Beispiel 28a) durch Aetherspaltung von 1,2-Dimethoxy-4-(3-methylphenylsulfonyl)-5-methylbenzol mittels Pyridin-Hydrochlorid hergestellt ; Smp. 172-174° (aus Toluol/Aethylacetat)

b) Obiges 1,2-Dimethoxy-4-(3-methylphenylsulfonyl)-5-methylbenzol erhält man durch Umsetzung von 3-Methylbenzolsulfonsäure mit 1,2-Dimethoxy-4-methylbenzol analog Beispiel 28b) ; Smp. 135-140° (aus Aether)

Beispiel 30

13,1 g (0,043 8 Mol) 4-(3-Chlorphenylsulfonyl)-5-methyl-1,2-benzendiol, 30,0 g (0,217 Mol) Kaliumcarbonat und 14,7 g (0,065 Mol) Dibromessigsäure (97 %ig) werden in 150 ml Dimethylformamid für 5 Stunden unter Inertgasatmosphäre bei einer Innentemperatur von 80° gerührt. Nach Aufarbeitung, Reinigung über den Aethylester und Verseifung analog Beispiel 27 erhält man farblose Kristalle der 5-(3-Chlorphenylsulfonyl)-6-methyl-1,3-benzodioxol-2-carbonsäure ; Smp. 172-174° (aus Toluol/Ethylacetat).

a) Oben eingesetztes 4-(3-Chlorphenylsulfonyl)-5-methyl-1,2-benzendiol erhält man analog Beispiel 28a) durch Aetherspaltung aus 1,2-Dimethoxy-4-(3-chlorphenylsulfonyl)-5-methyl 185-187° (aus Toluol/Ethylacetat).

b) Das im Beispiel 30a) eingesetzte 1,2-Dimethoxy-4-(3-chlorphenylsulfonyl)-5-methylbenzol erhält man analog Beispiel 28b) durch Kondensation von 3-Chlorbenzolsulfonsäure mit 1,2-Dimethoxy-4-methylbenzol in Phosphorpentoxyd/Methansulfosäure bei einstündigem Erhitzen auf 80° ; Smp. 151-152° (aus Toluol).

Beispiel 31

9,2 g (0,029 Mol) 4-Chlor-5-(3-chlorphenylsulfonyl)-1,2-benzendiol, 18,0 g (0,13 Mol) Kaliumcarbonat und 9,5 g (0,042 Mol) Dibromessigsäure (97 %ig) werden in 70 ml Dimethylformamid analog Beispiel 27 umgesetzt. Nach Aufarbeitung und Veresterung analog Beispiel 27 wird der Rohester mit dem Lösungsmittelgemisch Hexan/Aethylacetat (9 : 1) über 200 g Kieselgel chromatographiert, die einheitlichen Fraktionen vereinigt, eingedampft und anschliessend mit 2-n NaOH verseift. Nach Freisetzung und Isolierung der 5-Chlor-6-(3-chlorphenylsulfonyl)-1,3-benzodioxol-2-carbonsäure wird diese aus Toluol/Aethylacetat umkristallisiert ; Smp. 182-184°. In analoger Weise erhält man die 4,5-Dichlor-6-(3-chlorphenylsulfonyl)-1,3-benzdioxol-2-carbonsäure, die 5-Brom-6-(3-chlorphenylsulfonyl)-1,3-benzdioxol-2-carbonsäure, Smp. 189-192, und die 5-Chlor-6-(3-methylphenylsulfonyl)-1,3-benzdioxol-2-carbonsäure.

Oben eingesetztes 4-Chlor-5-(3-chlorphenylsulfonyl)-1,2-benzendiol kann auf folgende Weisen hergestellt werden :

a) Durch oxidative Kupplung von 3-Chlorbenzolsulfinsäure mit 4-Chlor-1,2-benzendiol analog Beispiel 3a) erhält man nach Kristallisation aus Toluol/Aethylacetat das 4-Chlor-5-(3-chlorphenylsulfonyl)-1,2-benzendiol vom Smp. 192-194°.

b) Durch Aetherspaltung von 1,2-Dimethoxy-4-chlor-5-(3-chlorphenylsulfonyl)-benzol mittels Pyridin-Hydrochlorid analog Beispiel 28a) erhält man ebenfalls das 4-Chlor-5-(3-chlorphenylsulfonyl)-1,2-benzendiol.

c) Das in Beispiel 31b) eingesetzte 1,2-Dimethoxy-4-chlor-5-(3-chlorphenylsulfonyl)-benzol erhält man durch Umsetzung von 3-Chlorbenzolsulfonsäure mit 1,2-Dimethoxy-4-chlorbenzol analog Beispiel 28b) ; Smp. 154-156°.

Beispiel 32

19,7 g (0,065 Mol) 4-Chlor-5-(4-fluorphenylsulfonyl)-1,2-benzendiol, 40,1 g (0,29 Mol) Kaliumcarbonat und 23,6 g (0,108 Mol) Dibromessigsäure (97 %ig) werden in 160 ml Dimethylformamid während 20 Stunden unter Inertgasatmosphäre bei 80° gerührt. Man dampft am Rotationsverdampfer unter Wasserstrahlvacuum das Dimethylformamid ab und nimmt den Rückstand in Wasser auf. Mit HCl conc.

wird pH 1-2 eingestellt, dann extrahiert mann mit Aethylacetat. Die Extraktlösung wird eingedampft und der Rückstand in 250 ml Aethanol abs. gelöst. Nach Zusatz von 0,2 ml HCl conc. wird für 2 Stunden am Rückfluss gekocht und anschliessend zur Trockne eingedampft. Der harzige Rückstand wird mit einem Lösungsmittelgemisch von Chloroform/Hexan/Aethylacetat (1 : 1 : 1) über 400 g Kieselgel chromatographiert. Die einheitlichen Fraktionen werden gesammelt und eingedampft. Der so erhaltene Aethylester der 5-Chlor-6-(4-fluorphenylsulfonyl)-1,3-benzodioxol-2-carbonsäure wird mit 100 ml 2-n. NaOH versetzt und eine Stunde bei 80° gerührt. Die so entstehende gelbe Lösung wird in der Wärme mit Aktivkohle versetzt und filtriert. Beim Abkühlen kristallisiert das Natriumsalz der 5-Chlor-6-(4-fluorphenylsulfonyl)-1,3-benzodioxol-2-carbonsäure in feinen farblosen Blättchen ; Smp. 216-220° (Zers.).

a) Das oben eingesetzte 4-Chlor-5-(4-fluorphenylsulfonyl)-1,2-benzendiol erhält man analog Beispiel 3a) durch oxidative Kupplung von 4-Chlor-1,2-benzendiol mit 4-Fluorbenzolsulfinsäure ; Smp. 180-182° (aus Aethylacetat/Toluol).

Beispiel 33

18,1 g (0,05 Mol) 4-Chlor-5-(4-bromphenylsulfonyl)-1,2-benzendiol, 31,0 g (0,244 Mol) Kaliumcarbonat und 18,4 g (0,083 Mol) Dibromessigsäure (97 %ig) werden in 120 ml Dimethylformamid analog Beispiel (7 umgesetzt. Nach Aufarbeitung, Reinigung über den Aethylester und Verseifung ganz analog Beispiel 27 erhält man 5,8 g 5-Chlor-6-(4-bromphenylsulfonyl)-1,3-benzdioxol-2-carbonsäure ; Smp. 193-196° (aus Toluol).

a) Das oben eingesetzte 4-Chlor-5-(4-bromphenylsulfonyl)-1,2-benzdiol erhält man durch oxidative Kupplung von 4-Brombenzolsulfinsäure mit 4-Chlor-1,2-benzendiol analog Beispiel 3a) ; Smp. 183-187°.

Beispiel 34

12,3 g (0,037 Mol) 5-Phenylsulfinyl-6-methyl-1,3-benzodioxol-2-carbonsäureäthylester werden mit 20 ml 2-n NaOH $^1/_2$ Stunde lang auf 60-70° erwärmt. Man kühlt ab, stellt mit 2-n HCl auf pH 1-2 und extrahiert mit Aethylacetat. Die Extraktlösung wird mit Wasser gewaschen, über Natriumsulfat getrocknet, filtriert und eingedampft. Der kristalline Rückstand von 10 g wird mit 300 ml Aethylacetat und 50 ml Aceton heiss gelöst, mit Aktivkohle versetzt und filtriert. Das Filtrat wird am Rotationsverdampfer auf 100 ml eingeengt. Es fallen dabei beigegefärbte Kristalle der 5-Phenylsulfinyl-6-methyl-1,3-benzodioxol-2-carbonsäure an ; Smp. ab 170° (Zers.).

Der oben eingesetze Aethylester wird auf folgende Art hergestellt :

a) 20,8 g (0,1 Mol) 5-Methyl-1,3-benzodioxol-2-carbonsäureäthylester (Beispiel 2b) und 19,2 g (0,125 Mol) Phenylsulfinsäurechlorid werden in 200 ml 1,2-Dichloräthan gelöst und mit einem Eisbad auf 5° gekühlt. Bei 5-10° werden unter Rühren in ca. 15 Minuten 13,3 g (0,1 Mol) Aluminiumchlorid eingetragen. Man rührt eine Stunde im Eisbad weiter und giesst dann auf Eiswasser aus. Die so entstehende Emulsion wird mit 200 ml Dichloräthan verdünnt und über eine Schicht eines Filterhilfsmittels (z. B. Hyflo) filtriert. Das Filtrat wird in einen Scheidetrichter gegeben und die Schichten getrennt. Die organische Phase wird über Natriumsulfat getrocknet, filtriert und zur Trockne eingedampft. Man erhält ein hellbraunes honigartiges Produkt, den 5-Phenylsulfinyl-6-methyl-1,3-benzodioxol-2-carbonsäureäthylester.

Beispiel 35

24,4 g (0,065 Mol) 5-Chlor-6-(4-chlorphenylsulfonyl)-1,3-benzodioxol-2-carbonsäure (nach Beispiel 25 erhalten) werden mit 250 ml Aethanol abs. und 0,2 ml HCl conc. versetzt und $^1/_2$ Stunde am Rückfluss gekocht. Man destilliert 100 ml Aethanol ab und aus der zurückbleibenden Lösung kristallisiert beim Abkühlen in groben, farblosen Kristallen der 5-Chlor-6-(4-chlorphenylsulfonyl)-1,3-benzodioxol-2-carbonsäureäthylester aus ; Smp. 122-123°.

Beispiel 36

3,88 g (0,006 9 Mol) 5-Chlor-6-(4-chlorphenylsulfonyl)-1,3-benzodioxol-2-carbonsäureäthylester werden in 20 ml Dimethylformamid gelöst und bei Raumtemperatur 0,71 g (0,012 Mol) Guanidin zugesetzt. Man lässt bei Raumtemperatur zwei Stunden lang rühren, dann giesst man auf 200 ml Wasser aus. Nach $^1/_2$ Stunde Rühren bildet sich eine Kristallsuspension. Man filtriert, wäscht mit Wasser und trocknet das Rohprodukt im Vacuumtrockenschrank bei 30-40°. Das Rohprodukt wird nun mit 25 ml Aethylacetat digeriert, wobei teilweise Lösung und Rekristallisation eintritt. Man kühlt $^1/_2$ Stunde im Eisbad, filtriert, trocknet und erhält, als beige Kristalle, das 5-Chlor-6-(4-chlorphenylsulfonyl)- 1,3-benzodioxol-2-carbonsäure-guanidid ; Smp. ab 215° (Zers.).

Beispiel 37

4,03 Mol 5-Chlor-6-(4-chlorphenylsulfonyl)-1,3-benzodioxol-2-carbonsäure-äthylester werden in 50 ml Dichlormethan bei Raumtemperatur gelöst. Unter kräftigem Rühren setzt man 50 ml einer konzentrierten wässrigen Ammoniaklösung zu und setzt das Rühren während 18 Stunden fort. Aus der anfänglichen Emulsion bildet sich eine dicke Kristallsuspension. Durch Zusatz von 6-n HCl stellt man jetzt bei Raumtemperatur auf pH 2-3, filtriert und wäscht mit Wasser nach. Nach dem Trocknen erhält man farblose Kristalle des 5-Chlor-6-(4-chlorphenylsulfonyl)-1,3-benzodioxol-2-carbonsäureamids ; Smp. 240-242°.

Beispiel 38

34,8 g (0,1 Mol) 5-Phenylsulfonyl-6-methyl-1,3-benzodioxol-2-carbonsäureäthylester (aus Beispiel 2) werden in 350 ml Dioxan abs. gelöst und in einem Druckautoklaven mit ca. 20 g Ammoniakgas behandelt. Man erhitzt das Reaktionsgemisch im Autoklaven für 10 Stunden auf 80°, entspannt und dampft die Lösung unter vermindertem Druck zur Trockne ein. Der Rückstand wird aus 120 ml Aethanol umkristallisiert, und man erhält farblose Kristalle von 5-Phenylsulfonyl-6-methyl-1,3-benzodioxol-2-carbonsäureamid ; Smp. 138-141°.

Beispiel 39

Tabletten enthaltend 50 mg 5-Methyl-6-phenylsulfonyl-1,3-benzodioxol-2-carbonsäure können beispielsweise in folgender Zusammensetzung hergestellt werden :

| Zusammensetzung | pro Tablette |
|---|---|
| 5-Methyl-6-phenylsulfonyl-1,3-benzodioxol-2-carbonsäure | 50 mg |
| Milchzucker | 50 mg |
| Weizenstärke | 73 mg |
| Kolloidale Kieselsäure | 13 mg |
| Talk | 12 mg |
| Magnesiumstearat | 2 mg |
| | 200 mg |

Herstellung

Der Wirkstoff wird mit Milchzucker, einem Teil der Weizenstärke und mit kolloidaler Kieselsäure gemischt und die Mischung durch ein Sieb getrieben. Ein weiterer Teil der Weizenstärke wird mit der 5-fachen Menge Wasser auf dem Wasserbad verkleistert und die Pulvermischung mit diesem Kleister angeknetet, bis eine schwach plastische Masse entstanden ist. Die Masse wird durch ein Sieb von ca. 3 mm Maschenweite getrieben, getrocknet und das trockene Granulat nochmals durch ein Sieb getrieben. Darauf werden die restliche Weizenstärke, Talk und Magnesiumstearat zugemischt. Die erhaltene Mischung wird zu Tabletten von 250 mg mit Bruchkerbe(n) verpresst.

Beispiel 40

Um 1000 Kapseln mit je 50 mg Wirkstoffgehalt herzustellen, mischt man 50,0 g 5-Methyl-6-phenylsulfonyl-1,3-benzodioxol-2-carbonsäure mit 223,0 g Lactose, befeuchtet die Mischung gleichmässig mit einer wässrigen Lösung von 2,0 g Gelatine und granuliert sie durch ein geeignetes Sieb (z. B. Sieb III nach Ph. Helv. V.). Das Granulat mischt man mit 10,0 g getrockneter Maisstärke und 15,0 g Talk und füllt es gleichmässig in 1000 Hartgelatinekapseln der Grösse 1.

Anstelle von 5-Methyl-6-phenylsulfonyl-1,3-benzodioxol-2-carbonsäure kann man in den Beispielen 39 und 40 auch eine andere Verbindung der allgemeinen Formel I oder ein pharmazeutisch annehmbares Salz einer zur Salzbildung befähigten Verbindung der allgemeinen Formel I, z. B. eine der in den Beispielen 3 bis 38 beschriebenen Verbindungen oder ein pharmazeutisch annehmbares Salz einer solchen, verwenden.

**Patentansprüche** (für die Vertragsstaaten : BE, CH, LI, DE, FR, GB, IT, LU, NL, SE)

1. Verbindungen der allgemeinen Formel I

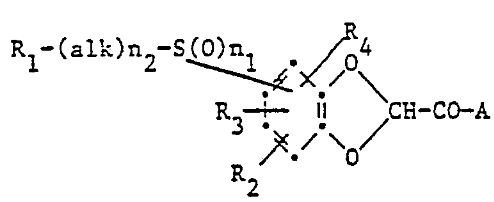

(I)

in denen $R_1$ ein unsubstituierter oder durch Halogen bis Atomnummer 35, Niederalkoxy oder Niederalkylthio substituierter Alkyl-, Alkenyl- oder Alkinylrest mit insgesamt, d. h. einschliesslich der Substituenten, höchstens 12 Kohlenstoffatomen, oder ein unsubstituierter oder durch Niederalkyl, Niedercycloalkyl, Niederalkoxy, Niederalkylthio, Niederalkylsulfonyl, Trifluormethyl, Hydroxy, Niederalkanoyl, Niederalkanoylamino, Niederalkoxycarbonylamino und/oder Niederalkylsulfonylamino substituierter Phenyl oder Naphthylrest, ein monocyclischer fünfgliedriger Heteroring aromatischen Charakters mit 2 Stickstoffatomen oder 1 Stickstoffatom und/oder ein Sauerstoff- oder Schwefelatom im Ring oder ein sechsgliedriger Heteroring aromatischen Charakters mit 1 oder 2 Stickstoffatomen im Ring oder ein bicylischer Rest, bestehend aus den genannten fünf- oder sechsgliedrigen Heteroringen aromatischen Charakters mit ankondensiertem Benzolring ist, alk einem Alkylen-, Alkenylen- oder Alkylidenrest mit höchstens 5 Kohlenstoffatomen, $n_1$ Null, eins oder zwei und $n_2$ Null oder eins bedeutet, $R_2$, $R_3$ und $R_4$ unabhängig voneinander Wasserstoff, Niederalkyl, Niederalkoxy oder Halogen bedeuten und A den Rest —O—$R_5$, worin $R_5$ für Wasserstoff oder einen unsubstituierten oder durch Niederalkoxy oder Halogen substituierten Alkylrest mit höchstens 12 C-Atomen, 2- oder 3-Niederalkenyl, 2-Niederalkinyl, Phenylniederalkyl oder Cinnamyl, in welchen der Phenylrest in gleicher Weise wie unter $R_1$ substituiert sein kann, oder den Rest

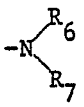

bedeutet, worin $R_6$ und $R_7$ unabhängig voneinander für Wasserstoff oder Niederalkyl, oder unter sich verbunden und zusammen mit dem anliegenden Stickstoffatom für gegebenenfalls niederalkylsubstituiertes Tetra- bis Hexamethylenimino, 4-Morpholinyl oder 1H-Tetrazol-1-yl stehen, Salze von Verbindungen der allgemeinen Formel I, in denen A $OR_5$ und darin $R_5$ Wasserstoff bedeuten, mit Basen, sowie Säureadditionssalze von Verbindungen der allgemeinen Formel I, deren Rest $R_1$ basischen Charakter aufweist, wobei die mit « nieder » bezeichneten Reste höchstens 7 Kohlenstoffatome aufweisen.

2. Verbindungen der im Anspruch 1 angegebenen allgemeinen Formel I, in denen $R_1$ durch Niederalkyl, Niederalkoxy, Halogen bis Atomnummer 35, Niederalkanoyl und/oder Niederalkanoylamino höchstens dreifach substituiertes oder unsubstituiertes Phenyl, Thienyl, Furyl oder ar-Benzothiazolyl, alk Alkylen, oder Alkenylen mit höchstens 3 Kohlenstoffatomen, $R_2$ Niederalkyl oder Halogen bis Atomnummer 35, $R_3$ Wasserstoff, Niederalkyl oder Halogen bis Atomnummer 35 und $R_4$ Wasserstoff bedeutet und $n_1$, $n_2$ und A die im Anspruch 1 angegebene Bedeutung haben, sowie die Salze von solchen Verbindungen, in denen $R_5$ Wasserstoff ist, mit Basen, wobei die mit « nieder » bezeichneten Reste höchstens 7 Kohlenstoffatome aufweisen.

3. Verbindungen der im Anspruch 1 angegebenen allgemeinen Formel I, in denen $R_1$ durch Niederalkyl, Niederalkoxy, Halogen bis Atomnummer 35, Niederalkanoyl und/oder Niederalkanoylamino höchstens dreifach substituiertes oder unsubstituiertes Phenyl oder Thienyl, alk Methylen, $n_1$ zwei, $n_2$ null oder eins, $R_2$ Niederalkyl oder Halogen, $R_3$ Wasserstoff oder Niederalkyl, $R_4$ Wasserstoff, und A $OR_5$ und darin $R_5$ Wasserstoff oder Niederalkyl, bedeutet sowie die pharmazeutisch annehmbaren Salze von solchen Verbindungen, in denen $R_5$ Wasserstoff ist, wobei die mit « nieder » bezeichneten Reste höchstens 7 Kohlenstoffatome aufweisen.

4. Verbindungen der im Anspruch 1 angegebenen allgemeinen Formel I, in denen $R_1$ durch Niederalkyl, Niederalkoxy und/oder Halogen bis Atomnummer 35, höchstens dreifach substituiertes oder unsubstituiertes Phenyl, alk Methylen, $n_1$ zwei, $n_2$ null oder eins, $R_2$ Niederalkyl oder Halogen, $R_3$ Wasserstoff oder Niederalkyl, $R_4$ Wasserstoff und A $OR_5$ und darin $R_5$ Wasserstoff oder Niederalkyl bedeutet, und der Phenylsulfonylrest in 5- oder 6-Stellung gebunden ist, sowie die pharmazeutisch annehmbaren Salze von solchen Verbindungen, in denen $R_5$ Wasserstoff ist, mit Basen, wobei die mit « nieder » bezeichneten Reste höchstens 7 Kohlenstoffatome aufweisen.

5. Verbindungen der im Anspruch 1 angegebenen allgemeinen Formel I, in denen $R_1$ durch Methyl, Methoxy und/oder Chlor, höchstens dreifach substituiertes oder unsubstituiertes Phenyl, alk Methylen, $n_1$ zwei, $n_1$ null oder eins, $R_2$ Methyl oder Chlor, $R_3$ und $R_4$ Wasserstoff und A $OR_5$ und darin $R_5$ Wasserstoff oder Niederalkyl bedeuten, und der Phenylsulfonylrest in 5- oder 6-Stellung gebunden ist, sowie die pharmazeutisch annehmbaren Salze von solchen Verbindungen, in denen $R_5$ Wasserstoff ist, mit Basen, wobei die mit « nieder » bezeichneten Reste höchstens 7 Kohlenstoffatome aufweisen.

6. Die 5-Methyl-6-phenylsulfonyl-1,3-benzodioxol-2-carbonsäure und ihre pharmazeutisch annehmbaren Salze mit Basen.

7. Die 5-Chlor-6-(2-chlorphenylsulfonyl)-1,3-benzodioxol-2-carbonsäure und ihre pharmazeutisch annehmbaren Salze mit Basen.

8. Die 5-(2-Chlorphenylsulfonyl)-6-methyl-1,3-benzodioxol-2-carbonsäure und ihre pharmazeutisch annehmbaren Salze mit Basen.

9. Die 5-Chlor-6-(4-chlorphenylsulfonyl)-1,3-benzodioxol-2-carbonsäure und ihre pharmazeutisch annehmbaren Salze mit Basen.

10. Die 5-(3-Chlorphenylsulfonyl)-6-methyl-1,3-benzodioxol-2-carbonsäure und ihre pharmazeutisch annehmbaren Salze mit Basen.

11. Die 5-Chlor-6-(3-chlorphenylsulfonyl)-1,3-benzodioxol-2-carbonsäure und ihre pharmazeutisch annehmbaren Salze mit Basen.

12. 5-Chlor-6-(4-chlorphenylsulfonyl)-1,3-benzodioxol-2-carbonsäureäthylester.

13. Pharmazeutische Präparate, enthaltend eine Verbindung gemäss einem der Ansprüche 1-9 oder ein pharmazeutisch annehmbares Salz einer zur Salzbildung befähigten Verbindung gemäss einem dieser Ansprüche.

14. Pharmazeutische Präparate, enthaltend eine Verbindung gemäss einem der Ansprüche 10-12 oder ein pharmazeutisch annehmbares Salz einer zur Salzbildung befähigten Verbindung gemäss einem dieser Ansprüche.

15. Verbindungen gemäss einem der Ansprüche 1-9 und pharmazeutisch annehmbare Salze von zur Salzbildung befähigten Verbindungen gemäss einem dieser Ansprüche zur Anwendung in einem Verfahren zur therapeutischen Behandlung des tierischen oder menschlichen Körpers.

16. Verbindungen gemäss einem der Ansprüche 10-12 und pharmazeutisch annehmbare Salze von zur Salzbildung befähigten Verbindungen gemäss einem dieser Ansprüche zur Anwendung in einem Verfahren zur therapeutischen Behandlung des tierischen oder menschlichen Körpers.

17. Verbindungen gemäss einem der Ansprüche 1-9 und pharmazeutisch annehmbare Salze von zur Salzbildung befähigten Verbindungen gemäss einem dieser Ansprüche als Diuretika mit urikosurischer Zusatzwirkung.

18. Verbindungen gemäss einem der Ansprüche 10-12 und pharmazeutisch annehmbare Salze von zur Salzbildung befähigten Verbindungen gemäss einem dieser Ansprüche als Diurektika mit urikosurischer Zusatzwirkung.

19. Verwendung von Verbindungen gemäss einem der Ansprüche 1-9 14 oder von pharmazeutisch annehmbaren Salzen von zur Salzbildung befähigten Verbindungen gemäss einem dieser Ansprüche zur Herstellung von Arzneimitteln auf nicht-chemischem Wege.

20. Verwendung von Verbindungen gemäss einem der Ansprüche 10-12 oder von pharmazeutisch annehmbaren Salzen von zur Salzbildung befähigten Verbindungen gemäss einem dieser Ansprüche zur Herstellung von Arzneimitteln auf nicht-chemischem Wege.

21. Verfahren zur Herstellung von Verbindungen der im Anspruch 1 angegebenen allgemeinen Formel I, in welcher $R_1$, $R_2$, $R_3$, $R_4$, alk, $n_1$, $n_2$ und A die dort angegebene Bedeutung haben, und von Salzen solcher Verbindungen, in denen A $OR_5$ und darin $R_5$ Wasserstoff ist, oder welche basischen Charakter besitzen, dadurch gekennzeichnet, dass man

a) eine Verbindung der allgemeinen Formel II

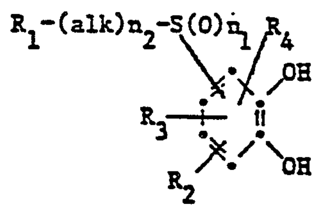

(II)

in welcher $R_1$, alk, $n_1$, $n_2$ $R_2$, $R_3$ und $R_4$ die im Anspruch 1 angegebene Bedeutung haben, oder ein Salz davon mit einer Verbindung der allgemeinen Formel III

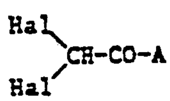

(III)

in welcher Hal Halogen bedeutet und A die im Anspruch 1 angegebene Bedeutung hat, oder einem Salz einer solchen Verbindung, in der A $OR_5$ und darin $R_5$ Wasserstoff bedeutet, umsetzt, oder

b) in einer Verbindung der allgemeinen Formel IV

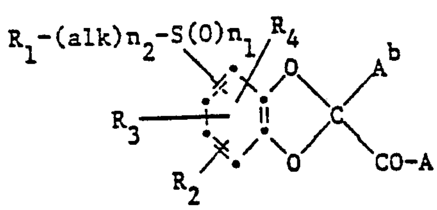

(IV)

in welcher $A^b$ Carboxy, Niederalkoxycarbonyl oder Acetyl bedeutet, und $R_1$, alk, $n_1$, $n_2$, $R_2$, $R_3$, $R_4$ und A die im Anspruch 1 angegebene Bedeutung haben, den Rest $A^b$ durch Wasserstoff ersetzt, oder

c) zur Herstellung einer Verbindung der allgemeinen Formel I, in welcher A die im Anspruch 1

angegebene Bedeutung hat mit Ausnahme eines Restes $OR_5$, in welchem $R_5$ Wasserstoff bedeutet, und $R_1$, alk, $n_2$, $R_2$, $R_3$ und $R_4$ die im Anspruch 1 angegebene Bedeutung haben und $n_1$ eins oder zwei bedeutet, eine Verbindung der allgemeinen Formel V

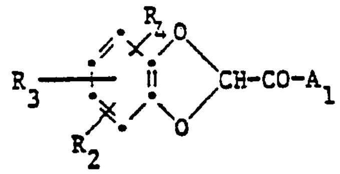

(V)

mit einer Verbindung der allgemeinen Formel VI,

$$R_1\text{—(alk)}n_2\text{—S(O)}n_1{}^c\text{—OH} \qquad \text{(VI)},$$

oder einem Anhydrid derselben, worin $A_1$ die im Anspruch 1 für A angegebene Bedeutung hat mit Ausnahme eines Restes $OR_5$, in welchem $R_5$ Wasserstoff bedeutet, $n_1{}^c$ eins oder zwei bedeutet und $R_2$, $R_3$ und $R_4$ bzw. $R_1$, alk und $n_2$ die im Anspruch 1 angegebene Bedeutung haben, umsetzt, oder

d) zur Herstellung einer Verbindungen, in welcher $n_2$ eins oder $R_1$ ein definitionsgemässer aliphatischer Rest ist und $R_1$ bzw. $n_2$, alk, $n_2$, $R_2$, $R_3$, $R_4$ und A die im Anspruch 1 angegebene Bedeutung haben, eine Verbindung der allgemeinen Formel VII

$$R_1\text{—(alk)}n_2{}^d\text{—}Z^d \qquad \text{(VII)}$$

in welcher $Z^d$ eine reaktionsfähig veresterte Hydroxygruppe, insbesondere Halogen mit einer Atomnummer von mindestens 17, bedeutet, $R_1$ die im Anspruch 1 angegebene Bedeutung hat und $n_2$ eins bedeutet, oder falls $R_2$ ein aliphatischer Rest ist, auch null sein kann, oder eine Verbindung der allgemeinen Formel VIII

$$R_1\text{—(alk)}n_1\text{—}\overset{\oplus}{N}\equiv N \quad Z^{dd\ominus} \qquad \text{(VIII)},$$

in welcher $Z^{dd\ominus}$ ein einwertiges Anion oder das Normaläquivalent eines mehrwertigen Anions bedeutet und $R_1$ und $n_1$ die in Anspruch 1 angegebene Bedeutung haben, mit einem Salz einer Verbindung der allgemeinen Formel IX

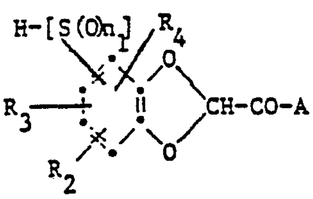

(IX)

in welcher $n_1$, $R_2$, $R_3$, $R_4$ und A die im Anspruch 1 angegebene Bedeutung haben, umsetzt oder

e) eine gegebenenfalls in situ gebildete Metallverbindung der allgemeinen Formel Xa oder Xb

$$R_1\text{-(alk)}n_2\text{-}M_1 \qquad \text{(Xa)}$$

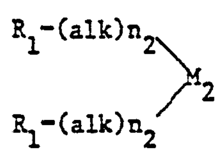

(Xb)

in welchen $M_1$ ein einwertiges bzw. $M_2$ ein zweiwertiges Metallion bedeutet und R, alk und $n_2$ die im Anspruch 1 angegebene Bedeutung haben, mit einer Verbindung der allgemeinen Formel XIa oder XIb,

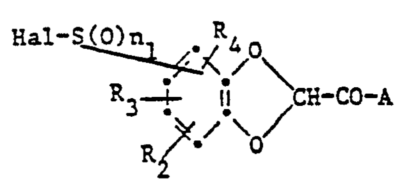

(XIa)

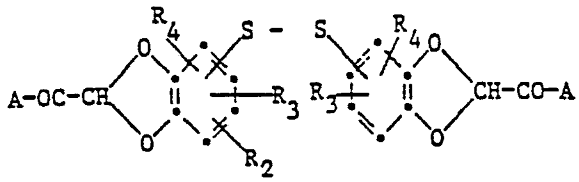

(XIb)

in welchen Hal Halogen bedeutet und $n_1$, $R_2$, $R_3$, $R_4$ und A die Anspruch 1 angegebene Bedeutung haben, umsetzt, oder

f) zur Herstellung einer Verbindung der allgemeinen Formel I, in welcher A $OR_5$ und darin $R_5$ Wasserstoff bedeutet und $R_1$, alk, $n_1$, $n_2$, $R_2$, $R_3$ und $R_4$ die im Anspruch 1 angegebene Bedeutung haben, oder eines Salzes dieser Verbindung, in einer Verbindung der allgemeinen Formel XIII,

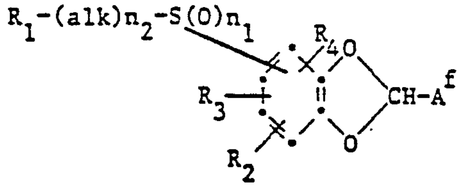

(XIII)

in welcher $A^f$ eine in die Carboxygruppe überführbare Gruppe bedeutet und $R_1$, alk, $n_1$, $n_2$, $R_2$, $R_3$ und $R_4$ die im Anspruch 1 angegebene Bedeutung haben, die Gruppe $A^f$ in die Carboxygruppe in freier oder Salzform überführt, oder

g) zur Herstellung einer Verbindung der allgemeinen Formel I, in welcher A die im Anspruch 1 angegebene Bedeutung hat mit Ausnahme eines Restes $OR_5$, in welchem $R_5$ Wasserstoff bedeutet, und $R_1$, alk, $n_1$, $n_2$, $R_2$ und $R_3$ die im Anspruch 1 angegebene Bedeutung haben, in einer Verbindung der allgemeinen Formel XIV

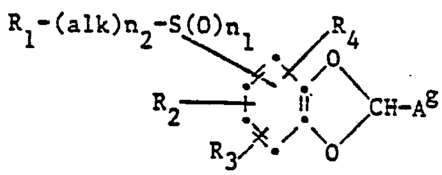

(XIV)

in welcher $A^g$ einen in einen Rest CO—$A_1$, in welchem $A_1$ die im Anspruch 1 für A angegebene Bedeutung hat mit Ausnahme eines Restes $OR_5$, in welchem $R_5$ Wasserstoff bedeutet, überführbaren Rest bedeutet, und $R_1$, alk, $n_1$, $n_2$, $R_2$, $R_3$ und $R_4$ die in Anspruch 1 angegebene Bedeutung haben, den Rest $A^g$ in den Rest —CO—$A_1$ überführt, und

h) gewünschtenfalls eine Verbindung der allgemeinen Formel I, in der $n_1$ null oder eins bedeutet, zur entsprechenden Verbindung oxidiert, in der $n_1$ eins oder zwei bedeutet, oder

i) gewünschtenfalls eine Verbindung der allgemeinen Formel I in der $n_1$ zwei oder eins bedeutet, zur entsprechenden Verbindung reduziert, in der $n_1$ eins oder null bedeutet, und/oder gewünschtenfalls eine erhaltene Verbindung der allgemeinen Formel I in anderer an sich bekannter Weise in eine andere Verbindung der allgemeinen Formel I, überführt, und/oder eine als Racemat erhaltene Verbindung der allgemeinen Formel I in die optischen Antipoden zerlegt, und/oder eine erhaltene Verbindung der allgemeinen Formel I, in welcher A $OR_5$ und darin $R_5$ Wasserstoff bedeutet, in ein Salz mit eine Base überführt oder eine solche Verbindung aus einem erhaltenen Salz freisetzt, oder eine erhaltene Verbindung der allgemeinen Formel I von basischem Charakter in ein Säureadditionssalz überführt oder eine solche Verbindung aus einem erhaltenen Salz freisetzt.

22. Die nach dem Verfahren des Anspruches 21 erhältlichen Verbindungen.

**Patentansprüche** (für den Vertragsstaat AT)

1. Verfahren zur Herstellung von Verbindungen der Formel I

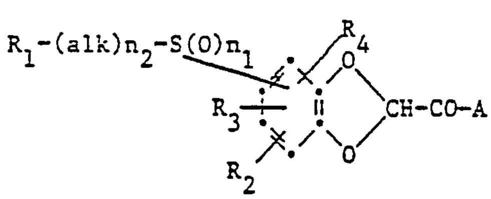

(I)

in denen $R_1$ ein unsubstituierter oder durch Halogen bis Atomnummer 35, Niederalkoxy oder Niederalkylthio substituierter Alkyl-, Alkenyl- oder Alkinylrest mit insgesamt, d. h. einschliesslich der Substituenten, höchstens 12 Kohlenstoffatomen, oder ein unsubstituierter oder durch Niederalkyl, Niedercycloalkyl, Niederalkoxy, Niederalkylthio, Niederalkylsulfonyl, Trifluormethyl, Hydroxy, Niederalkanoyl, Niederalkanoylamino, Niederalkoxycarbonylamino und/oder Niederalkylsulfonylamino substituierter Phenyl- oder Naphthylrest, ein monocyclischer fünfgliedriger Heteroring aromatischen Charakters mit 2 Stickstoffatomen oder 1 Stickstoffatom und/oder ein Sauerstoff- oder Schwefelatom im Ring oder ein sechsgliedriger Heteroring aromatischen Charakters mit 1 oder 2 Stickstoffatomen im Ring oder ein bicyclischer Rest, bestehend aus den genannten fünf- oder sechsgliedrigen Heteroringen aromatischen Charakters mit ankondensiertem Benzolring ist, alk einen Alkylen-, Alkenylen- oder Alkylidenrest mit höchstens 5 Kohlenstoffatomen, $n_1$ Null, eins oder zwei und $n_2$ Null oder eins bedeutet, $R_2$, $R_3$ und $R_4$ unabhängig voneinander Wasserstoff, Niederalkyl, Niederalkoxy oder Halogen bedeuten und A den Rest —O—$R_5$, worin $R_5$ für Wasserstoff oder einen unsubstituierten oder durch Niederalkoxy oder Halogen substituierten Alkylrest mit höchstens 12 C-Atomen, 2- oder 3-Niederalkenyl, 2-Niederalkinyl, Phenylniederalkyl oder Cinnamyl, in welchen der Phenylrest in gleicher Weise wie unter $R_1$ substituiert sein kann, oder den Rest

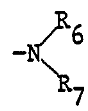

bedeutet, worin $R_6$ und $R_7$ unabhängig voneinander für Wasserstoff oder Niederalkyl, oder unter sich verbunden und zusammen mit dem anliegenden Stickstoffatom für gegebenenfalls niederalkylsubstituiertes Tetra- bis Hexamethylenimino, 4-Morpholinyl oder 1H-Tetrazol-1-yl stehen, Salze von Verbindungen der allgemeinen Formel I, in denen A $OR_5$ und darin $R_5$ Wasserstoff bedeuten, mit Basen, sowie Säureadditionssalze von Verbindungen der allgemeinen Formel I, deren Rest $R_1$ basischen Charakter aufweist, wobei die mit « nieder » bezeichneten Reste höchstens 7 Kohlenstoffatome aufweisen, dadurch gekennzeichnet, dass man

a) eine Verbindung der allgemeinen Formel II

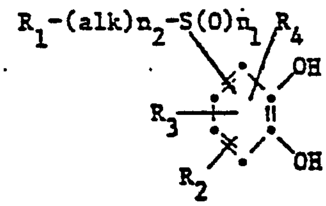

(II)

in welcher $R_1$, alk, $n_1$, $n_2$, $R_2$, $R_3$ und $R_4$ die oben angegebene Bedeutung haben, oder ein Salz davon mit einer Verbindung der allgemeinen Formel III

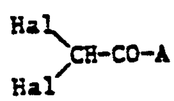

(III)

in welcher Hal Halogen bedeutet und A die im oben angegebene Bedeutung hat, oder einem Salz einer solchen Verbindung, in der A $OR_5$ und darin $R_5$ Wasserstoff bedeutet, umsetzt, oder

b) in einer Verbindung der allgemeinen Formel IV

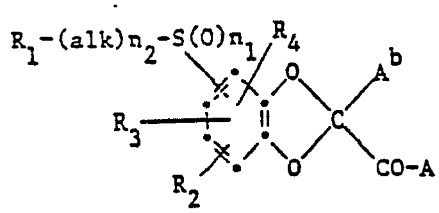

(IV)

in welcher $A^b$ Carboxy, Niederalkoxycarbonyl oder Acetyl bedeutet, und $R_1$, alk, $n_1$, $n_2$, $R_2$, $R_3$, $R_4$ und A die im Anspruch 1 angegebene Bedeutung haben, den Rest $A^b$ durch Wasserstoff ersetzt, oder

c) zur Herstellung einer Verbindung der allgemeinen Formel I, in welcher A die oben angegebene Bedeutung hat mit Ausnahme eines Restes $OR_5$, in welchem $R_5$ Wasserstoff bedeutet, und $R_1$, alk, $n_2$, $R_2$, $R_3$ und $R_4$ die oben angegebene Bedeutung haben und $n_1$ eins oder zwei bedeutet, eine Verbindung der

allgemeinen Formel V

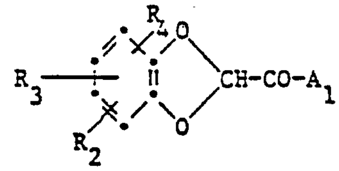

(V)

mit einer Verbindung der allgemeinen Formel VI,

$$R_1\text{—(alk)}n_2\text{—S(O)}n_1^c\text{—OH} \qquad \text{(VI)},$$

oder einem Anhydrid derselben, worin $A_1$ die oben für A angegebene Bedeutung hat mit Ausnahme eines Restes $OR_5$, in welchem $R_5$ Wasserstoff bedeutet, $n_1^c$ eins oder zwei bedeutet und $R_2$, $R_3$ und $R_4$ bzw. $R_1$, alk und $n_2$ die oben angegebene Bedeutung haben umsetzt, oder

d) zur Herstellung einer Verbindungen, in welcher $n_2$ eins oder $R_1$ ein definitionsgemässer aliphatischer Rest ist und $R_1$ bzw. $n_2$, alk, $n_2$, $R_2$, $R_3$, $R_4$ und A die oben angegebene Bedeutung haben, eine Verbindung der allgemeinen Formel VII

$$R_1\text{—(alk)}n_2^d\text{—}Z^d \qquad \text{(VII)}$$

in welcher $Z^d$ eine reaktionsfähig veresterte Hydroxygruppe, insbesondere Halogen mit einer Atomnummer von mindestens 17, bedeutet, $R_1$ die oben angegebene Bedeutung hat und $n_2$ eins bedeutet, oder falls $R_2$ ein aliphatischer Rest ist, auch null sein kann, oder eine Verbindung der allgemeinen Formel VIII

$$R_1\text{—(alk)}n_1\text{—}\overset{\oplus}{N}\equiv N \quad Z^{dd\ominus} \qquad \text{(VIII)},$$

in welcher $Z^{dd\ominus}$ ein einwertiges Anion oder das Normaläquivalent eines mehrwertigen Anions bedeutet und $R_1$ und $n_1$ die oben angegebene Bedeutung haben, mit einem Salz einer Verbindung der allgemeinen Formel IX

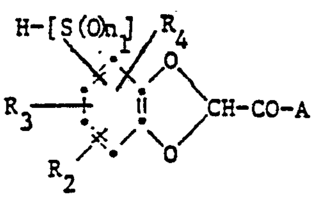

(IX)

in welcher $n_1$, $R_2$, $R_3$, $R_4$ und A die im Anspruch 1 angegebene Bedeutung haben, umsetzt, oder

e) eine gegebenenfalls in situ gebildete Metallverbindung der allgemeinen Formel Xa oder Xb

$R_1$-(alk)$n_2$-$M_1$ (Xa)

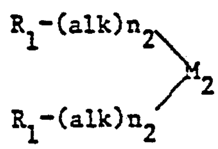

(Xb)

in welchen $M_1$ ein einwertiges bzw. $M_2$ ein zweiwertiges Metallion bedeutet und R, alk und $n_2$ die im oben angegebene Bedeutung haben, mit einer Verbindung der allgemeinen Formel XIa oder XIb,

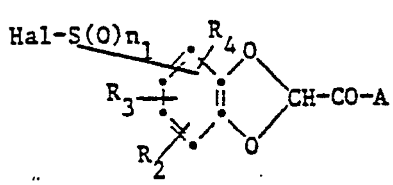

(XIa)

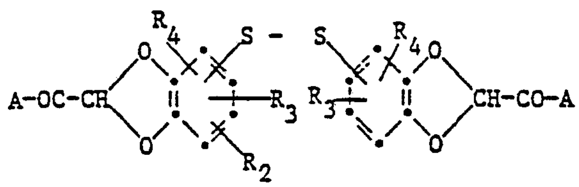

(XIb)

in welchen Hal Halogen bedeutet und $n_1$, $R_2$, $R_3$, $R_4$ und A die oben angegebene Bedeutung haben, umsetzt, oder

f) zur Herstellung einer Verbindung der allgemeinen Formel I, in welcher A $OR_5$ und darin $R_5$ Wasserstoff bedeutet und $R_1$, alk, $n_1$, $n_2$, $R_2$, $R_3$ und $R_4$ die oben angegebene Bedeutung haben, oder eines Salzes dieser Verbindung, in einer Verbindung der allgemeinen Formel XIII,

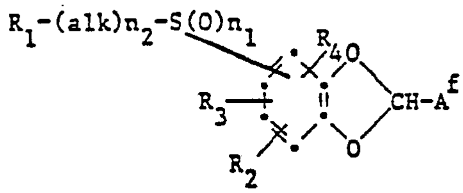

(XIII)

in welcher $A^f$ eine in die Carboxygruppe überführbare Gruppe bedeutet und $R_1$, alk, $n_1$, $n_2$, $R_2$, $R_3$ und $R_4$ die oben angegebene Bedeutung haben, die Gruppe $A^f$ in die Carboxygruppe in freier oder Salzform überführt, oder

g) zur Herstellung einer Verbindung der allgemeinen Formel I, in welcher A die oben angegebene Bedeutung hat mit Ausnahme eines Restes $OR_5$, in welchem $R_5$ Wasserstoff bedeutet, und $R_1$, alk, $n_1$, $n_2$ und $R_3$ und $R_3$ die oben angegebene Bedeutung haben, in einer Verbindung der allgemeinen Formel XIV

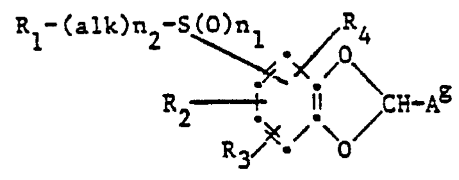

(XIV)

in welcher $A^g$ einen in einen Rest $CO—A_1$, in welchem $A_1$ die oben für A angegebene Bedeutung hat mit Ausnahme eines Restes $OR_5$, in welchem $R_5$ Wasserstoff bedeutet, überführbaren Rest bedeutet, und $R_1$, alk, $n_1$, $n_2$, $R_2$, $R_3$ und $R_4$ die in Anspruch 1 angegebene Bedeutung haben, den Rest $A^g$ in den Rest $—CO—A_1$ überführt, und

h) gewünschtenfalls eine Verbindung der allgemeinen Formel I, in der $n_1$ null oder eins bedeutet, zur entsprechenden Verbindung oxidiert, in der $n_1$ eins oder zwei bedeutet, oder

i) gewünschtenfalls eine Verbindung der allgemeinen Formel I in der $n_1$ zwei oder eins bedeutet, zur entsprechenden Verbindung reduziert, in der $n_1$ eins oder null bedeutet, und/oder gewünschtenfalls eine erhaltene Verbindung der allgemeinen Formel I in anderer an sich bekannter Weise in eine andere Verbindung der allgemeinen Formel I, überführt, und/oder eine als Racemat erhaltene Verbindung der allgemeinen Formel I in die optischen Antipoden zerlegt, und/oder eine erhaltene Verbindung der allgemeinen Formel I, in welcher A $OR_5$ und darin $R_5$ Wasserstoff bedeutet, in ein Salz mit einer Base überführt oder eine solche Verbindung aus einem erhaltenen Salz freisetzt, oder eine erhaltene Verbindung der allgemeinen Formel I von basischem Charakter in ein Säureadditionssalz überführt oder eine solche Verbindung aus einem erhaltenen Salz freisetzt.

2. Verfahren nach Patentanspruch 1, dadurch gekennzeichnet, dass man Verbindungen der Formel I, in denen $R_1$ durch Niederalkyl, Niederalkoxy, Halogen bis Atomnummer 35, Niederalkanoyl und/oder Niederalkanoylamino höchstens dreifach substituiertes oder unsubstituiertes Phenyl, Thienyl, Furyl oder ar-Benzothiazolyl, alk Alkylen, Alkyliden oder Alkenylen mit höchstens 3 Kohlenstoffatomen, $R_2$ Niederalkyl oder Halogen bis Atomnummer 35, $R_3$ Wasserstoff, Niederalkyl oder Halogen bis Atomnummer 35 und $R_4$ Wasserstoff bedeutet und $n_1$, $n_2$ und A die im Anspruch 1 angegebene Bedeutung haben, sowie die Salze von solchen Verbindungen, in denen $R_5$ Wasserstoff ist, mit Basen herstellt, wobei mit « nieder » bezeichneten Reste höchstens 7 Kohlenstoffatome aufweisen.

3. Verfahren nach Patentanspruch 1, dadurch gekennzeichnet, dass man Verbindungen der Formel I in denen $R_1$ durch Niederalkyl, Niederalkoxy, Halogen bis Atomnummer 35, Niederalkanoyl und/oder Niederalkanoylamino höchstens dreifach substituiertes oder unsubstituiertes Phenyl oder Thienyl, alk Methylen, $n_1$ zwei, $n_2$ null oder eins, $R_2$ Niederalkyl oder Halogen, $R_3$ Wasserstoff oder Niederalkyl, $R_4$ Wasserstoff, und A $OR_5$ und darin $R_5$ Wasserstoff oder Niederalkyl, bedeutet sowie die pharmazeutisch annehmbaren Salze von solchen Verbindungen, in denen $R_5$ Wasserstoff ist, mit Basen herstellt, wobei die mit « nieder » bezeichneten Reste höchstens 7 Kohlenstoffatome aufweisen.

4. Verfahren nach Patentanspruch 1, dadurch gekennzeichnet, dass man Verbindungen der Formel I, in denen $R_1$ durch Niederalkyl, Niederalkoxy und/oder Halogen bis Atomnummer 35, höchstens dreifach substituiertes oder unsubstituiertes Phenyl, alk Methylen, $n_1$ zwei, $n_2$ null oder eins, $R_2$ Niederalkyl oder Halogen, $R_3$ Wasserstoff oder Niederalkyl, $R_4$ Wasserstoff und A $OR_5$ und darin $R_5$ Wasserstoff oder Niederalkyl bedeutet, und der Phenylsulfonylrest in 5- oder 6-Stellung gebunden ist, sowie die pharmazeutisch annehmbaren Salze von solchen Verbindungen, in denen $R_5$ Wasserstoff ist, mit Basen

herstellt, wobei die mit « nieder » bezeichneten Reste höchstens 7 Kohlenstoffatome aufweisen.

5. Verfahren nach Patentanspruch 1, dadurch gekennzeichnet, dass man Verbindungen der Formel I, in denen $R_1$ durch Methyl, Methoxy und/oder Chlor, höchstens dreifach substituiertes oder unsubstituiertes Phenyl, alk Methylen, $n_1$ zwei, $n_1$ null oder eins, $R_2$ Methyl oder Chlor, $R_3$ und $R_4$ Wasserstoff und A $OR_5$ und darin $R_5$ Wasserstoff oder Niederalkyl bedeuten, und der Phenylsulfonylrest in 5- oder 6-Stellung gebunden ist, sowie die pharmazeutisch annehmbaren Salze von solchen Verbindungen, in denen $R_5$ Wasserstoff ist, mit Basen herstellt, wobei die mit « nieder » bezeichneten Reste höchstens 7 Kohlenstoffatome aufweisen.

6. Verfahren nach Patentanspruch 1, dadurch gekennzeichnet, dass man die 5-Methyl-6-phenylsulfonyl-1,3-benzodioxol-2-carbonsäure und ihre pharmazeutisch annehmbaren Salze mit Basen herstellt.

7. Verfahren nach Patentanspruch 1, dadurch gekennzeichnet, dass man die 5-Chlor-6-(2-chlorphenylsulfonyl)-1,3-benzodioxol-2-carbonsäure und ihre pharmazeutisch annehmbaren Salze mit Basen herstellt.

8. Verfahren nach Patentanspruch 1, dadurch gekennzeichnet, dass man die 5-(2-Chlorphenylsulfonyl)-6-methyl-1,3-benzodioxol-2-carbonsäure und ihre pharmazeutisch annehmbaren Salze mit Basen herstellt.

9. Verfahren nach Patentanspruch 1, dadurch gekennzeichnet, dass man die 5-Chlor-6-(4-chlorphenylsulfonyl)-1,3-benzodioxol-2-carbonsäure und ihre pharmazeutisch annehmbaren Salze mit Basen herstellt.

10. Verfahren nach Patentanspruch 1, dadurch gekennzeichnet, dass man die 5-(3-Chlorphenylsulfonyl)-6-methyl-1,3-benzodioxol-2-carbonsäure und ihre pharmazeutisch annehmbaren Salze mit Basen herstellt.

11. Verfahren nach Patentanspruch 1, dadurch gekennzeichnet, dass man die 5-Chlor-6-(3-chlorphenylsulfonyl)-1,3-benzodioxol-2-carbonsäure und ihre pharmazeutisch annehmbaren Salze mit Basen herstellt.

12. Verfahren nach Patentanspruch 1, dadurch gekennzeichnet, dass man den 5-Chlor-6-(4-chlorphenylsulfonyl)-1,3-benzodioxol-2-carbonsäure herstellt.

13. Verfahren zur Herstellung eines pharmazeutischen Präparates, gekennzeichnet durch die Verarbeitung eines erfindungsgemässen Wirkstoffes nach den Ansprüchen 1-8 mit einem pharmazeutischen Trägermittel.

14. Verfahren zur Herstellung eines pharmazeutischen Präparates, gekennzeichnet durch die Verarbeitung eines erfindungsgemässen Wirkstoffes nach den Ansprüchen 9-11 mit einem pharmazeutischen Trägermittel.

**Claims** (for the Contracting States : BE, CH, LI, DE, FR, GB, IT, LU, NL, SE)

1. Compounds of the general formula I

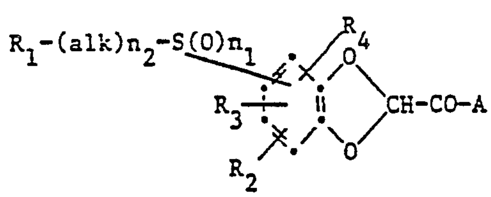

(I)

in which $R_1$ represents an alkyl, alkenyl or alkynyl radical that is unsubstituted or substituted by halogen having an atomic number of up to 35, lower alkoxy or by lower alkylthio and has, in total, that is to say including the substituents, a maximum of 12 carbon atoms, or represents a phenyl or naphthyl radical that is unsubstituted or substituted by lower alkyl, lower cycloalkyl, lower alkoxy, lower alkylthio, lower alkylsulphonyl, trifluoromethyl, hydroxy, lower alkanoyl, lower alkanoylamino, lower alkoxycarbonylamino and/or by lower alkylsulphonylamino, a monocyclic five-membered hetero ring of aromatic character having two nitrogen atoms or one nitrogen atom and/or one oxygen or sulphur atom in the ring or a six-membered hetero ring of aromatic character having one or two nitrogen atoms in the ring, or a bicyclic radical consisting of the said five- or six-membered hetero rings of aromatic character with a fused benzene ring, alk represents an alkylene, alkenylene or alkylidene radical having a maximum of 5 carbon atoms, $n_1$ represents 0, 1 or 2 and $n_2$ represents 0 or 1, $R_2$, $R_3$ and $R_4$ each represents, independently of the others, hydrogen, lower alkyl, lower alkoxy or halogen, and A represents the radical —O—$R_5$ wherein $R_5$ represents hydrogen or an alkyl radical having a maximum of 12 carbon atoms that is unsubstituted or is substituted by lower alkoxy or by halogen, 2- or 3-lower alkenyl, 2-lower alkynyl, phenyl-lower alkyl or cinnamyl, in which the phenyl radical may be substituted in the same manner as under $R_1$, or A represents the radical

(See scheme page 33)

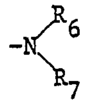

in which either $R_6$ and $R_7$ each represents, independently of the other, hydrogen or lower alkyl, or $R_6$ and $R_7$ are bonded to one another and, together with the adjacent nitrogen atom, represent optionally lower alkyl-substituted tetra- to hexa-methyleneimino, 4-morpholinyl or 1H-tetrazol-1-yl, salts of compounds of the general formula I in which A represents $OR_5$ wherein $R_5$ represents hydrogen, with bases, and acid addition salts of compounds of the general formula I in which the radical $R_1$ has a basic character, radicals designated « lower » having a maximum of 7 carbon atoms.

2. Compounds of the general formula I given in claim 1 in which $R_1$ represents phenyl, thienyl, furyl or ar-benzothiazolyl, each of which is unsubstituted or substituted a maximum of three times by lower alkyl, lower alkoxy, halogen having an atomic number of up to 35, lower alkanoyl and/or by lower alkanoylamino, alk represents alkylene, alkylidene or alkenylene having a maximum of 3 carbon atoms, $R_2$ represents lower alkyl or halogen having an atomic number of up to 35, $R_3$ represents hydrogen, lower alkyl or halogen having an atomic number of up to 35, $R_4$ represents hydrogen and $n_1$, $n_2$ and A have the meanings given in claim 1, and the salts of those compounds in which $R_5$ represents hydrogen with bases, radicals designated « lower » having a maximum of 7 carbon atoms.

3. Compounds of the general formula I given in claim 1 in which $R_1$ represents phenyl or thienyl, each of which is unsubstituted or substituted a maximum of three times by lower alkyl, lower alkoxy, halogen having an atomic number of up to 35, lower alkanoyl and/or by lower alkanoylamino, alk represents methylene, $n_1$ represents 2, $n_2$ represents 0 or 1, $R_2$ represents lower alkyl or halogen, $R_3$ represents hydrogen or lower alkyl, $R_4$ represents hydrogen, and A represents $OR_5$ wherein $R_5$ represents hydrogen or lower alkyl, and the pharmaceutically acceptable salts of those compounds in which $R_5$ represents hydrogen, radicals designated « lower » having a maximum of 7 carbon atoms.

4. Compounds of the general formula I given in claim 1 in which $R_1$ represents phenyl that is unsubstituted or substituted a maximum of three times by lower alkyl, lower alkoxy and/or by halogen having an atomic number of up to 35, alk represents methylene, $n_1$ represents 2, $n_2$ represents 0 or 1, $R_2$ represents lower alkyl or halogen, $R_3$ represents hydrogen or lower alkyl, $R_4$ represents hydrogen, and A represents $OR_5$ wherein $R_5$ represents hydrogen or lower alkyl, and the phenylsulphonyl radical is bonded in the 5- or 6-position, and the pharmaceutically acceptable salts of those compound in which $R_5$ represents hydrogen with bases, radicals designated « lower » having a maximum of 7 carbon atoms.

5. Compounds of the general formula I given in claim 1 in which $R_1$ represents phenyl that is unsubstituted or substituted a maximum of three times by methyl, methoxy and/or by chlorine, alk represents methylene, $n_1$ represents 2, $n_1$ represents 0 or 1, $R_2$ represents methyl or chlorine, $R_3$ and $R_4$ represent hydrogen, and A represents $OR_5$ wherein $R_5$ represents hydrogen or lower alkyl, and the phenylsulphonyl radical is bonded in the 5- or 6-position, and the pharmaceutically acceptable salts of those compounds in which $R_5$ represents hydrogen with bases, radicals designated « lower » having a maximum of 7 carbon atoms.

6. 5-methyl-6-phenylsulphonyl-1,3-benzodioxole-2-carboxylic acid and the pharmaceutically acceptable salts thereof with bases.

7. 5-chloro-6-(2-chlorophenylsulphonyl)-1,3-benzodioxole-2-carboxylic acid and the pharmaceutically acceptable salts thereof with bases.

8. 5-(2-chlorophenylsulphonyl)-6-methyl-1,3-benzodioxole-2-carboxylic acid and the pharmaceutically acceptable salts thereof with bases.

9. 5-chloro-6-(4-chlorophenylsulphonyl)-1,3-benzodioxole-2-carboxylic acid and the pharmaceutically acceptable salts thereof with bases.

10. 5-(3-chlorophenylsulphonyl)-6-methyl-1,3-benzodioxole-2-carboxylic acid and the pharmaceutically acceptable salts thereof with bases.

11. 5-chloro-6-(3-chlorophenylsulphonyl)-1,3-benzodioxole-2-carboxylic acid and the pharmaceutically acceptable salts thereof with bases.

12. 5-chloro-6-(4-chlorophenylsulphonyl)-1,3-benzodioxole-2-carboxylic acid ethyl ester.

13. Pharmaceutical preparations containing a compound according to any one of claims 1-9 or a pharmaceutically acceptable salt of a compound according to any one of these claims that is capable of salt formation.

14. Pharmaceutical preparations containing a compound according to any one of claims 10-12 or a pharmaceutically acceptable salt of a compound according to any one of these claims that is capable of salt formation.

15. Compounds according to any one of claims 1-9 and pharmaceutically acceptable salts of compounds according to any one of these claims that are capable of salt formation for use in a method for the therapeutic treatment of the human or animal body.

16. Compounds according to any one of claims 10-12 and pharmaceutically acceptable salts of compounds according to any one of these claims that are capable of salt formation for use in a method for the therapeutic treatment of the human or animal body.

17. Compounds according to any one of claims 1-9 and pharmaceutically acceptable salts of

compounds according to any one of these claims that are capable of salt formation as diuretics having supplementary uricosuric action.

18. Compounds according to any one of claims 10-12 and pharmaceutically acceptable salts of compounds according to any one of these claims that are capable of salt formation as diuretics having supplementary uricosuric action.

19. Use of compounds according to any one of claims 1-9 14 or of pharmaceutically acceptable salts of compounds according to any one of these claims that are capable of salt formation for the manufacture of medicaments by non-chemical method.

20. Use of compounds according to any one of claims 10-12 or of pharmaceutically acceptable salts of compounds according to any one of these claims that are capable of salt formation for the manufacture of medicaments by non-chemical method.

21. A process for the manufacture of compounds of the general formula I given in claim 1 in which $R_1$, $R_2$, $R_3$, $R_4$, alk, $n_1$, $n_2$ and A have the meanings given in claim 1 and salts of those compounds in which A represents $OR_5$ wherein $R_5$ is hydrogen, or that have a basic character, characterised in that

a) a compound of the general formula II

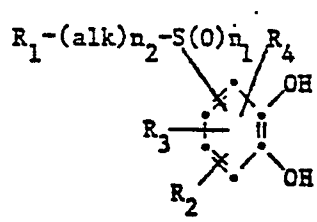

(II)

in which $R_1$, alk, $n_1$, $n_2$, $R_2$, $R_3$ and $R_4$ have the meanings given in claim 1, or a salt thereof, is reacted with a compound of the general formula III

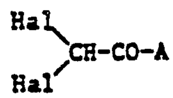

(III)

in which Hal represents halogen and A has the meaning given in claim 1, or with a salt of such a compound in which A represents $OR_5$ wherein $R_5$ represents hydrogen, or

b) in a compound of the general formula IV

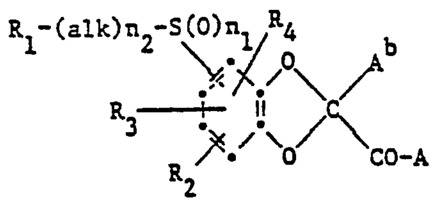

(IV)

in which $A^b$ represents carboxy, lower alkoxycarbonyl or acetyl, and $R_1$, alk, $n_1$, $n_2$, $R_2$, $R_3$, $R_4$ and A have the meanings given in claim 1, the radical $A^b$ is replaced by hydrogen, or

c) for the manufacture of a compound of the general formula I in which A has the meaning given in claim 1 with the exception of a radical $OR_5$ in which $R_5$ represents hydrogen, and $R_1$, alk, $n_2$, $R_2$, $R_3$ and $R_4$ have the meanings given in claim 1 and $n_1$ represents 1 or 2, a compound of the general formula V

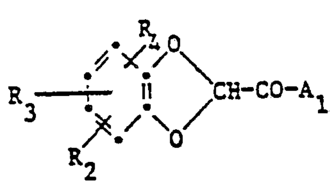

(V)

is reacted with a compound of the general formula VI

$$R_1\text{—}(alk)n_2\text{—}S(O)n_1{}^c\text{—}OH \qquad (VI),$$

or with an anhydride thereof, in which $A_1$ has the meaning given for A in claim 1 with the exception of a radical $OR_5$ in which $R_5$ represents hydrogen, $n_1{}^c$ represents 1 or 2 and $R_2$, $R_3$ and $R_4$, and $R_1$, alk and $n_2$ have the meanings given in claim 1, or

d) for the manufacture of a compound in which $n_2$ is 1 or $R_1$ is an aliphatic radical according to the definition and $R_1$ or $n_2$, as the case may be, alk, $n_2$, $R_2$, $R_3$, $R_4$ and A have the meanings given in claim 1, a

compound of the general formula VII

$$R_1—(alk)n_2^d—Z^d \qquad (VII),$$

in which $Z^d$ represents a reactively esterified hydroxy group, especially halogen having an atomic number of at least 17, $R_1$ has the meaning given in claim 1 and $n_2$ represents 1 or, if $R_2$ is an aliphatic radical, may alternatively be 0, or a compound of the general formula VIII

$$R_1—(alk)n_1—\overset{\oplus}{N}{\equiv}N \quad Z^{dd\ominus} \qquad (VIII),$$

in which $Z^{dd\ominus}$ represents a monovalent anion or the normal equivalent of a polyvalent anion and $R_1$ and $n_1$ have the meanings given in claim 1, is reacted with a salt of a compound of the general formula IX

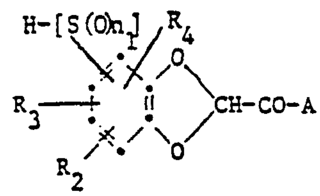

(IX)

in which $n_1$, $R_2$, $R_3$, $R_4$ and A have the meanings given in claim 1, or

e) a metal compound, optionally formed in situ, of the general formula Xa or Xb

$-(alk)n_2-M_1$ (Xa)

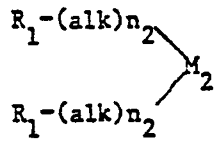

(Xb)

in which $M_1$ represents a monovalent metal ion and $M_2$ represents a divalent metal ion and R, alk and $n_2$ have the meanings given in claim 1, is reacted with a compound of the general formula XIa or XIb

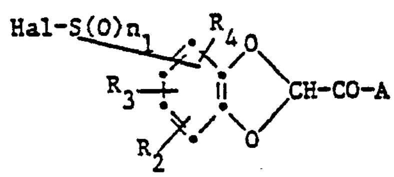

(XIa)

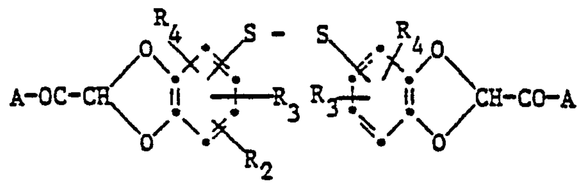

(XIb)

in which Hal represents halogen and $n_1$, $R_2$, $R_3$, $R_4$ and A have the meanings given in claim 1, or

f) for the manufacture of a compound of the general formula I in which A represents $OR_5$ wherein $R_5$ represents hydrogen, and $R_1$, alk, $n_1$, $n_2$, $R_2$, $R_3$ and $R_4$ have the meanings given in claim 1, or a salt of this compound : in a compound of the general formula XIII

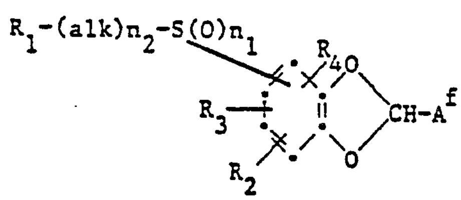

(XIII)

in which $A^f$ represents a group that can be converted into the carboxy group and $R_1$, alk, $n_1$, $n_2$, $R_2$, $R_3$ and $R_4$ have the meanings given in claim 1, the group $A^f$ is converted into the carboxy group in free or salt form, or

g) for the manufacture of a compound of the general formula I in which A has the meaning given in

claim 1 with the exception of a radical $OR_5$ in which $R_5$ represents hydrogen, and $R_1$, alk, $n_1$, $n_2$, $R_2$ and $R_3$ have the meanings given in claim 1 : in a compound of the general formula XIV

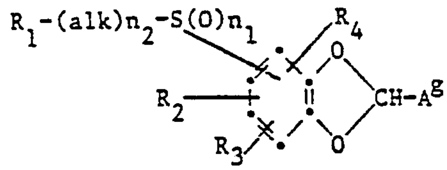

(XIV)

in which $A^g$ represents a radical that can be converted into a radical CO—$A_1$ in which $A_1$ has the meaning given for A in claim 1 with the exception of a radical $OR_5$ in which $R_5$ represents hydrogen, and $R_1$, alk, $n_1$, $n_2$, $R_2$, $R_3$ and $R_4$ have the meanings given in claim 1, the radical $A^g$ is converted into the radical —CO—$A_1$, and

h) if desired, a compound of the general formula I in which $n_1$ represents 0 or 1 is oxidised to form the corresponding compound in which $n_1$ represents 1 or 2, or

i) if desired, a compound of the general formula I in which $n_1$ represents 2 or 1 is reduced to form the corresponding compound in which $n_1$ represents 1 or 0, and/or, if desired, a resulting compound of the general formula I is converted in a different manner known per se into a different compound of the general formula I, and/or a compound of the general formula I obtained in the form of a racemate is separated into the optical antipodes, and/or a resulting compound of the general formula I in which A represents $OR_5$ wherein $R_5$ represents hydrogen is converted into a salt with a base, or such a compound is freed from a resulting salt, or a resulting compound of the general formula I of basic character is converted into an acid addition salt, or such a compound is freed from a resulting salt.

22. The compounds obtainable according to the process of claim 21.

**Claims** (for the Contracting State AT)

1. A process for the manufacture of compounds of the formula I

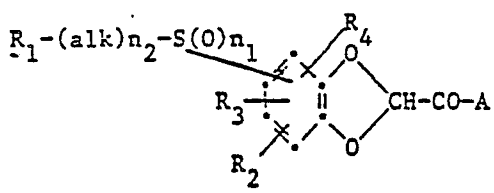

(I)

in which $R_1$ represents an alkyl, alkenyl or alkynyl radical that is unsubstituted or substituted by halogen having an atomic number of up to 35, lower alkoxy or by lower alkylthio and has, in total, that is to say including the substituents, a maximum of 12 carbon atoms, or represents a phenyl or naphthyl radical that is unsubstituted or substituted by lower alkyl, lower cycloalkyl, lower alkoxy, lower alkylthio, lower alkylsulphonyl, trifluoromethyl, hydroxy, lower alkanoyl, lower alkanoylamino, lower alkoxycarbonylamino and/or by lower alkylsulphonylamino, a monocyclic five-membered hetero ring of aromatic character having two nitrogen atoms or one nitrogen atom and/or one oxygen or sulphur atom in the ring or a six-membered hetero ring of aromatic character having one or two nitrogen atoms in the ring, or a bicyclic radical consisting of the said five- or six-membered hetero rings of aromatic character with a fused benzene ring, alk represents an alkylene, alkenylene or alkylidene radical having a maximum of 5 carbon atoms, $n_1$ represents 0, 1 or 2 and $n_2$ represents 0 or 1, $R_2$, $R_3$ and $R_4$ each represents, independently of the others, hydrogen, lower alkyl, lower alkoxy or halogen, and A represents the radical —O—$R_5$ wherein $R_5$ represents hydrogen or an alkyl radical having a maximum of 12 carbon atoms that is unsubstituted or is substituted by lower alkoxy or by halogen, 2- or 3-lower alkenyl, 2-lower alkynyl, phenyl-lower alkyl or cinnamyl, in which the phenyl radical may be substituted in the same manner as under $R_1$, or A represents the radical

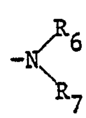

in which either $R_6$ and $R_7$ each represents, independently of the other, hydrogen or lower alkyl, or $R_6$ and $R_7$ are bonded to one another and, together with the adjacent nitrogen atom, represent optionally lower alkyl-substituted tetra- to hexa-methyleneimino, 4-morpholinyl or 1H-tetrazol-1-yl, salts of compounds of

the general formula I in which A represents $OR_5$ wherein $R_5$ represents hydrogen with bases, and acid addition salts of compounds of the general formula I in which the radical $R_1$ has a basic character, radicals designated « lower » having a maximum of 7 carbon atoms, characterised in that

a) a compound of the general formula II

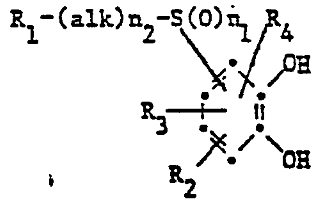

(II)

in which $R_1$, alk, $n_1$, $n_2$, $R_2$, $R_3$ and $R_4$ have the meanings given above, or a salt thereof, is reacted with a compound of the general formula III

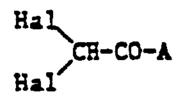

(III)

in which Hal represents halogen and A has the meaning given above, or with a salt of such a compound in which A represents $OR_5$ wherein $R_5$ represents hydrogen, or

b) in a compound of the general formula IV

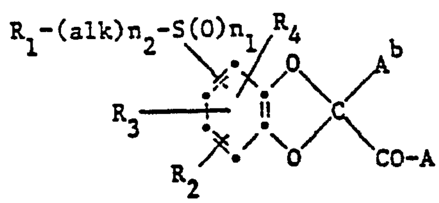

(IV)

in which $A^b$ represents carboxy, lower alkoxycarbonyl or acetyl, and $R_1$, alk, $n_1$, $n_2$, $R_2$, $R_3$, $R_4$ and A have the meanings given in claim 1, the radical $A^b$ is replaced by hydrogen, or

c) for the manufacture of a compound of the general formula I in which A has the meaning given above with the exception of a radical $OR_5$ in which $R_5$ represents hydrogen, and $R_1$, alk, $n_2$, $R_2$, $R_3$ and $R_4$ have the meanings given above and $n_1$ represents 1 or 2, a compound of the general formula V

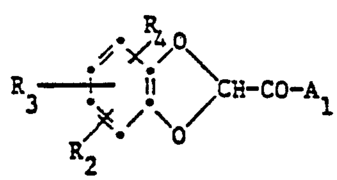

(V)

is reacted with a compound of the general formula VI

$$R_1—(alk)n_2—S(O)n_1^c—OH \qquad (VI),$$

or with an anhydride thereof, in which $A_1$ has the meaning given above for A with the exception of a radical $OR_5$ in which $R_5$ represents hydrogen, $n_1^c$ represents 1 or 2 and $R_2$, $R_3$ and $R_4$, and $R_1$, alk and $n_2$ have the meanings given above, or

d) for the manufacture of a compound in which $n_2$ is 1 or $R_1$ is an aliphatic radical according to the definition and $R_1$ or $n_2$, as the case may be, alk, $n_2$, $R_2$, $R_3$, $R_4$ and A have the meanings given above, a compound of the general formula VII

$$R_1—(alk)n_2^d—Z^d \qquad (VII),$$

in which $Z^d$ represents a reactively esterified hydroxy group, especially halogen having an atomic number of at least 17, $R_1$ has the meaning given above and $n_2$ represents 1 or, if $R_2$ is an aliphatic radical, may alternatively be 0, or a compound of the general formula VIII

$$R_1—(alk)n_1—\overset{\oplus}{N}\equiv N \quad Z^{dd\ominus} \qquad (VIII),$$

in which $Z^{dd\ominus}$ represents a monovalent anion or the normal equivalent of a polyvalent anion and $R_1$ and $n_1$ have the meanings given above, is reacted with a salt of a compound of the general formula IX

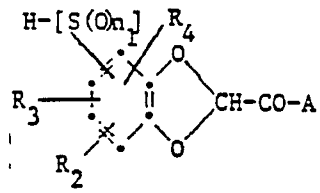

(IX)

in which $n_1$, $R_2$, $R_3$, $R_4$ and A have the meanings given in claim 1, or

e) a metal compound, optionally formed in situ, of the general formula Xa or Xb

$R_1-(alk)n_2-M_1$ (Xa)

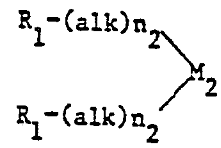

(Xb)

in which $M_1$ represents a monovalent metal ion and $M_2$ represents a divalent metal ion and R, alk and $n_2$ have the meanings given above, is reacted with a compound of the general XIa or XIb

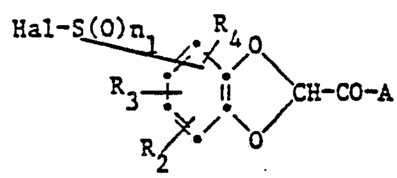

(XIa)

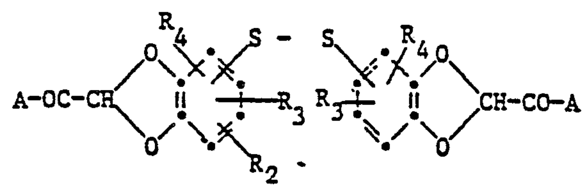

(XIb)

in which Hal represents halogen and $n_1$, $R_2$, $R_3$, $R_4$ and A have the meanings given above, or

f) for the manufacture of a compound of the general formula I in which A represents $OR_5$ wherein $R_5$ represents hydrogen, and $R_1$, alk, $n_1$, $n_2$, $R_2$, $R_3$ and $R_4$ have the meanings given above, or a salt of this compound : in a compound of the general formula XIII

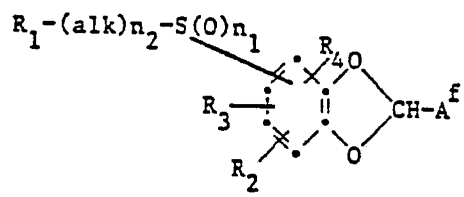

(XIII)

in which $A^f$ represents a group that can be converted into the carboxy group and $R_1$, alk, $n_1$, $n_2$, $R_2$, $R_3$ and $R_4$ have the meanings given above, the group $A^f$ is converted into the carboxy group in free or salt form, or

g) for the manufacture of a compound of the general formula I in which A has the meaning given above with the exception of a radical $OR_5$ in which $R_5$ represents hydrogen, and $R_1$, alk, $n_1$, $n_2$, $R_2$ and $R_3$ have the meanings given above : in a compound of the general formula XIV

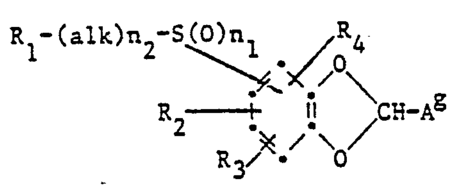

(XIV),

in which $A^g$ represents a radical that can be converted into a radical $CO—A_1$ in which $A_1$ has the meaning given above for A with the exception of a radical $OR_5$ in which $R_5$ represents hydrogen, and $R_1$, alk, $n_1$, $n_2$, $R_2$, $R_3$ and $R_4$ have the meanings given in claim 1, the radical $A^g$ is converted into the radical $—CO—A_1$, and

h) if desired, a compound of the general formula I in which $n_1$ represents 0 or 1 is oxidised to form the corresponding compound in which $n_1$ represents 1 or 2, or

i) if desired, a compound of the general formula I in which $n_1$ represents 2 or 1 is reduced to form the corresponding compound in which $n_1$ represents 1 or 0, and/or, if desired, a resulting compound of the general formula I is converted in a different manner known per se into a different compound of the general formula I, and/or a compound of the general formula I obtained in the form of a racemate is separated into the optical antipodes, and/or a resulting compound of the general formula I in which A represents $OR_5$ wherein $R_5$ represents hydrogen is converted into a salt with a base, or such a compound is freed from a resulting salt, or a resulting compound of the general formula I of basic character is converted into an acid addition salt, or such a compound is freed from a resulting salt.

2. A process according to patent claim 1, characterised in that compounds of the formula I are produced in which $R_1$ represents phenyl, thienyl, furyl or arbenzothiazolyl, each of which is unsubstituted or substituted a maximum of three times by lower alkyl, lower alkoxy, halogen having an atomic number of up to 35, lower alkanoyl and/or by lower alkanoylamino, alk represents alkylene, alkylidene or alkenylene having a maximum of 3 carbon atoms, $R_2$ represents lower alkyl or halogen having an atomic number of up to 35, $R_3$ represents hydrogen, lower alkyl or halogen having an atomic number of up to 35, $R_4$ represents hydrogen and $n_1$, $n_2$ and A have the meanings given in claim 1, and the salts of those compounds in which $R_5$ represents hydrogen with bases, radicals designated « lower » having a maximum of 7 carbon atoms.

3. A process according to patent claim 1, characterised in that compounds of the formula I are produced in which $R_1$ represents phenyl or thienyl, each of which is unsubstituted or substituted a maximum of three times by lower alkyl, lower alkoxy, halogen having an atomic number of up to 35, lower alkanoyl and/or by lower alkanoylamino, alk represents methylene, $n_1$ represents 2, $n_2$ represents 0 or 1, $R_2$ represents lower alkyl or halogen, $R_3$ represents hydrogen or lower alkyl, $R_4$ represents hydrogen, and A represents $OR_5$ wherein $R_5$ represents hydrogen or lower alkyl, and the pharmaceutically acceptable salts of those compounds in which $R_5$ represents hydrogen with bases, radicals designated « lower » having a maximum of 7 carbon atoms.

4. A process according to patent claim 1, characterised in that compounds of the formula I are produced in which $R_1$ represents phenyl that is unsubstituted or substituted a maximum of three times by lower alkyl, lower alkoxy and/or by halogen having an atomic number of up to 35, alk represents methylene, $n_1$ represents 2, $n_2$ represents 0 or 1, $R_2$ represents lower alkyl or halogen, $R_3$ represents hydrogen or lower alkyl, $R_4$ represents hydrogen, and A represents $OR_5$ wherein $R_5$ represents hydrogen or lower alkyl, and the phenylsulphonyl radical is bonded in the 5- or 6-position, and the pharmaceutically acceptable salts of those compounds in which $R_5$ represents hydrogen with bases, radicals designated « lower » having a maximum of 7 carbon atoms.

5. A process according to patent claim 1, characterised in that compounds of the formula I are produced in which $R_1$ represents phenyl that is unsubstituted or substituted a maximum of three times by methyl, methoxy and/or by chlorine, alk represents methylene, $n_1$ represents 2, $n_1$ represents 0 or 1, $R_2$ represents methyl or chlorine, $R_3$ and $R_4$ represent hydrogen, and A represents $OR_5$ wherein $R_5$ represents hydrogen or lower alkyl, and the phenylsulphonyl radical is bonded in the 5- or 6-position, and the pharmaceutically acceptable salts of those compounds in which $R_5$ represents hydrogen with bases, radicals designated « lower » having a maximum of 7 carbon atoms.

6. A process according to patent claim 1, characterised in that 5-methyl-6-phenylsulphonyl-1,3-benzodioxole-2-carboxylic acid and the pharmaceutically acceptable salts thereof with bases are produced.

7. A process according to patent claim 1, characterised in that 5-chloro-6-(2-chlorophenylsulphonyl)-1,3-benzodioxole-2-carboxylic acid and the pharmaceutically acceptable salts thereof with bases are produced.

8. A process according to patent claim 1, characterised in that 5-(2-chlorophenylsulphonyl)-6-methyl-1,3-benzodioxole-2-carboxylic acid and the pharmaceutically acceptable salts thereof with bases are produced.

9. A process according to patent claim 1, characterised in that 5-chloro-6-(4-chlorophenylsulphonyl)-1,3-benzodioxole-2-carboxylic acid and the pharmaceutically acceptable salts thereof with bases are produced.

10. A process according to patent claim 1, characterised in that 5-(3-chlorophenylsulphonyl)-6-methyl-1,3-benzodioxole-2-carboxylic acid and the pharmaceutically acceptable salts thereof with bases are produced.

11. A process according to patent claim 1, characterised in that 5-chloro-6-(3-chlorophenylsulphonyl)-1,3-benzodioxole-2-carboxylic acid and the pharmaceutically acceptable salts thereof with bases are produced.

12. A process according to patent claim 1, characterised in that 5-chloro-6-(4-chlorophenylsul-

phonyl)-1,3-benzodioxole-2-carboxylic acid is produced.

13. A process for the manufacture of a pharmaceutical preparation characterised by processing an active ingredient of the invention according to claims 1 to 8 with a pharmaceutical carrier.

14. A process for the manufacture of a pharmaceutical preparation characterised by processing an active ingredient of the invention according to claims 9 to 11 with a pharmaceutical carrier.

**Revendications** (pour les Etats contractants : BE, CH, LI, DE, FR, GB, IT, LU, NL, SE)

1. Composés de formule générale I

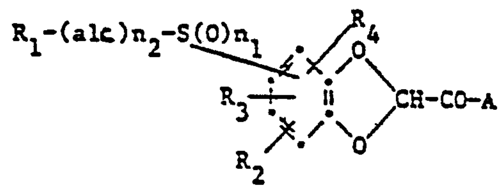

(I)

où R1 représente un radical alcoyle, alcényle ou alcynyle non substitué ou substitué par un halogène dont le numéro atomique va jusqu'à 35, un alcoxy inférieur ou un alcoyle inférieur-thio, avec au total, c'est-à-dire y compris les substituants, au plus 12 atomes de carbone, ou un radical phényle ou naphtyle, non substitué ou substitué par un alcoyle inférieur, cycloalcoyle inférieur, alcoxy inférieur, alcoyle inférieur-thio, alcoyle inférieur-sulfonyle, trifluorométhyle, hydroxy, alcanoyle inférieur, alcanoyle inférieur-amino, alcoxy inférieur-carbonylamino et/ou alcoyle inférieur-sulfonylamino, un hétérocycle monocyclique à 5 chaînons de caractère aromatique avec 2 atomes d'azote ou 1 atome d'azote et/ou un atome d'oxygène ou de soufre dans son noyau ou un hétérocycle à 6 chaînons de caractère aromatique avec 1 ou 2 atomes d'azote dans son noyau ou un radical bicyclique, constitué des hétérocycles à 5 ou 6 chaînons de caractère aromatique mentionnés avec un noyau benzène condensé, alc est un radical alcoylène, alcénylène ou alcoylidène avec au plus 5 atomes de carbone, n1 vaut zéro, un ou deux et n2 vaut zéro ou un, R2, R3 et R4 représentent indépendamment l'un de l'autre un hydrogène, alcoyle inférieur, alcoxy inférieur ou halogène et A le radical —O—R5, où R5 représente un hydrogène ou un radical alcoyle non substitué ou substitué par un alcoxy inférieur ou un halogène et ayant au plus 12 atomes de carbone, un 2- ou 3-alcényle inférieur, un 2-alcynyle inférieur, phénylalcoyle inférieur ou cinnamyle, dans lequel le radical phényle peut être substitué de la même manière que sous R1, ou le radical

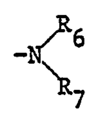

où R6 et R7 représentent indépendamment l'un de l'autre un hydrogène ou un alcoyle inférieur, ou liés entre eux et avec l'atome d'azote joint un tétra- à hexaméthylèneimino, 4-morpholinyle ou 1H-tétrazol-1-yle éventuellement substitué par un alcoyle inférieur, les sels de composés de formule générale I dans lesquels A représente OR5 avec R5 = hydrogène, avec des bases, ainsi que les sels d'addition d'acides de composés de formule générale I dont le radical R1 présente un caractère basique, où les radicaux décrits comme « inférieurs » présentent au plus 7 atomes de carbone.

2. Composés de formule générale I donnée dans la revendication 1, où R1 représente un phényle, thiényle, furyle ou ar-benzothiazolyle au plus trisubstitué par un alcoyle inférieur, alcoxy inférieur, halogène de numéro atomique allant jusqu'à 35, alcanoyle inférieur et/ou alcanoyle inférieur-amino, ou non substitué, alc représente un alcoylène, alcoylidène ou alcénylène avec au plus 3 atomes de carbone, R2 représente un alcoyle inférieur ou un halogène de numéro atomique allant jusqu'à 35, R3 représente un hydrogène, alcoyle inférieur ou halogène de numéro atomique allant jusqu'à 35 et R4 représente un hydrogène et n1, n2 et A ont la signification donnée dans la revendication 1, ainsi que les sels de tels composés dans lesquels R5 est un hydrogène, avec les bases, où les radicaux décrits comme « inférieurs » présentent au plus 7 atomes de carbone.

3. Composés de formule générale I donnée dans la revendication 1, où R1 représente un phényle ou un thiényle au plus trisubstitué par un alcoyle inférieur, alcoxy inférieur, halogène de numéro atomique allant jusqu'à 35, alcanoyle inférieur et/ou alcanoyle inférieur-amino, ou non substitué, alc représente un méthylène, n1 vaut deux, n2 vaut zéro ou un, R2 représente un alcoyle inférieur ou un halogène, R3 un hydrogène ou un alcoyle inférieur, R4 un hydrogène, et A représente OR5 avec R5 = hydrogène ou alcoyle inférieur, ainsi que les sels pharmaceutiquement acceptables de tels composés où R5 est un hydrogène, où les radicaux désignés comme « inférieurs » présentent au plus 7 atomes de carbone.

4. Composés de formule générale I donnée dans la revendication 1, où R1 représente un phényle au plus trisubstitué par un alcoyle inférieur, alcoxy inférieur et/ou halogène de numéro atomique allant

jusqu'à 35, ou non substitué, alc représente un méthylène, n1 vaut deux, n2 vaut zéro ou un, R2 représente un alcoyle inférieur ou un halogène, R3 représente un hydrogène ou un alcoyle inférieur, R4 représente un hydrogène et A représente OR5 avec R5 = hydrogène ou alcoyle inférieur, et le radical phénylsulfonyle est lié en position 5 ou 6, ainsi que les sels pharmaceutiquement acceptables de tels composés, où R5 est un hydrogène, avec des bases, où les radicaux décrits comme « inférieurs » présentent au plus 7 atomes de carbone.

5. Composés de formule générale I donnée dans la revendication 1, où R1 représente un phényle au plus trisubstitué par un méthyle, méthoxy et/ou chlore, ou non substitué, alc représente un méthylène, n1 vaut deux, n2 vaut zéro ou un, R2 représente un méthyle ou un chlore, R3 et R4 représentent un hydrogène et A représente OR5 avec R5 hydrogène ou alcoyle inférieur, et le radical phényl-sulfonyle est lié en position 5 ou 6, ainsi que les sels pharmaceutiques de tels composés, où R5 est un hydrogène, avec des bases, où les radicaux décrits comme « inférieurs » présentent au plus 7 atomes de carbone.

6. L'acide 5-méthyl-6-phénylsulfonyl-1,3-benzodioxol-2-carboxylique et ses sels pharmaceutiquement acceptables avec des bases.

7. L'acide 5-chloro-6-(2-chlorophénylsulfonyl)-1,3-benzodioxol-2-carboxylique et ses sels pharmaceutiquement acceptables avec des bases.

8. L'acide 5-(2-chlorophénylsulfonyl)-6-méthyl-1,3-benzodioxol-2-carboxylique et ses sels pharmaceutiquement acceptables avec des bases.

9. L'acide 5-chloro-6-(4-chlorophénylsulfonyl)-1,3-benzodioxol-2-carboxylique et ses sels pharmaceutiquement acceptables avec des bases.

10. L'acide 5-(3-chlorophénylsulfonyl)-6-méthyl-1,3-benzodioxol-2-carboxylique et ses sels pharmaceutiquement acceptables avec des bases.

11. L'acide 5-chloro-6-(3-chlorophénylsulfonyl)-1,3-benzodioxol-2-carboxylique et ses sels pharmaceutiquement acceptables avec des bases.

12. L'ester éthylique de l'acide 5-chloro-6-(4-chlorophénylsulfonyl)-1,3-benzodioxol-2-carboxylique.

13. Préparations pharmaceutiques contenant un composé selon l'une des revendications 1-9 ou un sel pharmaceutiquement acceptable d'un composé apte à la salification selon l'une de ces revendications.

14. Préparations pharmaceutiques contenant un composé selon l'une des revendications 10-12 ou un sel pharmaceutiquement acceptable d'un composé apte à la salification selon l'une de ces revendications.

15. Composés selon l'une des revendications 1-9 et sels pharmaceutiquement acceptables de composés aptes à la salification selon l'une de ces revendications aux fins d'application dans un procédé de traitement thérapeutique du corps animal ou humain.

16. Composés selon l'une des revendications 10-12 et sels pharmaceutiquement acceptables de composés aptes à la salification selon l'une de ces revendications aux fins d'application dans un procédé de traitement thérapeutique du corps animal ou humain.

17. Composés selon l'une des revendications 1-9 et sels pharmaceutiquement acceptables de composés aptes à la salification selon l'une de ces revendications comme diurétiques à effet additionnel uricosurique.

18. Composés selon l'une des revendications 10-12 et sels pharmaceutiquement acceptables de composés aptes à la salification selon l'une de ces revendications comme diurétiques à effet additionnel uricosurique.

19. Application de composés selon l'une des revendications 1 à 9 ou de sels pharmaceutiquement acceptables de composés aptes à la salification selon l'une de ces revendications à la préparation de médicaments par un moyen non chimique.

20. Application de composés selon l'une des revendications 10-12 ou de sels pharmaceutiquement acceptables de composés aptes à la salification selon l'une de ces revendications à la préparation de médicaments par un moyen non chimique.

21. Procédé de préparation de composés de formule générale I donnée dans la revendication 1, où R1, R2, R3, R4, alc, n1, n2 et A ont la signification qui y est donnée, et de sels de tels composés où A représente OR5 avec R5 = hydrogène, ou qui possèdent un caractère basique, caractérisé en ce que

a) on fait réagir un composé de formule générale II

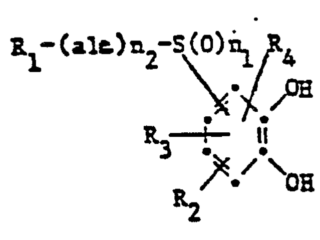

(II)

où R1, alc, n1, n2, R2, R3 et R4 ont la signification donnée dans la revendication 1, ou un de ses sels, avec un composé de formule générale III

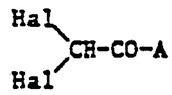

(III)

où Hal représente un halogène et A a la signification donnée dans la revendication 1, ou un sel d'un tel composé, où A représente OR5 avec R5 oxygène, ou

b) dans un composé de formule générale IV

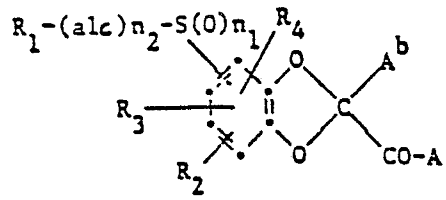

(IV)

où $A^b$ représente un carboxy, alcoxy inférieur-carbonyle ou acétyle, et R1, alc, n1, n2, R2, R3, R4 et A ont la signification donnée dans la revendication 1, on remplace le radical $A^b$ par un hydrogène, ou

c) pour préparer un composé de formule générale I où A a la signification donnée dans la revendication 1 à l'exception d'un radical OR5 où R5 représente un hydrogène, et R1, alc, n2, R2, R3 et R4 ont la signification donnée dans la revendication 1 et n1 vaut un ou deux, on fait réagir un composé de formule générale V

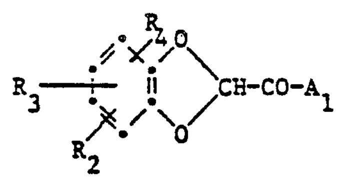

(V)

avec un composé de formule générale VI

$$R_1\text{—(alc)}n_2\text{—S(O)}n_1{}^c\text{—OH} \qquad \text{(VI)},$$

ou un de ses anhydrides où A1 a la signification donnée dans la revendication 1 pour A à l'exception d'un radical OR5 où R5 représente un hydrogène, $n_1{}^c$ vaut un ou deux et R2, R3 et R4 ou selon les cas R1, alc et n2 ont la signification donnée dans la revendication 1, ou

d) pour préparer un composé où n2 vaut un ou R1 est un radical aliphatique selon la définition et R1 ou selon les cas n2, alc, n2, R2, R3, R4 et A ont la signification donnée dans la revendication 1, on fait réagir un composé de formule générale VII

$$R_1\text{—(alc)}n_2{}^d\text{—}Z^d \qquad \text{(VII)}$$

où $Z^d$ représente un groupe hydroxy estérifié réactif, en particulier un halogène ayant un numéro atomique d'au moins 17, R1 a la signification donnée dans la revendication 1 et n2 vaut un, ou si R2 est un radical aliphatique, peut également valoir zéro, ou un composé de formule générale VIII

$$R_1\text{—(alc)}n_1\text{—}\overset{\oplus}{N}\equiv N \quad Z^{dd\ominus} \qquad \text{(VIII)},$$

où $Z^{dd\ominus}$ représente un anion monovalent ou l'équivalent normal d'un anion polyvalent et R1 et n1 ont la signification donnée dans la revendication 1, avec un sel d'un composé de formule générale IX

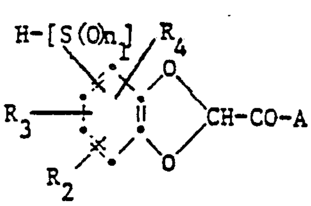

(IX)

où n1, R2, R3, R4 et A ont la signification donnée dans la revendication 1, ou

e) on fait réagir un composé métallique de formule générale Xa ou Xb éventuellement formé in situ

$R_1-(alc)n_2-M_1$ (Xa)

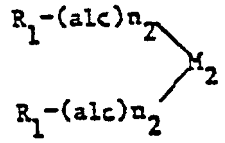 (Xb)

où M1 représente un ion métallique monovalent ou selon les cas M2 un ion métallique bivalent et R, alc et n2 ont la signification donnée dans la revendication 1, avec un composé de formule générale XIa ou XIb,

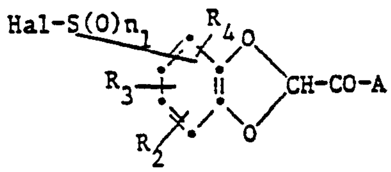 (XIa)

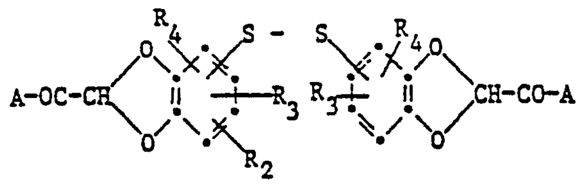 (XIb)

où Hal représente un halogène et n1, R2, R3, R4 et A ont la signification donnée pour la revendication 1, ou

f) pour préparer un composé de formule générale I où A représente OR5 avec R5 = hydrogène et R1, alc, n1, n2, R2, R3 et R4 ont la signification donnée dans la revendication 1, ou un sel de ce composé, dans un composé de formule générale XIII

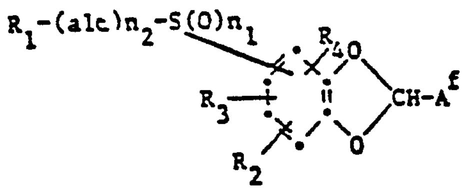 (XIII)

où A$^f$ représente un groupe transformable en le groupe carboxy et R1, alc, n1, n2, R2, R3 et R4 ont la signification donnée dans la revendication 1, on transforme le groupe A$^f$ en le groupe carboxy sous forme libre ou sous forme de sel, ou

g) pour préparer un composé de formule générale I où A a la signification donnée dans la revendication 1 à l'exception d'un radical OR5 dans lequel R5 représente un hydrogène, et R1, alc, n1, n2, R2 et R3 ont la signification donnée dans la revendication 1, dans un composé de formule générale XIV

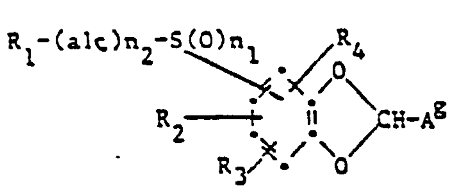 (XIV)

où A$^g$ représente un radical transformable en un radical CO-A1, où A1 a la signification donnée pour A dans la revendication 1 à l'exception d'un radical OR5 où R5 représente un hydrogène, et R1, alc, n1, n2, R2, R3 et R4 ont la signification donnée dans la revendication 1, le radical A$^g$ en le radical —CO—A1, et

h) si on le désire on oxyde un composé de formule générale I où N1 vaut zéro ou un, en le composé correspondant où n1 vaut un ou deux, ou

i) si on le désire on réduit un composé de formule générale I où n1 vaut un ou deux, en le composé correspondant où n1 vaut un ou zéro, et/ou si on le désire on transforme un composé de formule générale I obtenu, d'une autre façon connue, en un autre composé de formule générale I, et/ou on dédouble un composé de formule générale I obtenu sous forme de racémate, en les antipodes optiques, et/ou on transforme un composé de formule générale I où A représente OR5 avec R5 = hydrogène, en un sel avec une base ou on libère un tel composé à partir d'un sel obtenu, ou on transforme un composé obtenu de

formule générale II de caractère basique en un sel d'addition d'acide ou on libère un tel composé à partir d'un sel obtenu.

22. Les composés que l'on peut obtenir selon le procédé de la revendication 21.

**Revendications** (pour l'Etat contractant AT)

1. Procédé de préparation de composés de formule I

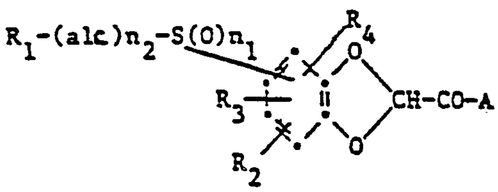

(I)

où R1 représente un radical alcoyle, alcényle ou alcynyle non substitué ou substitué par un halogène dont le numéro atomique va jusqu'à 34, un alcoxy inférieur ou un alcoyle inférieur-thio, avec au total, c'est-à-dire y compris les substituants, au plus 12 atomes de carbone, ou un radical phényle ou naphtyle, non substitué ou substitué par un alcoyle inférieur, cycloalcoyle inférieur, alcoxy inférieur, alcoyle inférieur-thio, alcoyle inférieur-thio, alcoyle inférieur-sulfonyle, trifluorométhyle, hydroxy, alcanoyle inférieur, alcanoyle inférieur-amino, alcoxy inférieur-carbonylamino et/ou alcoyle inférieur-sulfonylamino, un hétérocycle monocyclique à 5 chaînons de caractère aromatique avec 2 atomes d'azote ou 1 atome d'azote et/ou un atome d'oxygène ou de soufre dans son noyau ou un hétérocycle à 6 chaînons de caractère aromatique avec 1 ou 2 atomes d'azote dans son noyau ou un radical bicyclique, constitué des hétérocycles à 5 ou 6 chaînons de caractère aromatique mentionnés avec un noyau benzène condensé, alc est un radical alcoylène, alcénylène ou alcoylidène avec au plus 5 atomes de carbone, n1 vaut zéro, un ou deux et n2 vaut zéro ou un, R2, R3 et R4 représentent indépendamment l'un de l'autre un hydrogène, alcoyle inférieur, alcoxy inférieur ou halogène et A le radical —O—R5, où R5 représente un hydrogène ou un radical alcoyle non substitué ou substitué par un alcoxy inférieur ou un halogène et ayant au plus 12 atomes de carbone, un 2- ou 3-alcényle inférieur, un 2-alcynyle inférieur, phénylalcoyle inférieur ou cinnamyle, dans lequel le radical phényle peut être substitué de la même manière que sous R1, ou le radical

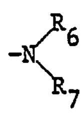

où R6 et R7 représentent indépendamment l'un de l'autre un hydrogène ou un alcoyle inférieur, ou liés entre eux et avec l'atome d'azote joint un tétra- à hexaméthylèneimino, 4-morpholinyle ou 1H-tétrazol-1-yle éventuellement substitué par un alcoyle inférieur, les sels de composés de formule générale I dans lesquels A représente OR5 avec R5 = hydrogène, avec des bases, ainsi que les sels d'addition d'acides de composés de formule générale I dont le radical R1 présente un caractère basique, où les radicaux décrits comme « inférieurs » présentent au plus 7 atomes de carbone, caractérisé en ce que

a) on fait réagir un composé de formule générale II

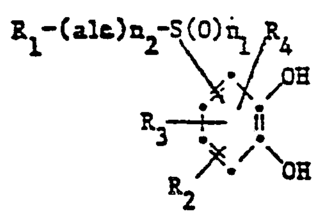

(II)

où R1, alc, n1, n2, R2, R3 et R4 ont la signification donnée dans la revendication 1, ou un de ses sels, avec un composé de formule générale III

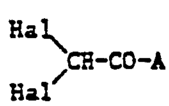

(III)

où Hal représente un halogène et A a la signification donnée dans la revendication 1, ou un sel d'un tel composé, où A représente OR5 avec R5 oxygène, ou

b) dans un composé de formule générale IV

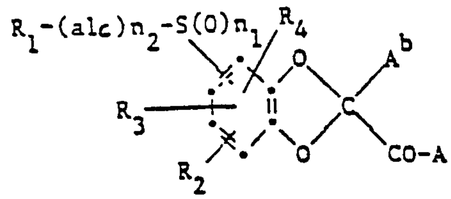

(IV)

où $A^b$ représente un carboxy, alcoxy inférieur-carbonyle ou acétyle, et R1, alc, n1, n2, R2, R3, R4 et A ont la signification donnée dans la revendication 1, on remplace le radical $A^b$ par un hydrogène, ou

c) pour préparer un composé de formule générale I où A a la signification donnée dans la revendication 1 à l'exception d'un radical OR5 où R5 représente un hydrogène, et R1, alc, n2, R2, R3 et R4 ont la signification donnée dans la revendication 1 et n1 vaut un ou deux, on fait réagir un composé de formule générale V

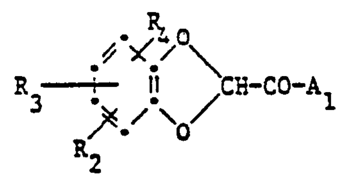

(V)

avec un composé de formule générale VI

$$R_1—(alc)n_2—S(O)n_1^c—OH \qquad (VI),$$

ou un de ses anhydrides où A1 a la signification donnée dans la revendication 1 pour A à l'exception d'un radical OR5 où R5 représente un hydrogène, $n_1^c$ vaut un ou deux et R2, R3 et R4 ou selon les cas R1, alc et n2 ont la signification donnée dans la revendication 1, ou

d) pour préparer un composé où n2 vaut un ou R1 est un radical aliphatique selon la définition et R1 ou selon les cas n2, alc, n2, R2, R3, R4 et A ont la signification donnée dans la revendication 1, on fait réagir un composé de formule générale VII

$$R_1—(alc)n_2^d—Z^d \qquad (VII)$$

où $Z^d$ représente un groupe hydroxy estérifié réactif, en particulier un halogène ayant un numéro atomique d'au moins 17, R1 a la signification donnée dans la revendication 1 et n2 vaut un, ou si R2 est un radical aliphatique, peut également valoir zéro, ou un composé de formule générale VIII

$$R_1—(alc)n_1—\overset{\oplus}{N}\equiv N \quad Z^{dd\ominus} \qquad (VIII),$$

où $Z^{dd\ominus}$ représente un anion monovalent ou l'équivalent normal d'un anion polyvalent et R1 et n1 ont la signification donnée dans la revendication 1, avec un sel d'un composé de formule générale IX

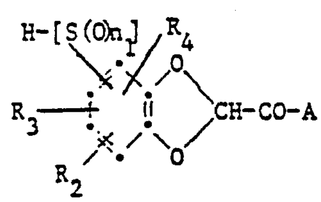

(IX)

où n1, R2, R3, R4 et A ont la signification donnée dans la revendication 1, ou

e) on fait réagir un composé métallique de formule générale Xa ou Xb éventuellement formé in situ

$R_1-(alc)n_2-M_1$ (Xa)

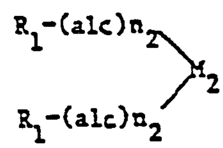

(Xb)

où M1 représente un ion métallique monovalent ou selon les cas M2 un ion métallique bivalent et R, alc et

n2 ont la signification donnée dans la revendication 1, avec un composé de formule générale XIa ou XIb,

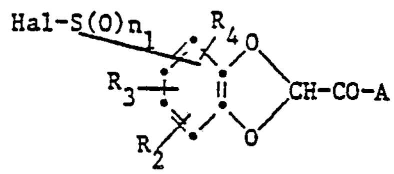

(XIa)

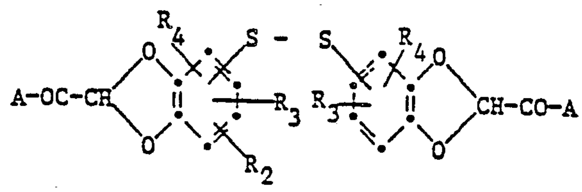

(XIb)

où Hal représente un halogène et n1, R2, R3, R4 et A ont la signification donnée pour la revendication 1, ou

f) pour préparer un composé de formule générale I où A représente OR5 avec R5 = hydrogène et R1, alc, n1, n2, R2, R3 et R4 ont la signification donnée dans la revendication 1, ou un sel de ce composé, dans un composé de formule générale XIII

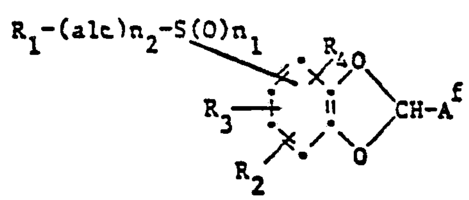

(XIII)

où A$^f$ représente un groupe transformable en le groupe carboxy et R1, alc, n1, n2, R2, R3 et R4 ont la signification donnée dans la revendication 1, on transforme le groupe A$^f$ en le groupe carboxy sous forme libre ou sous forme de sel, ou

g) pour préparer un composé de formule générale I où A a la signification donnée dans la revendication 1 à l'exception d'un radical OR5 dans lequel R5 représente un hydrogène, et R1, alc, n1, n2, R2 et R3 ont la signification donnée dans la revendication 1, dans un composé de formule générale XIV

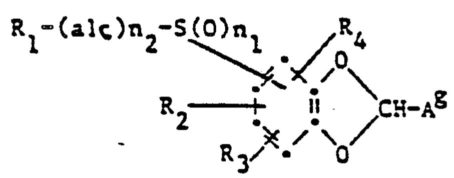

(XIV)

où A$^g$ représente un radical transformable en un radical CO—A1, où A1 a la signification donnée pour A dans la revendication 1 à l'exception d'un radical OR5 où R5 représente un hydrogène, et R1, alc, n1, n2, R2, R3 et R4 ont la signification donnée dans la revendication 1, le radical A$^g$ en le radical —CO—A1, et

h) si on le désire on oxyde un composé de formule générale I où N1 vaut zéro ou un, en le composé correspondant où n1 vaut un ou deux, ou

i) si on le désire on réduit un composé de formule générâle I où n1 vaut un ou deux, en le composé correspondant où n1 vaut un ou zéro, et/ou si on le désire on transforme un composé de formule générale I obtenu, d'une autre façon connue, en un autre composé de formule générale I, et/ou on dédouble un composé de formule générale I obtenu sous forme de racémate, en les antipodes optiques, et/ou on transforme un composé obtenu de formule générale I où A représente OR5 avec R5 = hydrogène, en un sel avec une base ou on libère un tel composé à partir d'un sel obtenu, ou on transforme un composé obtenu de formule générale II de caractère basique en un sel d'addition d'acide ou on libère un tel composé à partir d'un sel obtenu.

2. Procédé selon la revendication 1, caractérisé en ce qu'on prépare des composés de formule générale I donnée dans la revendication 1, où R1 représente un phényle, thiényle, furyle ou ar-benzothiazolyle au plus trisubstitué par un alcoyle inférieur, alcoxy inférieur, halogène de numéro atomique allant jusqu'à 35, alcanoyle inférieur et/ou alcanoyle inférieur-amino, ou non substitué, alc représente un alcoylène, alcoylidène ou alcénylène avec au plus 3 atomes de carbone, R2 représente un alcoyle inférieur ou un halogène de numéro atomique allant jusqu'à 35, R3 représente un hydrogène, alcoyle inférieur ou halogène de numéro atomique allant jusqu'à 35 et R4 représente un hydrogène et n1,

n2 et A ont la signification donnée dans la revendication 1, ainsi que les sels de tels composés dans lesquels R5 est un hydrogène, avec les bases, où les radicaux décrits comme « inférieurs » présentent au plus 7 atomes de carbone.

3. Procédé selon la revendication 1, caractérisé en ce qu'on prépare des composés de formule générale I donnée dans la revendication 1 où R1 représente un phényle ou un thiényle au plus trisubstitué par un alcoyle inférieur, alcoxy inférieur, halogène de numéro atomique allant jusqu'à 35, alcanoyle inférieur et/ou alcanoyle inférieur-amino, ou non substitué, alc représente un méthylène, n1 vaut deux, n2 vaut zéro ou un, R2 représente un alcoyle inférieur ou un halogène, R3 un hydrogène ou un alcoyle inférieur, R4 un hydrogène, et A représente OR5 avec R5 = hydrogène ou alcoyle inférieur, ainsi que les sels pharmaceutiquement acceptables de tels composés où R5 est un hydrogène, où les radicaux désignés comme « inférieurs » présentent au plus 7 atomes de carbone.

4. Procédé selon la revendication 1, caractérisé en ce qu'on prépare des composés de formule générale I donnée dans la revendication 1, où R1 représente un phényle au plus trisubstitué par un alcoyle inférieur, alcoxy inférieur et/ou halogène de numéro atomique allant jusqu'à 35, ou non substitué, alc représente un méthylène, n1 vaut deux, n2 vaut zéro ou un, R2 représente un alcoyle inférieur ou un halogène, R3 représente un hydrogène ou un alcoyle inférieur, R4 représente un hydrogène et A représente OR5 avec R5 = hydrogène ou alcoyle inférieur, et le radical phénylsulfonyle est lié en position 5 ou 6, ainsi que les sels pharmaceutiquement acceptables de tels composés, où R5 est un hydrogène, avec des bases, où les radicaux décrits comme « inférieurs » présentent au plus 7 atomes de carbone.

5. Procédé selon la revendication 1, caractérisé en ce qu'on prépare des composés de formule générale I donnée dans la revendication 1, où R1 représente un phényle au plus trisubstitué par un méthyle, méthoxy et/ou chlore, ou non substitué, alc représente un méthylène, n1 vaut deux, n2 vaut zéro ou un, R2 représente un méthyle ou un chlore, R3 et R4 représentent un hydrogène et A représente OR5 avec R5 hydrogène ou alcoyle inférieur, et le radical phénylsulfonyle est lié en position 5 ou 6, ainsi que les sels pharmaceutiquement acceptables de ces composés où R5 est un hydrogène, avec des bases, les radicaux désignés comme « inférieurs » présentant au plus 7 atomes de carbone.

6. Procédé selon la revendication 1, caractérisé en ce qu'on prépare l'acide 5-méthyl-6-phénylsulfonyl-1,3-benzodioxol-2-carboxylique et ses sels pharmaceutiquement acceptables avec des bases.

7. Procédé selon la revendication 1, caractérisé en ce qu'on prépare l'acide 5-chloro-6-(2-chlorophénylsulfonyl)-1,3-benzodioxol-2-carboxylique et ses sels pharmaceutiquement acceptables avec des bases.

8. Procédé selon la revendication 1, caractérisé en ce qu'on prépare l'acide 5-(2-chlorophénylsulfonyl)-6-méthyl-1,3-benzodioxol-2-carboxylique et ses sels pharmaceutiquement acceptables avec des bases.

9. Procédé selon la revendication 1, caractérisé en ce qu'on prépare l'acide 5-chloro-6-(4-chlorophénylsulfonyl)-1,3-benzodioxol-2-carboxylique et ses sels pharmaceutiquement acceptables avec des bases.

10. Procédé selon la revendication 1, caractérisé en ce qu'on prépare l'acide 5-(3-chlorophénylsulfonyl)-6-méthyl-1,3-benzodioxol-2-carboxylique et ses sels pharmaceutiquement acceptables avec des bases.

11. Procédé selon la revendication 1, caractérisé en ce qu'on prépare l'acide 5-chloro-6-(3-chlorophénylsulfonyl)-1,3-benzodioxol-2-carboxylique et ses sels pharmaceutiquement acceptables avec des bases.

12. Procédé selon la revendication 1, caractérisé en ce qu'on prépare l'acide 5-chloro-6-(4-chlorophénylsulfonyl)-1,3-benzodioxol-2-carboxylique.

13. Procédé de préparation d'une préparation pharmaceutique, caractérisé par le traitement d'une substance active de l'invention selon les revendications 1-8 avec un support pharmaceutique.

14. Procédé de préparation d'une préparation pharmaceutique, caractérisé par le traitement d'une substance active selon l'invention selon les revendications 9-11 avec un support pharmaceutique.